# EUROPEAN PATENT APPLICATION

(11) **EP 3 736 293 A1**
(43) Date of publication of application: **11.11.2020**
(21) Application number: 20175944.6
(22) Date of filing: 12.02.2014
(51) Int. Cl.: C07K 16/28, C07K 16/30, G01N 33/574, A61P 35/00

(54) **THERAPEUTIC AND DIAGNOSTIC TARGET FOR CANCER COMPRISING DLL3 BINDING REAGENTS**

(30) Priority: 12.02.2013 GB 201302447
(62) Divisional of application: 14705561.0
(71) Applicant: BOEHRINGER INGELHEIM INTERNATIONAL GMBH, 55216 Ingelheim am Rhein (DE)
(72) Inventor: HUDSON, Lindsey Jane, Oxfordshire, Abingdon OX14 4RZ (GB)
(74) Representative: Simon, Elke Anna Maria

(57) **Abstract**

The present disclosure provides methods and compositions for treatment, screening, diagnosis and prognosis of cancer, such as lung cancer, pancreatic cancer and skin cancer, for monitoring the effectiveness of cancer, such as lung cancer, pancreatic cancer and skin cancer treatment, and for drug development.

SEQUENCE LISTING

<110>  BOEHRINGER INGELHEIM INTERNATIONAL GMBH

<120>  THERAPEUTIC AND DIAGNOSTIC TARGET

<130>  12-0390-EP-2

<160>  12

<170>  PatentIn version 3.5

<210>  1
<211>  618
<212>  PRT
<213>  Homo sapiens

<400>  1

<210>  2
<211>  587
<212>  PRT
<213>  Homo sapiens

<400>  2

Met Val Ser Pro Arg Met Ser Gly Leu Leu Ser Gln Thr Val Ile Leu
1               5                   10                  15

Ala Leu Ile Phe Leu Pro Gln Thr Arg Pro Ala Gly Val Phe Glu Leu
            20                  25                  30

Gln Ile His Ser Phe Gly Pro Gly Pro Gly Pro Gly Ala Pro Arg Ser
        35                  40                  45

Pro Cys Ser Ala Arg Leu Pro Cys Arg Leu Phe Phe Arg Val Cys Leu
    50                  55                  60

Lys Pro Gly Leu Ser Glu Glu Ala Ala Glu Ser Pro Cys Ala Leu Gly
65                  70                  75                  80

Ala Ala Leu Ser Ala Arg Gly Pro Val Tyr Thr Glu Gln Pro Gly Ala
                85                  90                  95

Pro Ala Pro Asp Leu Pro Leu Pro Asp Gly Leu Leu Gln Val Pro Phe
            100                 105                 110

Arg Asp Ala Trp Pro Gly Thr Phe Ser Phe Ile Ile Glu Thr Trp Arg
        115                 120                 125

Glu Glu Leu Gly Asp Gln Ile Gly Gly Pro Ala Trp Ser Leu Leu Ala
    130                 135                 140

Arg Val Ala Gly Arg Arg Arg Leu Ala Ala Gly Gly Pro Trp Ala Arg
145                 150                 155                 160

Asp Ile Gln Arg Ala Gly Ala Trp Glu Leu Arg Phe Ser Tyr Arg Ala
                165                 170                 175

Arg Cys Glu Pro Pro Ala Val Gly Thr Ala Cys Thr Arg Leu Cys Arg
            180                 185                 190

Pro Arg Ser Ala Pro Ser Arg Cys Gly Pro Gly Leu Arg Pro Cys Ala
        195                 200                 205

Pro Leu Glu Asp Glu Cys Glu Ala Pro Leu Val Cys Arg Ala Gly Cys
    210                 215                 220

Ser Pro Glu His Gly Phe Cys Glu Gln Pro Gly Glu Cys Arg Cys Leu
225                 230                 235                 240

Glu Gly Trp Thr Gly Pro Leu Cys Thr Val Pro Val Ser Thr Ser Ser
                245                 250                 255

Cys Leu Ser Pro Arg Gly Pro Ser Ser Ala Thr Thr Gly Cys Leu Val
            260                 265                 270

Pro Gly Pro Gly Pro Cys Asp Gly Asn Pro Cys Ala Asn Gly Gly Ser
        275                 280                 285

Cys Ser Glu Thr Pro Arg Ser Phe Glu Cys Thr Cys Pro Arg Gly Phe
    290                 295                 300

Tyr Gly Leu Arg Cys Glu Val Ser Gly Val Thr Cys Ala Asp Gly Pro
305                 310                 315                 320

Cys Phe Asn Gly Gly Leu Cys Val Gly Gly Ala Asp Pro Asp Ser Ala
                325                 330                 335

Tyr Ile Cys His Cys Pro Pro Gly Phe Gln Gly Ser Asn Cys Glu Lys
            340                 345                 350

Arg Val Asp Arg Cys Ser Leu Gln Pro Cys Arg Asn Gly Gly Leu Cys
        355                 360                 365

Leu Asp Leu Gly His Ala Leu Arg Cys Arg Cys Arg Ala Gly Phe Ala
    370                 375                 380

Gly Pro Arg Cys Glu His Asp Leu Asp Asp Cys Ala Gly Arg Ala Cys
385                 390                 395                 400

Ala Asn Gly Gly Thr Cys Val Glu Gly Gly Gly Ala His Arg Cys Ser
                405                 410                 415

Cys Ala Leu Gly Phe Gly Gly Arg Asp Cys Arg Glu Arg Ala Asp Pro
            420                 425                 430

Cys Ala Ala Arg Pro Cys Ala His Gly Gly Arg Cys Tyr Ala His Phe
        435                 440                 445

Ser Gly Leu Val Cys Ala Cys Ala Pro Gly Tyr Met Gly Ala Arg Cys
    450                 455                 460

Glu Phe Pro Val His Pro Asp Gly Ala Ser Ala Leu Pro Ala Ala Pro
465                 470                 475                 480

Pro Gly Leu Arg Pro Gly Asp Pro Gln Arg Tyr Leu Leu Pro Pro Ala
                485                 490                 495

Leu Gly Leu Leu Val Ala Ala Gly Val Ala Gly Ala Ala Leu Leu Leu
            500                 505                 510

Val His Val Arg Arg Arg Gly His Ser Gln Asp Ala Gly Ser Arg Leu
        515                 520                 525

Leu Ala Gly Thr Pro Glu Pro Ser Val His Ala Leu Pro Asp Ala Leu

530                 535                 540

Asn Asn Leu Arg Thr Gln Glu Gly Ser Gly Asp Gly Pro Ser Ser Ser
545                 550                 555                 560

Val Asp Trp Asn Arg Pro Glu Asp Val Asp Pro Gln Gly Ile Tyr Val
                565                 570                 575

Ile Ser Ala Pro Ser Ile Tyr Ala Arg Glu Ala
            580                 585

<210>  3
<211>  2389
<212>  DNA
<213>  Homo sapiens

<400>  3
agatataagg cttggaagcc agcagctgcg actcccgaga cccccccacc agaaggccat      60

ggtctcccca cggatgtccg ggctcctctc ccagactgtg atcctagcgc tcattttcct     120

cccccagaca cggcccgctg gcgtcttcga gctgcagatc cactctttcg ggccgggtcc     180

aggccctggg gccccgcggt ccccctgcag cgcccggctc ccctgccgcc tcttcttcag     240

agtctgcctg aagcctgggc tctcagagga ggccgccgag tccccgtgcg ccctgggcgc     300

ggcgctgagt gcgcgcggac cggtctacac cgagcagccc ggagcgcccg cgcctgatct     360

cccactgccc gacggcctct tgcaggtgcc cttccgggac gcctggcctg gcaccttctc     420

tttcatcatc gaaacctgga gagaggagtt aggagaccag attggaggcc ccgcctggag     480

cctgctggcg cgcgtggctg gcaggcggcg cttggcagcc ggaggcccgt gggcccggga     540

cattcagcgc gcaggcgcct gggagctgcg cttctcgtac cgcgcgcgct gcgagccgcc     600

tgccgtcggg accgcgtgca cgcgcctctg ccgtccgcgc agcgccccct cgcggtgcgg     660

tccgggactg cgcccctgcg caccgctcga ggacgaatgt gaggcgccgc tggtgtgccg     720

agcaggctgc agccctgagc atggcttctg tgaacagccc ggtgaatgcc gatgcctaga     780

gggctggact ggacccctct gcacggtccc tgtctccacc agcagctgcc tcagccccag     840

gggcccgtcc tctgctacca ccggatgcct tgtccctggg cctgggccct gtgacgggaa     900

cccgtgtgcc aatggaggca gctgtagtga gacacccagg tcctttgaat gcacctgccc     960

gcgtgggttc tacgggctgc ggtgtgaggt gagcggggtg acatgtgcag atggaccctg    1020

cttcaacggc ggcttgtgtg tcgggggtgc agaccctgac tctgcctaca tctgccactg    1080

cccacccggt ttccaaggct ccaactgtga gaagagggtg gaccggtgca gcctgcagcc    1140

atgccgcaat ggcggactct gcctggacct gggccacgcc ctgcgctgcc gctgccgcgc    1200

cggcttcgcg ggtcctcgct gcgagcacga cctggacgac tgcgcgggcc gcgcctgcgc    1260

taacggcggc acgtgtgtgg agggcggcgg cgcgcaccgc tgctcctgcg cgctgggctt 1320

cggcggccgc gactgccgcg agcgcgcgga cccgtgcgcc gcgcgcccct gtgctcacgg 1380

cggccgctgc tacgcccact tctccggcct cgtctgcgct tgcgctcccg gctacatggg 1440

agcgcggtgt gagttcccag tgcaccccga cggcgcaagc gccttgcccg cggccccgcc 1500

gggcctcagg cccggggacc ctcagcgcta ccttttgcct ccggctctgg gactgctcgt 1560

ggccgcgggc gtggccggcg ctgcgctctt gctggtccac gtgcgccgcc gtggccactc 1620

ccaggatgct gggtctcgct tgctggctgg gaccccggag ccgtcagtcc acgcactccc 1680

ggatgcactc aacaacctaa ggacgcagga gggttccggg gatggtccga gctcgtccgt 1740

agattggaat cgccctgaag atgtagaccc tcaagggatt tatgtcatat ctgctccttc 1800

catctacgct cgggaggtag cgacgcccct ttcccccccg ctacacactg ggcgcgctgg 1860

gcagaggcag cacctgcttt ttccctaccc ttcctcgatt ctgtccgtga aatgaattgg 1920

gtagagtctc tggaaggttt taagcccatt ttcagttcta acttactttc atcctatttt 1980

gcatccctct tatcgttttg agctacctgc catcttctct ttgaaaaacc tatgggcttg 2040

aggaggtcac gatgccgact ccgccagagc ttttccactg attgtactca gcggggaggc 2100

aggggaggca gaggggcagc ctctctaatg cttcctactc attttgtttc taggcctgac 2160

gcgtctcctc catccgcacc tggagtcaga gcgtggattt ttgtatttgc tcggtggtgc 2220

ccagtctctg ccccagaggc tttggagttc aatcttgaag gggtgtctgg gggaacttta 2280

ctgttgcaag ttgtaaataa tggttattta tatcctattt tttctcaccc catctctcta 2340

gaaacaccta taaaggctat tattgtgatc agttttgact aacaaaaaa 2389

<210> 4
<211> 2052
<212> DNA
<213> Homo sapiens

<400> 4
agatataagg cttggaagcc agcagctgcg actcccgaga cccccccacc agaaggccat 60

ggtctcccca cggatgtccg ggctcctctc ccagactgtg atcctagcgc tcattttcct 120

cccccagaca cggcccgctg gcgtcttcga gctgcagatc cactctttcg ggccgggtcc 180

aggccctggg gccccgcggt ccccctgcag cgcccggctc ccctgccgcc tcttcttcag 240

agtctgcctg aagcctgggc tctcagagga ggccgccgag tccccgtgcg ccctgggcgc 300

ggcgctgagt gcgcgcggac cggtctacac cgagcagccc ggagcgcccg cgcctgatct 360

cccactgccc gacggcctct tgcaggtgcc cttccgggac gcctggcctg gcaccttctc 420

tttcatcatc gaaacctgga gagaggagtt aggagaccag attggagggc ccgcctggag 480

cctgctggcg cgcgtggctg gcaggcggcg cttggcagcc ggaggcccgt gggcccggga 540

cattcagcgc gcaggcgcct gggagctgcg cttctcgtac cgcgcgcgct gcgagccgcc 600

tgccgtcggg accgcgtgca cgcgcctctg ccgtccgcgc agcgccccct cgcggtgcgg 660

tccgggactg cgcccctgcg caccgctcga ggacgaatgt gaggcgccgc tggtgtgccg 720

agcaggctgc agccctgagc atggcttctg tgaacagccc ggtgaatgcc gatgcctaga 780

gggctggact ggacccctct gcacggtccc tgtctccacc agcagctgcc tcagccccag 840

gggcccgtcc tctgctacca ccggatgcct tgtccctggg cctgggccct gtgacgggaa 900

cccgtgtgcc aatggaggca gctgtagtga gacacccagg tcctttgaat gcacctgccc 960

gcgtgggttc tacgggctgc ggtgtgaggt gagcggggtg acatgtgcag atggaccctg 1020

cttcaacggc ggcttgtgtg tcgggggtgc agaccctgac tctgcctaca tctgccactg 1080

cccacccggt ttccaaggct ccaactgtga gaagagggtg gaccggtgca gcctgcagcc 1140

atgccgcaat ggcggactct gcctggacct gggccacgcc ctgcgctgcc gctgccgcgc 1200

cggcttcgcg ggtcctcgct gcgagcacga cctggacgac tgcgcgggcc gcgcctgcgc 1260

taacggcggc acgtgtgtgg agggcggcgg cgcgcaccgc tgctcctgcg cgctgggctt 1320

cggcggccgc gactgccgcg agcgcgcgga cccgtgcgcc gcgcgcccct gtgctcacgg 1380

cggccgctgc tacgcccact tctccggcct cgtctgcgct tgcgctcccg gctacatggg 1440

agcgcggtgt gagttcccag tgcaccccga cggcgcaagc gccttgcccg cggccccgcc 1500

gggcctcagg cccggggacc ctcagcgcta ccttttgcct ccggctctgg gactgctcgt 1560

ggccgcgggc gtggccggcg ctgcgctctt gctggtccac gtgcgccgcc gtggccactc 1620

ccaggatgct gggtctcgct tgctggctgg gaccccggag ccgtcagtcc acgcactccc 1680

ggatgcactc aacaacctaa ggacgcagga gggttccggg gatggtccga gctcgtccgt 1740

agattggaat cgccctgaag atgtagaccc tcaagggatt tatgtcatat ctgctccttc 1800

catctacgct cgggaggcct gacgcgtctc ctccatccgc acctggagtc agagcgtgga 1860

tttttgtatt tgctcggtgg tgcccagtct ctgccccaga ggctttggag ttcaatcttg 1920

aaggggtgtc tgggggaact ttactgttgc aagttgtaaa taatggttat ttatatccta 1980

ttttttctca ccccatctct ctagaaacac ctataaaggc tattattgtg atcagttttg 2040

actaacaaaa aa 2052

<210> 5
<211> 25
<212> PRT
<213> Homo sapiens

<400> 5

Val Cys Leu Lys Pro Gly Leu Ser Glu Glu Ala Ala Glu Ser Pro Cys
1               5                   10                  15

Ala Leu Gly Ala Ala Leu Ser Ala Arg
            20                  25

<210>  6
<211>  7
<212>  PRT
<213>  Homo sapiens

<400>  6

Ala Gly Ala Trp Glu Leu Arg
1               5

<210>  7
<211>  12
<212>  PRT
<213>  Homo sapiens

<400>  7

Cys Glu Pro Pro Ala Val Gly Thr Ala Cys Thr Arg
1               5                   10

<210>  8
<211>  17
<212>  PRT
<213>  Homo sapiens

<400>  8

Ala Gly Cys Ser Pro Glu His Gly Phe Cys Glu Gln Pro Gly Glu Cys
1               5                   10                  15

Arg

<210>  9
<211>  8
<212>  PRT
<213>  Homo sapiens

<400>  9

Ser Phe Glu Cys Thr Cys Pro Arg
1               5

<210>  10
<211>  13
<212>  PRT
<213>  Homo sapiens

<400>  10

Asn Gly Gly Leu Cys Leu Asp Leu Gly His Ala Leu Arg
1               5                   10

<210>  11
<211>  10
<212>  PRT
<213>  Homo sapiens

<400>  11

Cys Ser Cys Ala Leu Gly Phe Gly Gly Arg
1               5                   10


<210>  12
<211>  466
<212>  PRT
<213>  Homo sapiens

<400>  12

Ala Gly Val Phe Glu Leu Gln Ile His Ser Phe Gly Pro Gly Pro Gly
1               5                   10                  15

Pro Gly Ala Pro Arg Ser Pro Cys Ser Ala Arg Leu Pro Cys Arg Leu
            20                  25                  30

Phe Phe Arg Val Cys Leu Lys Pro Gly Leu Ser Glu Glu Ala Ala Glu
        35                  40                  45

Ser Pro Cys Ala Leu Gly Ala Ala Leu Ser Ala Arg Gly Pro Val Tyr
    50                  55                  60

Thr Glu Gln Pro Gly Ala Pro Ala Pro Asp Leu Pro Leu Pro Asp Gly
65                  70                  75                  80

Leu Leu Gln Val Pro Phe Arg Asp Ala Trp Pro Gly Thr Phe Ser Phe
                85                  90                  95

Ile Ile Glu Thr Trp Arg Glu Glu Leu Gly Asp Gln Ile Gly Gly Pro
            100                 105                 110

Ala Trp Ser Leu Leu Ala Arg Val Ala Gly Arg Arg Arg Leu Ala Ala
        115                 120                 125

Gly Gly Pro Trp Ala Arg Asp Ile Gln Arg Ala Gly Ala Trp Glu Leu
    130                 135                 140

Arg Phe Ser Tyr Arg Ala Arg Cys Glu Pro Pro Ala Val Gly Thr Ala
145                 150                 155                 160

Cys Thr Arg Leu Cys Arg Pro Arg Ser Ala Pro Ser Arg Cys Gly Pro
                165                 170                 175

Gly Leu Arg Pro Cys Ala Pro Leu Glu Asp Glu Cys Glu Ala Pro Leu
        180                 185                 190

Val Cys Arg Ala Gly Cys Ser Pro Glu His Gly Phe Cys Glu Gln Pro
    195                 200                 205

Gly Glu Cys Arg Cys Leu Glu Gly Trp Thr Gly Pro Leu Cys Thr Val
    210                 215                 220

Pro Val Ser Thr Ser Ser Cys Leu Ser Pro Arg Gly Pro Ser Ser Ala
225                 230                 235                 240

Thr Thr Gly Cys Leu Val Pro Gly Pro Gly Pro Cys Asp Gly Asn Pro
                245                 250                 255

Cys Ala Asn Gly Gly Ser Cys Ser Glu Thr Pro Arg Ser Phe Glu Cys
            260                 265                 270

Thr Cys Pro Arg Gly Phe Tyr Gly Leu Arg Cys Glu Val Ser Gly Val
        275                 280                 285

Thr Cys Ala Asp Gly Pro Cys Phe Asn Gly Gly Leu Cys Val Gly Gly
    290                 295                 300

Ala Asp Pro Asp Ser Ala Tyr Ile Cys His Cys Pro Pro Gly Phe Gln
305                 310                 315                 320

Gly Ser Asn Cys Glu Lys Arg Val Asp Arg Cys Ser Leu Gln Pro Cys
                325                 330                 335

Arg Asn Gly Gly Leu Cys Leu Asp Leu Gly His Ala Leu Arg Cys Arg
            340                 345                 350

Cys Arg Ala Gly Phe Ala Gly Pro Arg Cys Glu His Asp Leu Asp Asp
        355                 360                 365

Cys Ala Gly Arg Ala Cys Ala Asn Gly Gly Thr Cys Val Glu Gly Gly
    370                 375                 380

Gly Ala His Arg Cys Ser Cys Ala Leu Gly Phe Gly Gly Arg Asp Cys
385                 390                 395                 400

Arg Glu Arg Ala Asp Pro Cys Ala Ala Arg Pro Cys Ala His Gly Gly
                405                 410                 415

Arg Cys Tyr Ala His Phe Ser Gly Leu Val Cys Ala Cys Ala Pro Gly
            420                 425                 430

Tyr Met Gly Ala Arg Cys Glu Phe Pro Val His Pro Asp Gly Ala Ser
        435                 440                 445

Ala Leu Pro Ala Ala Pro Pro Gly Leu Arg Pro Gly Asp Pro Gln Arg
    450                 455                 460

Tyr Leu
465

## Description

### INTRODUCTION

The present invention relates to the identification of a membrane protein associated with cancer, such as lung cancer, pancreatic cancer and/or skin cancer which has utility as a therapeutic target for the treatment of cancers or as a marker for cancers. In particular, the protein represents a biological target against which affinity reagents including therapeutic antibodies, or other pharmaceutical agents, can be made. The invention also relates to the use of such affinity reagents for the treatment and/or diagnosis of cancers.

### BACKGROUND OF THE INVENTION

The major challenges in treatment of cancer, such as lung cancer, pancreatic cancer and skin cancer are to improve early detection rates, to find new non-invasive markers that can be used to follow disease progression and identify relapse, and to find improved and less toxic therapies, especially for more advanced disease where 5 year survival is still poor. There is a great need to identify targets which are more specific to the cancer cells, e.g. ones which are expressed on the surface of the tumour cells so that they can be attacked by promising new approaches like immunotherapeutics and targeted toxins.

Delta-like protein 3 is a type I membrane protein and is a member of the Delta family. The inventor has shown Delta-like protein 3 is expressed in cancer, suggesting affinity-based therapies directed against Delta-like protein 3 in patients including those with cancer will have a therapeutic effect.

### SUMMARY OF THE INVENTION

The present invention discloses the detection of Delta-like protein 3, hereinafter referred to as DLL3, in membrane extracts of various disease tissues, e.g. lung cancer, pancreatic cancer and skin cancer, hereinafter referred to as 'the diseases of the invention'.

The differential expression of DLL3 in various cancers permits the protein to be targeted using affinity reagent-, e.g. antibody-, based therapies for such cancers. Thus DLL3 can be used in the generation of affinity reagents, including antibodies, that bind specifically to epitopes within DLL3, and can be targeted by such affinity reagents as the basis of treatment. Affinity reagents, including antibodies, that target a protein on the cell surface of cancer cells may be employed in the treatment of cancer through a variety of mechanisms, including (i) lysis by complement mediated or antibody-dependent cellular cytotoxicity (ADCC), (ii) lysis by drugs or toxin(s) conjugated to such affinity reagents or (iii) inhibition of the physiological function of such protein, which may be driving growth of cancer cells, e.g. through signaling pathways. An important aspect of such affinity reagent-based treatment is that the normal expression profile of the protein target, in terms of tissue distribution and expression level, is such that any targeting of the protein target on normal tissues by the antibody does not give rise to adverse side-effects through binding to normal tissues.

The invention provides a method for the treatment or prophylaxis of cancer wherein DLL3 is expressed in said cancer, which comprises administering to a subject in need thereof a therapeutically effective amount of an affinity reagent which binds to DLL3.

The cancer is preferably one of the diseases of the invention.

The invention also provides an affinity reagent which binds to DLL3 for use in the treatment or prophylaxis of cancer, preferably wherein the cancer is one of the diseases of the invention.

The invention also provides the use of an affinity reagent which binds to DLL3 in the manufacture of a medicament for the treatment or prophylaxis of cancer, preferably wherein the cancer is one of the diseases of the invention.

The affinity reagents for use in the invention preferably bind specifically to DLL3.

The affinity reagent may be an antibody, e.g. a whole antibody, or a functional fragment thereof or an antibody mimetic. Preferred affinity reagents included antibodies for example monoclonal antibodies.

The affinity reagent may be a chimeric antibody, a human antibody, a humanized antibody, a single chain antibody, a defucosylated antibody or a bispecific antibody.

Functional antibody fragments include is a UniBody, a domain antibody or a Nanobody.

Antibody mimetics include an Affibody, a DARPin, an Anticalin, an Avimer, a Versabody or a Duocalin.

The affinity reagents for use in the invention may contain or be conjugated to a therapeutic moiety, such as a cytotoxic moiety or a radioactive isotope. The affinity reagent may be an antibody drug conjugate or immunoconjugate.

The affinity reagent may elicit antibody-dependent cellular cytotoxicity (ADCC) or may elicit complement dependent cytotoxicity (CDC). The affinity reagent may induce apoptosis of cancer cells, kill or reduce the number of cancer stem cells and/or kill or reduce the number of circulating cancer cells. Affinity reagents may modulate a physiological function of DLL3, inhibit ligand binding to DLL3 and/or inhibit a signal transduction pathway mediated by DLL3.

In an alternative embodiment, the invention also provides a method for the treatment or prophylaxis of cancer wherein DLL3 is expressed in said cancer, which comprises administering to a subject in need thereof a therapeutically effective amount of a hybridizing agent capable of hybridizing to nucleic acid encoding DLL3.

The invention also provides a hybridizing agent capable of hybridizing to nucleic acid encoding DLL3 for use in the treatment or prophylaxis of a cancer, preferably wherein the cancer is one of the diseases of the invention.

The invention also provides the use of a hybridizing agent capable of hybridizing to nucleic acid encoding DLL3 in the manufacture of a medicament for the treatment or prophylaxis of a cancer, preferably wherein the cancer is one of the diseases of the invention.

The hybridizing agents for use in the invention preferably bind specifically to nucleic acid encoding one or more extracellular domains of DLL3.

Suitable hybridizing agents for use in the invention include inhibitory RNA, short interfering RNA (siRNA), short hairpin RNA (shRNA), microRNA (miRNA), anti-sense nucleic acid, complementary DNA (cDNA), oligonucleotides and ribozymes.

The invention also provides a method of detecting, diagnosing and/or screening for or monitoring the progression of a cancer wherein DLL3 is expressed in said cancer, or of monitoring the effect of a cancer drug or therapy wherein DLL3 is expressed in said cancer, in a subject which comprises detecting the presence or level of DLL3, or one or more fragments thereof, or the presence or level of nucleic acid encoding DLL3 or which comprises detecting a change in the level thereof in said subject.

Such a method may comprise detecting the presence of DLL3, or one or more fragments thereof, or the presence of nucleic acid encoding DLL3, in which either (a) the presence of an elevated level of DLL3 or said one or more fragments thereof or an elevated level of nucleic acid encoding DLL3 in the subject as compared with the level in a healthy subject, or (b) the presence of a detectable level of DLL3 or said one or more fragments thereof or a detectable level of nucleic acid encoding DLL3 in the subject as compared with a corresponding undetectable level in a healthy subject is indicative of the presence of the cancer wherein DLL3 is expressed in said cancer, in said subject.

The invention also provides a method of detecting, diagnosing and/or screening for or monitoring the progression a cancer wherein DLL3 is expressed in said cancer, or of monitoring the effect of a cancer drug or therapy wherein DLL3 is expressed in said cancer, in a subject which comprises detecting the presence or level of antibodies capable of immunospecific binding to DLL3, or one or more fragments thereof.

In the methods according to the invention, the presence of DLL3, or one or more fragments thereof, or the presence of nucleic acid encoding DLL3, or the presence or level of antibodies capable of immunospecific binding to DLL3, or one or more fragments thereof, may be detected by analysis of a biological sample obtained from the subject.

The presence of DLL3, or one or more fragments thereof, may be detected using an affinity reagent which binds to DLL3. The affinity reagent may be any suitable affinity reagent as mentioned herein. The affinity reagent may contain or be conjugated to a detectable label.

In any of the aspects of the invention referred to herein, the subject may be a human.

The invention also provides methods for identifying an agent for the treatment or prophylaxis of cancer wherein DLL3 is expressed in said cancer, wherein the method comprises (a) contacting DLL3, or one or more fragments thereof, with a candidate agent; and (b) determining whether the agent binds to DLL3, or one or more fragments thereof. The method may also further comprise the step of testing the ability of an agent which binds to DLL3, or one or more fragments thereof, to inhibit cancer wherein DLL3 is expressed in said cancer. The agent may, *inter alia,* modulate an activity of DLL3, reduce ligand binding to DLL3 or reduce DLL3 dimerisation.

In the various embodiments of the invention described herein, particular cancer types which may be mentioned are one of the diseases of the invention.

In one embodiment the cancer to be detected, prevented or treated is lung cancer, e.g. non-small cell lung cancer and/or small cell lung cancer.

In another embodiment the cancer to be detected, prevented or treated is pancreatic cancer.

In another embodiment the cancer to be detected, prevented or treated is skin cancer, e.g. melanoma.

Other aspects of the present invention are set out below and in the claims herein.

### BRIEF DESCRIPTION OF THE FIGURES

Figure 1a shows the internalization of anti-DLL3 polyclonal antibodies by SHP-77 cells, using PabZAP assay.
Figure 1b shows the internalization of anti-DLL3 polyclonal antibodies by N82 cells, using PabZAP assay.
Figure 2 shows the specific lysis of DMS79 DLL3 expressing cells by activation of T cells by bispecific anti-DLL3-anti-CD3 polyclonal antibodies

### DETAILED DESCRIPTION OF THE INVENTION

The invention described in detail below encompasses the administration of therapeutic compositions to a subject, e.g. a mammalian subject, to treat or prevent cancer, e.g. the diseases of the invention. The invention also provides methods and compositions for clinical screening, diagnosis and prognosis of cancer, e.g. the diseases of the invention, in a mammalian subject for identifying patients most likely to respond to a particular therapeutic treatment, for monitoring the results of cancer e.g. the diseases of the invention therapy, for drug screening and drug development.

The invention is based on the finding that DLL3 protein is expressed in certain cancers. In particular, supporting data is enclosed herein which demonstrates the expression of DLL3 protein in the plasma membrane of lung cancer, pancreatic cancer and skin cancer. Therefore antibodies directed to DLL3 may have utility as therapeutics and diagnostics in these cancers and other cancer types showing expression of DLL3.

As used herein, the term "subject" refers to animal, preferably a mammal. The mammalian subject may be a non-human mammal, but is generally a human, such as a human adult.

The subject will in general be a living subject. However, whilst the uses, methods and compositions of the present invention are specially suited for screening, diagnosis and prognosis of a living subject, they may also be used for postmortem diagnosis in a subject, for example, to identify family members at risk of developing the same disease.

As used herein, the term "patient" refers to a subject who has or is suspected of having one or more of the diseases of the invention.

As used herein, the term "protein of the invention" refers to Delta-like protein 3 (GeneID: 10683), which is referred to herein as DLL3. This protein has been found to be differentially expressed in various cancers thus providing a new target for affinity-based therapies of these cancers. A human sequence of the DLL3 protein is given in SEQ ID NO: 1. The term DLL3 (in the context of a protein) encompasses proteins whose amino acid sequences consist of or comprise the amino acid sequence given in SEQ ID NO: 1 or derivatives or variants thereof, particularly naturally-occurring human derivatives or variants thereof.

This protein has been identified in membrane protein extracts of cancer tissue samples from cancer patients through the methods and apparatus described in Example 1 (e.g. by liquid chromatography-mass spectrometry of membrane protein extracts). Peptide sequences were compared to the SWISS PROT and TrEMBL databases (held by the Swiss Institute of Bioinformatics (SIB) and the European Bioinformatics Institute (EBI) which are available at www.expasy.org), and the entry Q9NYJ7, Delta-like protein 3 - DLL3, was identified. The nucleotide sequence encoding this protein is found at accession number NM_016941, as given in SEQ ID NO: 3.

According to SWISS-PROT, Delta-like protein 3 is a type I membrane protein of the Delta family and consists of one DSL domain, six EGF-like domains, one transmembrane region and an extracellular tail between amino acids 27-492 of SEQ ID NO: 1 (SEQ ID NO: 12). The inventor has shown Delta-like protein 3 is expressed in cancer, suggesting affinity-based therapies directed against Delta-like protein 3 in patients including those with cancer will have a therapeutic effect.

DLL3 is useful as are fragments particularly epitope containing fragments e.g. antigenic or immunogenic fragments thereof and derivatives thereof, particularly fragments comprising extracellular domains (e.g. extracellular tails or loops) of the protein. Epitope containing fragments, including antigenic or immunogenic fragments, will typically be of length 12 amino acids or more, e.g. 20 amino acids or more, e.g. 50 or 100 amino acids or more. Fragments may be 95% or more of the length of the full protein, e.g. 90% or more, e.g. 75% or 50% or 25% or 10% or more of the length of the full protein.

Alternatively, the protein/polypeptide employed or referred to herein may be limited to those proteins/polypeptides specifically recited/described in the present specification or to a variant or derivative which has at least 80, 85, 90, 91, 92, 93, 94, 95, 96, 97, 98 or 99% amino acid sequence identity or similarity thereto. Percentage amino acid sequence identity/similarity may be determined by any suitable algorithm, e.g. BLAST, CLUSTAL, using appropriate default parameters.

Hence the term "DLL3" in the context of a protein or polypeptide refers to a protein whose amino acid sequence consists of or comprises the amino sequence given in any of SEQ ID NO: 1 or 2 or a derivative or variant thereof which has at least 90% or 95% sequence identity to any of SEQ ID NO: 1 or 2 and which protein has essentially the same tissue distribution as DLL3.

In the context of a nucleic acid, the term "DLL3" refers to a nucleic acid whose nucleotide sequence encodes a protein comprising the amino sequence given in any of SEQ ID NO: 1 or 2 or a derivative or variant thereof which has at least 90% or 95% sequence identity to any of SEQ ID NO: 1 or 2 and which protein has essentially the same tissue distribution as DLL3 protein.

The term "DLL3" in the context of a nucleic acid also refers to a nucleic acid whose nucleotide sequence comprises the sequence given in any of SEQ ID NO: 3 or 4 or a derivative or variant thereof which has at least 90% or 95% sequence identity to any of SEQ ID NO: 3 or 4 and which encodes a protein which has essentially the same tissue distribution as DLL3 protein.

Epitope-containing fragments of DLL3 including antigenic or immunogenic fragments will be capable of eliciting a relevant immune response in a patient. DNA encoding DLL3 is also useful as are fragments thereof, e.g. DNA encoding fragments of DLL3 such as immunogenic fragments thereof. Fragments of nucleic acid (e.g. DNA) encoding DLL3 may be 95% or more of the length of the full coding region, e.g. 90% or more e.g. 75% or 50% or 25% or 10% or more of the length of the full coding region. Fragments of nucleic acid (e.g. DNA) may be 36 nucleotides or more, e.g. 60 nucleotides or more, e.g. 150 or 300 nucleotides or more in length.

Derivatives of DLL3 include variants on the sequence in which one or more (e.g. 1-20 such as 15 amino acids, or up to 20% such as up to 10% or 5% or 1% by number of amino acids based on the total length of the protein) deletions, insertions or substitutions have been made. Substitutions may typically be conservative substitutions. Derivatives will typically have essentially the same biological function as the protein from which they are derived. Derivatives will typically be comparably antigenic or immunogenic to the protein from which they are derived. Derivatives will typically have either the ligand-binding activity, or the active receptor-complex forming ability, or preferably both, of the protein from which they are derived. Derivatives and variants will generally have the same tissue distribution as DLL3.

Derivatives of proteins also include chemically treated protein such as carboxymethylated, carboxyamidated, acetylated proteins, for example treated during purification.

In one aspect, the invention provides DLL3 or a composition comprising DLL3. The protein may be in isolated or purified form. The invention further provides a nucleic acid encoding DLL3 and a composition comprising a nucleic acid encoding DLL3.

In a further aspect, there is provided a composition capable of eliciting an immune response in a subject, which composition comprises a DLL3 polypeptide and/or one or more antigenic or immunogenic fragments thereof, and one or more suitable carriers, excipients, diluents or adjuvants (suitable adjuvants are discussed below).

The composition capable of eliciting an immune response may for example be provided as a vaccine comprising a DLL3 polypeptide or derivative or variant thereof, and/or one or more antigenic or immunogenic fragments thereof, optionally together with one or more suitable carriers, excipients, diluents or adjuvants.

In another aspect, the invention provides a DLL3 polypeptide, or one or more fragments or derivatives or variants thereof, for the treatment or prophylaxis of e.g. one or more of the diseases of the invention.

In another aspect, the invention provides a use of a DLL3 polypeptide, or one or more fragments or derivatives or variants thereof, for the treatment or prophylaxis of e.g. one or more of the diseases of the invention.

The invention also provides a use of a DLL3 polypeptide, one or more fragments or derivatives or variants thereof, in the manufacture of a medicament for the treatment or prophylaxis of e.g. one or more of the diseases of the invention.

In one aspect there is provided a method of treatment comprising administering a therapeutically effective amount of a DLL3 polypeptide, one or more fragments or derivatives or variants thereof,, for the treatment or prophylaxis of e.g. one or more of the diseases of the invention.

The invention further provides a method for the treatment or prophylaxis of e.g. the diseases of the invention in a subject, or of vaccinating a subject against e.g. one or more of the diseases of the invention, which comprises the step of administering to the subject an effective amount of a DLL3 polypeptide and/or one or more antigenic or immunogenic fragments or derivatives or variants thereof, for example as a vaccine.

In another aspect, the invention provides methods of treating e.g. the diseases of the invention, comprising administering to a patient a therapeutically effective amount of a compound that modulates (e.g. upregulates or downregulates) or complements the expression or the biological activity (or both) of DLL3 in patients having e.g. the diseases of the invention, in order to (a) prevent the onset or development of e.g. the diseases of the invention; (b) prevent the progression of e.g. the diseases of the invention; or (c) ameliorate the symptoms of e.g. the diseases of the invention.

In yet a further embodiment, the invention provides a medicament comprising, separately or together:
(a) DLL3, and
(b) an anti-cancer agent,
for simultaneous, sequential or separate administration in the treatment of cancer, preferably in the treatment of one of the diseases of the invention.

DLL3 can be used for detection, prognosis, diagnosis, or monitoring of, e.g. the diseases of the invention or for drug development.

According to another aspect of the invention, we provide a method of detecting, diagnosing and/or screening for or monitoring the progression of e.g. the diseases of the invention or of monitoring the effect of e.g. an anti-cancer drug or therapy directed towards the diseases of the invention in a subject which comprises detecting the presence or level of DLL3, or one or more fragments thereof, or the presence or level of nucleic acid encoding DLL3 or the presence or level of the activity of DLL3 or which comprises detecting a change in the level thereof in said subject.

According to another aspect of the invention we provide a method of detecting, diagnosing and/or screening for e.g. the diseases of the invention in a candidate subject which comprises detecting the presence of DLL3, or one or more fragments thereof, or the presence of nucleic acid encoding DLL3 or the presence of the activity of DLL3 in said candidate subject, in which either (a) the presence of an elevated level of DLL3 or said one or more fragments thereof or an elevated level of nucleic acid encoding DLL3 or the presence of an elevated level of DLL3 activity in the candidate subject as compared with the level in a healthy subject or (b) the presence of a detectable level of DLL3 or said one or more fragments thereof or a detectable level of nucleic acid encoding DLL3 or the presence of a detectable level of DLL3 activity in the candidate subject as compared with a corresponding undetectable level in a healthy subject indicates the presence of e.g. the diseases of the invention in said subject.

According to another aspect of the invention, we provide a method of monitoring the progression of e.g. the diseases of the invention in a subject or of monitoring the effect of e.g. an anti-cancer drug or therapy directed towards the diseases of the invention which comprises detecting the presence of DLL3, or one or more fragments thereof, or the presence of nucleic acid encoding DLL3 or the presence of the activity of DLL3 in said candidate subject at a first time point and at a later time point, the presence of an elevated or lowered level of DLL3 or said one or more fragments thereof or an elevated or lowered level of nucleic acid encoding DLL3 or the presence of an elevated or lowered level of DLL3 activity in the subject at the later time point as compared with the level in the subject at said first time point, indicating the progression or regression of e.g. the diseases of the invention or indicating the effect or non-effect of e.g. an anti-cancer drug or therapy directed towards the diseases of the invention in said subject.

For DLL3, the detected level obtained upon analyzing tissue sample from subjects having e.g. the diseases of the invention relative to the detected level obtained upon analyzing tissue from subjects free from e.g. the diseases of the invention will depend upon the particular analytical protocol and detection technique that is used. Accordingly, the present invention contemplates that each laboratory will establish a reference range in subjects free from e.g. the diseases of the invention according to the analytical protocol and detection technique in use, as is conventional in the diagnostic art. Preferably, at least one control positive tissue sample from a subject known to have e.g. the diseases of the invention or at least one control negative tissue sample from a subject known to be free from e.g. the diseases of the invention (and more preferably both positive and negative control samples) are included in each batch of test samples analysed.

In one aspect of the invention, liquid chromatography-mass spectrometry analysis or other appropriate methods are used to analyze the diseases of the invention tissue samples from a subject, preferably a living subject, in order to measure the expression of DLL3 for screening or diagnosis of e.g. the diseases of the invention, to determine the prognosis of a the diseases of the invention patient, to monitor the effectiveness of the diseases of the invention therapy, or for drug development.

In any of the above methods, the level that may be detected in the candidate subject who has cancer, e.g. the diseases of the invention is preferably 2 or more fold higher than the level in the healthy subject.

In one embodiment of the invention, tissue sample from a subject (e.g. a subject suspected of having the diseases of the invention) is analysed by liquid chromatography-mass spectrometry for detection of DLL3. An increased abundance of DLL3 in the tissue from the subject relative to tissue from a subject or subjects free from the diseases of the invention (e.g. a control sample) or a previously determined reference range indicates the presence of the diseases of the invention.

In relation to fragments, epitope containing fragments, immunogenic fragments or antigenic fragments of DLL3:
for the relevant cancer applications, in one aspect of the invention these comprise the sequence identified as a tryptic sequence in Example 1.

As used herein, DLL3 is "isolated" when it is present in a preparation that is substantially free of contaminating proteins, i.e. a preparation in which less than 10% (for example less than 5%, such as less than 1%) of the total protein present is contaminating protein(s). A contaminating protein is a protein having a significantly different amino acid sequence from that of isolated DLL3, as determined by mass spectral analysis. As used herein, a "significantly different" sequence is one that permits the contaminating protein to be resolved from DLL3 by mass spectral analysis, performed according to the protocol described herein in Example 1.

In the diagnostic and prognostic methods of the invention, DLL3 can be assayed by any method known to those skilled in the art, including but not limited to, the Preferred Technologies described herein, kinase assays, enzyme assays, binding assays and other functional assays, immunoassays, and western blotting.

Alternatively, DLL3 can be detected in an immunoassay. In one embodiment, an immunoassay is performed by contacting a sample from a subject to be tested with an anti-DLL3 antibody (or other affinity reagent) under conditions such that binding (e.g. immunospecific binding) can occur if DLL3 is present, and detecting or measuring the amount of any binding (e.g. immunospecific binding) by the agent. DLL3 binding agents can be produced by the methods and techniques taught herein. In a particular embodiment, DLL3 is analysed using immunohistochemistry.

DLL3 may be detected by virtue of the detection of a fragment thereof e.g. an epitope containing (e.g. an immunogenic or antigenic) fragment thereof. Fragments may have a length of at least 10, more typically at least 20 amino acids e.g. at least 50 or 100 amino acids e.g. at least 150 or 200 amino acids; e.g. at least 300 or 500 amino acids; e.g. at least 700 or 900 amino acids.

In one embodiment, binding of an affinity reagent (e.g. an antibody) in tissue sections can be used to detect aberrant DLL3 localization or an aberrant level of DLL3. In a specific embodiment, an antibody (or other affinity reagent) to DLL3 can be used to assay a patient tissue (e.g. a lung, pancreas and skin tissue) for the level of DLL3 where an aberrant level of DLL3 is indicative of the diseases of the invention. As used herein, an "aberrant level" means a level that is increased compared with the level in a subject free from the diseases of the invention or a reference level.

Any suitable immunoassay can be used, including, without limitation, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

For example, DLL3 can be detected in a fluid sample (e.g. blood, urine, or saliva) by means of a two-step sandwich assay. In the first step, a capture reagent (e.g. an anti-DLL3 antibody or other affinity reagent) is used to capture DLL3. The capture reagent can optionally be immobilized on a solid phase. In the second step, a directly or indirectly labelled detection reagent is used to detect the captured DLL3. In one embodiment, the detection reagent is a lectin. Any lectin can be used for this purpose that preferentially binds to DLL3 rather than to other isoforms that have the same core protein as DLL3 or to other proteins that share the antigenic determinant recognized by the antibody. In a preferred embodiment, the chosen lectin binds DLL3 with at least 2-fold greater affinity, more preferably at least 5-fold greater affinity, still more preferably at least 10-fold greater affinity, than to said other isoforms that have the same core protein as DLL3 or to said other proteins that share the antigenic determinant recognized by the affinity reagent. Based on the present description, a lectin that is suitable for detecting DLL3 can readily be identified by methods well known in the art, for instance upon testing one or more lectins enumerated in Table I on pages 158-159 of Sumar et al., Lectins as Indicators of Disease-Associated Glycoforms, In: Gabius H-J & Gabius S (eds.), 1993, Lectins and Glycobiology, at pp. 158-174 (which is incorporated herein by reference in its entirety). In an alternative embodiment, the detection reagent is an antibody (or other affinity reagent), e.g. an antibody that specifically (e.g. immunospecifically) detects other post-translational modifications, such as an antibody that immunospecifically binds to phosphorylated amino acids. Examples of such antibodies include those that bind to phosphotyrosine (BD Transduction Laboratories, catalog nos.: P11230-050/P11230-150; P11120; P38820; P39020), those that bind to phosphoserine (Zymed Laboratories Inc., South San Francisco, CA, catalog no. 61-8100) and those that bind to phosphothreonine (Zymed Laboratories Inc., South San Francisco, CA, catalogue nos. 71-8200, 13-9200).

If desired, a gene encoding DLL3, a related gene, or related nucleic acid sequences or subsequences, including complementary sequences, can also be used in hybridization assays. A nucleotide encoding DLL3, or subsequences thereof comprising at least 8 nucleotides, preferably at least 12 nucleotides, and most preferably at least 15 nucleotides can be used as a hybridization probe. Hybridization assays can be used for detection, prognosis, diagnosis, or monitoring of conditions, disorders, or disease states, associated with aberrant expression of the gene encoding DLL3, or for differential diagnosis of subjects with signs or symptoms suggestive of e.g. the diseases of the invention. In particular, such a hybridization assay can be carried out by a method comprising contacting a subject's sample containing nucleic acid with a nucleic acid probe capable of hybridizing to a DNA or RNA that encodes DLL3, under conditions such that hybridization can occur, and detecting or measuring any resulting hybridization.

Hence nucleic acid encoding DLL3 (e.g. DNA or more suitably RNA) may be detected, for example, using a hybridizing agent (particularly an oligonucleotide probe) capable of hybridizing to nucleic acid encoding DLL3.

One such exemplary method comprises:
contacting one or more oligonucleotide probes comprising 10 or more consecutive nucleotides complementary to a nucleotide sequence encoding DLL3, with an RNA obtained from a biological sample from the subject or with cDNA copied from the RNA, wherein said contacting occurs under conditions that permit hybridization of the probe to the nucleotide sequence if present;
detecting hybridization, if any, between the probe and the nucleotide sequence; and
comparing the hybridization, if any, detected in step (b) with the hybridization detected in a control sample, or with a previously determined reference range.
The invention also provides diagnostic kits, comprising an anti-DLL3 antibody (or other affinity reagent). In addition, such a kit may optionally comprise one or more of the following:
   (1) instructions for using the anti-DLL3 affinity reagent for diagnosis, prognosis, therapeutic monitoring or any combination of these applications;
   (2) a labelled binding partner to the affinity reagent;
   (3) a solid phase (such as a reagent strip) upon which the anti-DLL3 affinity reagent is immobilized; and
   (4) a label or insert indicating regulatory approval for diagnostic, prognostic or therapeutic use or any combination thereof. If no labelled binding partner to the affinity reagent is provided, the anti-DLL3 affinity reagent itself can be labelled with a detectable marker, e.g. a chemilumine scent, enzymatic, fluorescent, or radioactive moiety.

The invention also provides a kit comprising a nucleic acid probe capable of hybridizing to nucleic acid, suitably RNA, encoding DLL3. In a specific embodiment, a kit comprises one or more containers a pair of primers (e.g. each in the size range of 6-30 nucleotides, more preferably 10-30 nucleotides and still more preferably 10-20 nucleotides) that under appropriate reaction conditions can prime amplification of at least a portion of a nucleic acid encoding DLL3, such as by polymerase chain reaction (see, e.g. Innis et al., 1990, PCR Protocols, Academic Press, Inc., San Diego, CA), ligase chain reaction (see EP 320,308) use of Qβ replicase, cyclic probe reaction, or other methods known in the art.

A kit can optionally further comprise a predetermined amount of DLL3 or a nucleic acid encoding DLL3, e.g. for use as a standard or control.

As used herein, the term "sample" includes a bodily fluid (e.g. blood, urine or saliva) and tissue biopsies taken from a subject at risk of having one or more of the diseases of the invention (e.g. a biopsy such as a lung, pancreas and skin biopsy) or homogenate thereof.

For example, the biological sample used can be from any source such as a serum sample or a tissue sample e.g. lung, pancreas and skin tissue. For instance, when looking for evidence of metastatic the diseases of the invention, one would look at major sites of the diseases of the invention metastasis, e.g. the brain, liver, bones and adrenal glands for lung cancer; the liver for pancreatic cancer or the lungs, brain and bones for skin cancer.

Alternatively the presence of DLL3, or one or more fragments thereof, or the presence of nucleic acid encoding DLL3 or the presence of the activity of DLL3 may be detected by analysis in situ.

In certain embodiments, methods of diagnosis described herein may be at least partly, or wholly, performed *in vitro* or *ex vivo.*

Suitably the presence of DLL3, or one or more fragments thereof, or the presence of nucleic acid encoding DLL3 or the presence of the activity of DLL3 is detected quantitatively.

For example, quantitatively detecting may comprise:
contacting a biological sample with an affinity reagent that is specific for DLL3, said affinity reagent optionally being conjugated to a detectable label; and
detecting whether binding has occurred between the affinity reagent and at least one species in the sample, said detection being performed either directly or indirectly.

Alternatively the presence of DLL3, or one or more fragments thereof, or the presence of nucleic acid encoding DLL3 or the presence of the activity of DLL3 may be detected quantitatively by means involving use of an imaging technology.

In another embodiment, the method of the invention involves use of immunohistochemistry on e.g. lung, pancreas and skin tissue sections in order to determine the presence of DLL3, or one or more fragments thereof, or the presence of nucleic acid encoding DLL3 or the presence of the activity of DLL3, and thereby to localise e.g. the diseases of the invention cells.

In one embodiment the presence of DLL3 or one or more epitope-containing fragments thereof is detected, for example using an affinity reagent capable of specific binding to DLL3 or one or more fragments thereof, such as an antibody.

In another embodiment the activity of DLL3 is detected.

### Use in Clinical Studies

The diagnostic methods and compositions of the present invention can assist in monitoring a clinical study, e.g. to evaluate drugs for therapy of the diseases of the invention. In one embodiment, candidate molecules are tested for their ability to restore DLL3 levels in a subject having e.g. the diseases of the invention to levels found in subjects free from the diseases of the invention or, in a treated subject, to preserve DLL3 levels at or near non-lung cancer, non-pancreatic cancer or non-skin cancer values.

In another embodiment, the methods and compositions of the present invention are used to screen candidates for a clinical study to identify individuals having e.g. the diseases of the invention; such individuals can then be excluded from the study or can be placed in a separate cohort for treatment or analysis.

### Production of Protein of the Invention and Corresponding Nucleic Acid

In one aspect the invention provides a method of treating or preventing e.g. the diseases of the invention, comprising administering to a subject in need of such treatment or prevention a therapeutically effective amount of nucleic acid encoding DLL3 or one or more fragments or derivatives thereof, for example in the form of a vaccine.

In another aspect there is provided a method of treating or preventing e.g. the diseases of the invention comprising administering to a subject in need of such treatment or prevention a therapeutically effective amount of nucleic acid that inhibits the function or expression of DLL3.

The methods (and/or other DNA aspects disclosed herein) of the invention may, for example include wherein the nucleic acid is a DLL3 anti-sense nucleic acid or ribozyme.

Thus the invention includes the use of nucleic acid encoding DLL3 or one or more fragments or derivatives thereof, in the manufacture of a medicament for treating or preventing e.g. the diseases of the invention.

There is also provided the use of nucleic acid that inhibits the function or expression of DLL3 in the manufacture of a medicament for treating or preventing e.g. one or more of the diseases of the invention.

A DNA employed in the present invention can be obtained by isolation as a cDNA fragment from cDNA libraries using as starter materials commercial mRNAs and determining and identifying the nucleotide sequences thereof. That is, specifically, clones are randomly isolated from cDNA libraries, which are prepared according to Ohara et al.'s method (DNA Research Vol.4, 53-59 (1997)). Next, through hybridization, duplicated clones (which appear repeatedly) are removed and then *in vitro* transcription and translation are carried out. Nucleotide sequences of both termini of clones, for which products of 50 kDa or more are confirmed, are determined.

Furthermore, databases of known genes are searched for homology using the thus obtained terminal nucleotide sequences as queries.

In addition to the above screening method, the 5' and 3' terminal sequences of cDNA are related to a human genome sequence. Then an unknown long-chain gene is confirmed in a region between the sequences, and the full-length of the cDNA is analyzed. In this way, an unknown gene that is unable to be obtained by a conventional cloning method that depends on known genes can be systematically cloned.

Moreover, all of the regions of a human-derived gene containing a DNA of the present invention can also be prepared using a PCR method such as RACE while paying sufficient attention to prevent artificial errors from taking place in short fragments or obtained sequences. As described above, clones having DNA of the present invention can be obtained.

In another means for cloning DNA of the present invention, a synthetic DNA primer having an appropriate nucleotide sequence of a portion of a polypeptide of the present invention is produced, followed by amplification by the PCR method using an appropriate library. Alternatively, selection can be carried out by hybridization of the DNA of the present invention with a DNA that has been incorporated into an appropriate vector and labelled with a DNA fragment or a synthetic DNA encoding some or all of the regions of the polypeptide of the present invention. Hybridization can be carried out by, for example, the method described in Current Protocols in Molecular Biology (edited by Frederick M. Ausubel et al., 1987). DNA of the present invention may be any DNA, as long as they contain nucleotide sequences encoding the polypeptides of the present invention as described above. Such a DNA may be a cDNA identified and isolated from cDNA libraries or the like that are derived from lung, pancreas and skin tissue. Such a DNA may also be a synthetic DNA or the like. Vectors for use in library construction may be any of bacteriophages, plasmids, cosmids, phargemids, or the like. Furthermore, by the use of a total RNA fraction or a mRNA fraction prepared from the above cells and/or tissues, amplification can be carried out by a direct reverse transcription coupled polymerase chain reaction (hereinafter abbreviated as "RT-PCR method").

DNA encoding the above polypeptide consisting of an amino acid sequence that is substantially identical to the amino acid sequence of DLL3 or DNA encoding the above polypeptide consisting of an amino acid sequence derived from the amino acid sequence of DLL3 by deletion, substitution, or addition of one or more amino acids composing a portion of the amino acid sequence can be easily produced by an appropriate combination of, for example, a site-directed mutagenesis method, a gene homologous recombination method, a primer elongation method, and the PCR method known by persons skilled in the art. In addition, at this time, a possible method for causing a polypeptide to have substantially equivalent biological activity is substitution of homologous amino acids (e.g. polar and nonpolar amino acids, hydrophobic and hydrophilic amino acids, positively-charged and negatively charged amino acids, and aromatic amino acids) among amino acids composing the polypeptide. Furthermore, to maintain substantially equivalent biological activity, amino acids within functional domains contained in the polypeptide of the present invention are preferably conserved.

Furthermore, examples of DNA of the present invention include DNA comprising a nucleotide sequence that encodes the amino acid sequence of DLL3 and DNA hybridizing under stringent conditions to the DNA and encoding a polypeptide (protein) having biological activity (function) equivalent to the function of the polypeptide consisting of the amino acid sequence of DLL3. Under such conditions, an example of such DNA capable of hybridizing to DNA comprising the nucleotide sequence that encodes the amino acid sequence of DLL3 is DNA comprising a nucleotide sequence that has a degree of overall mean homology with the entire nucleotide sequence of the DNA, such as approximately 80% or more, preferably approximately 90% or more, and more preferably approximately 95% or more. Hybridization can be carried out according to a method known in the art such as a method described in Current Protocols in Molecular Biology (edited by Frederick M. Ausubel et al., 1987) or a method according thereto. Here, "stringent conditions" are, for example, conditions of approximately "1*SSC, 0.1% SDS, and 37°C, more stringent conditions of approximately "0.5*SSC, 0.1% SDS, and 42°C, or even more stringent conditions of approximately "0.2*SSC, 0.1% SDS, and 65°C. With more stringent hybridization conditions, the isolation of a DNA having high homology with a probe sequence can be expected. The above combinations of SSC, SDS, and temperature conditions are given for illustrative purposes. Stringency similar to the above can be achieved by persons skilled in the art using an appropriate combination of the above factors or other factors (for example, probe concentration, probe length, and reaction time for hybridization) for determination of hybridization stringency.

A cloned DNA of the present invention can be directly used or used, if desired, after digestion with a restriction enzyme or addition of a linker, depending on purposes. The DNA may have ATG as a translation initiation codon at the 5' terminal side and have TAA, TGA, or TAG as a translation termination codon at the 3' terminal side. These translation initiation and translation termination codons can also be added using an appropriate synthetic DNA adapter.

In the methods/uses of the invention, DLL3 may for example be provided in isolated form, such as where the DLL3 polypeptide has been purified to at least to some extent. DLL3 polypeptide may be provided in substantially pure form, that is to say free, to a substantial extent, from other proteins. DLL3 polypeptide can also be produced using recombinant methods, synthetically produced or produced by a combination of these methods. DLL3 can be easily prepared by any method known by persons skilled in the art, which involves producing an expression vector containing appropriate DNA of the present invention or a gene containing a DNA of the present invention, culturing a transformant transformed using the expression vector, generating and accumulating a relevant polypeptide of the present invention or a recombinant protein containing the polypeptide, and then collecting the resultant.

Recombinant DLL3 polypeptide may be prepared by processes well known in the art from genetically engineered host cells comprising expression systems. Accordingly, the present invention also relates to expression systems which comprise a DLL3 polypeptide or nucleic acid, to host cells which are genetically engineered with such expression systems and to the production of DLL3 polypeptide by recombinant techniques. For recombinant DLL3 polypeptide production, host cells can be genetically engineered to incorporate expression systems or portions thereof for nucleic acids. Such incorporation can be performed using methods well known in the art, such as, calcium phosphate transfection, DEAD-dextran mediated transfection, transvection, microinjection, cationic lipid-mediated transfection, electroporation, transduction, scrape loading, ballistic introduction or infection (see e.g. Davis et al., Basic Methods in Molecular Biology, 1986 and Sambrook et al., Molecular Cloning: A Laboratory Manual, 2nd Ed., Cold Spring Harbour laboratory Press, Cold Spring Harbour, NY, 1989).

As host cells, for example, bacteria of the genus *Escherichia, Streptococci, Staphylococci, Streptomyces,* bacteria of the genus *Bacillus,* yeast, *Aspergillus* cells, insect cells, insects, and animal cells are used. Specific examples of bacteria of the genus *Escherichia,* which are used herein, include *Escherichia coli* K12 and DH1 (Proc. Natl. Acad. Sci. U.S.A., Vol. 60, 160 (1968)), JM103 (Nucleic Acids Research, Vol. 9, 309 (1981)), JA221 (Journal of Molecular Biology, Vol. 120, 517 (1978)), and HB101 (Journal of Molecular Biology, Vol. 41, 459 (1969)). As bacteria of the genus Bacillus, for example, Bacillus subtilis MI114 (Gene, Vol. 24, 255 (1983)) and 207-21 (Journal of Biochemistry, Vol. 95, 87 (1984)) are used. As yeast, for example, *Saccaromyces cerevisiae* AH22, AH22R-, NA87-11A, DKD-5D, and 20B-12, *Schizosaccaromyces pombe* NCYC1913 and NCYC2036, and *Pichia pastoris* are used. As insect cells, for example, Drosophila S2 and Spodoptera Sf9 cells are used. As animal cells, for example, COS-7 and Vero monkey cells, CHO Chinese hamster cells (hereinafter abbreviated as CHO cells), dhfr-gene-deficient CHO cells, mouse L cells, mouse AtT-20 cells, mouse myeloma cells, rat GH3 cells, human FL cells, COS, HeLa, C127,3T3, HEK 293, BHK and Bowes melanoma cells are used.

Cell-free translation systems can also be employed to produce recombinant polypeptides (e.g. rabbit reticulocyte lysate, wheat germ lysate, SP6/T7 in vitro T&T and RTS 100 *E. Coli* HY transcription and translation kits from Roche Diagnostics Ltd., Lewes, UK and the TNT Quick coupled Transcription/Translation System from Promega UK, Southampton, UK).

The expression vector can be produced according to a method known in the art. For example, the vector can be produced by (1) excising a DNA fragment containing a DNA of the present invention or a gene containing a DNA of the present invention and (2) ligating the DNA fragment downstream of the promoter in an appropriate expression vector. A wide variety of expression systems can be used, such as and without limitation, chromosomal, episomal and virus-derived systems, e.g. plasmids derived from Escherichia coli (e.g. pBR322, pBR325, pUC18, and pUC118), plasmids derived from Bacillus subtilis (e.g. pUB110, pTP5, and pC194), from bacteriophage, from transposons, from yeast episomes (e.g. pSH19 and pSH15), from insertion elements, from yeast chromosomal elements, from viruses such as baculoviruses, papova viruses such as SV40, vaccinia viruses, adenoviruses, fowl pox viruses, pseudorabies viruses and retroviruses, and vectors derived from combinations thereof, such as those derived from plasmid and bacteriophage (such as [lambda] phage) genetic elements, such as cosmids and phagemids. The expression systems may contain control regions that regulate as well as engender expression. Promoters to be used in the present invention may be any promoters as long as they are appropriate for hosts to be used for gene expression. For example, when a host is *Escherichia coli,* a trp promoter, a lac promoter, a recA promoter, a pL promoter, an lpp promoter, and the like are preferred. When a host is *Bacillus subtilis,* an SPO1 promoter, an SPO2 promoter, a penP promoter, and the like are preferred. When a host is yeast, a PHO5 promoter, a PGK promoter, a GAP promoter, an ADH promoter, and the like are preferred. When an animal cell is used as a host, examples of promoters for use in this case include an SRa promoter, an SV40 promoter, an LTR promoter, a CMV promoter, and an HSV-TK promoter. Generally, any system or vector that is able to maintain, propagate or express a nucleic acid to produce a polypeptide in a host may be used.

The appropriate nucleic acid sequence may be inserted into an expression system by any variety of well known and routine techniques, such as those set forth in Sambrook *et al.,* supra. Appropriate secretion signals may be incorporated into the DLL3 polypeptide to allow secretion of the translated protein into the lumen of the endoplasmic reticulum, the periplasmic space or the extracellular environment. These signals may be endogenous to the DLL3 polypeptide or they may be heterologous signals. Transformation of the host cells can be carried out according to methods known in the art. For example, the following documents can be referred to: Proc. Natl. Acad. Sci. U.S.A., Vol. 69, 2110 (1972); Gene, Vol. 17, 107 (1982); Molecular & General Genetics, Vol. 168, 111 (1979); Methods in Enzymology, Vol. 194, 182-187 (1991); Proc. Natl. Acad. Sci. U.S.A.), Vol. 75, 1929 (1978); Cell Technology, separate volume 8, New Cell Technology, Experimental Protocol. 263-267 (1995) (issued by Shujunsha); and Virology, Vol. 52, 456 (1973). The thus obtained transformant transformed with an expression vector containing a DNA of the present invention or a gene containing a DNA of the present invention can be cultured according to a method known in the art. For example, when hosts are bacteria of the genus *Escherichia,* the bacteria are generally cultured at approximately 15°C to 43°C for approximately 3 to 24 h. If necessary, aeration or agitation can also be added. When hosts are bacteria of the genus *Bacillus,* the bacteria are generally cultured at approximately 30°C to 40°C for approximately 6 to 24 h. If necessary, aeration or agitation can also be added. When transformants whose hosts are yeast are cultured, culture is generally carried out at approximately 20°C to 35°C for approximately 24 to 72 h using media with pH adjusted to be approximately 5 to 8. If necessary, aeration or agitation can also be added. When transformants whose hosts are animal cells are cultured, the cells are generally cultured at approximately 30°C to 40°C for approximately 15 to 60 h using media with the pH adjusted to be approximately 6 to 8. If necessary, aeration or agitation can also be added.

If a DLL3 polypeptide is to be expressed for use in cell-based screening assays, it is preferred that the polypeptide be produced at the cell surface. In this event, the cells may be harvested prior to use in the screening assay. If the DLL3 polypeptide is secreted into the medium, the medium can be recovered in order to isolate said polypeptide. If produced intracellularly, the cells must first be lysed before the DLL3 polypeptide is recovered.

DLL3 polypeptide can be recovered and purified from recombinant cell cultures or from other biological sources by well known methods including, ammonium sulphate or ethanol precipitation, acid extraction, anion or cation exchange chromatography, phosphocellulose chromatography, affinity chromatography, hydrophobic interaction chromatography, hydroxylapatite chromatography, molecular sieving chromatography, centrifugation methods, electrophoresis methods and lectin chromatography. In one embodiment, a combination of these methods is used. In another embodiment, high performance liquid chromatography is used. In a further embodiment, an antibody which specifically binds to a DLL3 polypeptide can be used to deplete a sample comprising a DLL3 polypeptide of said polypeptide or to purify said polypeptide.

To separate and purify a polypeptide or a protein of the present invention from the culture products, for example, after culture, microbial bodies or cells are collected by a known method, they are suspended in an appropriate buffer, the microbial bodies or the cells are disrupted by, for example, ultrasonic waves, lysozymes, and/or freeze-thawing, the resultant is then subjected to centrifugation or filtration, and then a crude extract of the protein can be obtained. The buffer may also contain a protein denaturation agent such as urea or guanidine hydrochloride or a surfactant such as Triton X-100(TM). When the protein is secreted in a culture solution, microbial bodies or cells and a supernatant are separated by a known method after the completion of culture and then the supernatant is collected. The protein contained in the thus obtained culture supernatant or the extract can be purified by an appropriate combination of known separation and purification methods. The thus obtained polypeptide (protein) of the present invention can be converted into a salt by a known method or a method according thereto. Conversely, when the polypeptide (protein) of the present invention is obtained in the form of a salt, it can be converted into a free protein or peptide or another salt by a known method or a method according thereto. Moreover, an appropriate protein modification enzyme such as trypsin or chymotrypsin is caused to act on a protein produced by a recombinant before or after purification, so that modification can be arbitrarily added or a polypeptide can be partially removed. The presence of a polypeptide (protein) of the present invention or a salt thereof can be measured by various binding assays, enzyme immunoassays using specific antibodies, and the like.

Techniques well known in the art may be used for refolding to regenerate native or active conformations of the DLL3 polypeptide when the polypeptide has been denatured during isolation and or purification. In the context of the present invention, DLL3 polypeptide can be obtained from a biological sample from any source, such as and without limitation, a blood sample or tissue sample, e.g. a lung, pancreas and skin tissue sample.

DLL3 polypeptide may be in the form of a "mature protein" or may be part of a larger protein such as a fusion protein. It is often advantageous to include an additional amino acid sequence which contains secretory or leader sequences, a pre-, pro- or prepro-protein sequence, or a sequence which aids in purification such as an affinity tag, for example, but without limitation, multiple histidine residues, a FLAG tag, HA tag or myc tag.

DLL3 may, for example, be fused with a heterologous fusion partner such as the surface protein, known as protein D from Haemophilus Influenza B, a non-structural protein from influenzae virus such as NS 1, the S antigen from Hepatitis B or a protein known as LYTA such as the C terminal thereof.

An additional sequence that may provide stability during recombinant production may also be used. Such sequences may be optionally removed as required by incorporating a cleavable sequence as an additional sequence or part thereof. Thus, a DLL3 polypeptide may be fused to other moieties including other polypeptides or proteins (for example, glutathione S-transferase and protein A). Such a fusion protein can be cleaved using an appropriate protease, and then separated into each protein. Such additional sequences and affinity tags are well known in the art. In addition to the above, features known in the art, such as an enhancer, a splicing signal, a polyA addition signal, a selection marker, and an SV40 replication origin can be added to an expression vector, if desired.

In one aspect the invention provides an agent capable of specific binding to DLL3, or a fragment thereof, or a hybridising agent capable of hybridizing to nucleic acid encoding DLL3 or an agent capable of detecting the activity of DLL3 for use in treating, screening for, detecting and/or diagnosing disease, such as cancer, and especially the diseases of the invention.

### Production of Affinity Reagents to DLL3

In one aspect, the invention provides an affinity or immunoaffinity reagent which is capable of specific binding to DLL3 or a fragment thereof, for example an affinity reagent which contains or is conjugated to a detectable label or contains or is conjugated to a therapeutic moiety, such as a cytotoxic moiety. The affinity agent may, for example, be an antibody. The affinity reagent may be an isolated affinity reagent or a purified affinity reagent.

The affinity reagent for use in the invention may bind to an epitope on DLL3, e.g. one or more of the portions of any of SEQ ID NO: 1 or 2. Preferably, the affinity reagent specifically binds to the extracellular domain (e.g. the extracellular tail or extracellular loop) of DLL3 (e.g. to SEQ ID NO: 12).

According to those in the art, there are three main types of immunoaffinity reagent - monoclonal antibodies, phage display antibodies and smaller antibody-derived molecules such as Affibodies, Domain Antibodies (dAbs), Nanobodies, UniBodies, DARPins, Anticalins, Duocalins, Avimers or Versabodies. In general in applications according to the present invention where the use of antibodies is stated, other affinity reagents (e.g. Affibodies, Domain Antibodies, Nanobodies, UniBodies, DARPins, Anticalins, Duocalins, Avimers or Versabodies) may be employed. Such substances may be said to be capable of immunospecific binding to DLL3. Where appropriate the term "affinity agent" shall be construed to embrace immunoaffinity reagents and other substances capable of specific binding to DLL3 including but not limited to ligands, lectins, streptavidins, antibody mimetics and synthetic binding agents.

### Production of Antibodies to DLL3

According to the invention DLL3, a DLL3 analog, a DLL3-related protein or a fragment or derivative of any of the foregoing may be used as an immunogen to generate antibodies which immunospecifically bind such an immunogen. Such immunogens can be isolated by any convenient means, including the methods described above. The term "antibody" as used herein refers to a peptide or polypeptide derived from, modeled after or substantially encoded by an immunoglobulin gene or immunoglobulin genes, or fragments thereof, capable of specifically binding an antigen or epitope. See, e.g. Fundamental Immunology, 3rd Edition, W.E. Paul, ed., Raven Press, N.Y. (1993); Wilson (1994) J. Immunol. Methods 175:267-273; Yarmush (1992) J. Biochem. Biophys. Methods 25:85-97. The term antibody includes antigen-binding portions, i.e., "antigen binding sites" (e.g. fragments, subsequences, complementarity determining regions (CDRs)) that retain capacity to bind antigen, including (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody". Antibodies of the invention include, but are not limited to polyclonal, monoclonal, bispecific, humanized or chimeric antibodies, single chain antibodies, Fab fragments and F(ab')₂ fragments, fragments produced by a Fab expression library, anti-idiotypic (anti-Id) antibodies, and epitope-binding fragments of any of the above. The immunoglobulin molecules of the invention can be of any class (e.g. IgG, IgE, IgM, IgD and IgA such as IgG) or subclass of immunoglobulin molecule.

The term "specifically binds" or "binds specifically" (or "immunospecifically binds") is not intended to indicate that an antibody binds exclusively to its intended target. Rather, an antibody "specifically binds" if its affinity for its intended target is typically about 5-fold greater when compared to its affinity for a non-target molecule. Suitably there is no significant cross-reaction or cross-binding with undesired substances, especially naturally occurring proteins or tissues of a healthy person or animal. Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In some embodiments, specific binding between an antibody or other binding agent and an antigen means a binding affinity of at least 10⁶ M⁻¹. Antibodies may, for example, bind with affinities of at least about 10⁷ M⁻¹, and preferably between about 10⁸ M⁻¹ to about 10⁹ M⁻¹, about 10⁹ M⁻¹ to about 10¹⁰ M⁻¹, or about 10¹⁰ M⁻¹ to about 10¹¹ M⁻¹.

Affinity is calculated as K_{d} =k_{off} /kₒₙ (k_{off} is the dissociation rate constant, kₒₙ is the association rate constant and K_{d} is the equilibrium constant. Affinity can be determined at equilibrium by measuring the fraction bound (r) of labelled ligand at various concentrations (c). The data are graphed using the Scatchard equation: r/c = K(n-r):
where
r = moles of bound ligand/mole of receptor at equilibrium;
c = free ligand concentration at equilibrium;
K = equilibrium association constant; and
n = number of ligand binding sites per receptor molecule
By graphical analysis, r/c is plotted on the Y-axis versus r on the X-axis thus producing a Scatchard plot. The affinity is the negative slope of the line. k_{off} can be determined by competing bound labelled ligand with unlabelled excess ligand (see, e.g. U.S. Pat No. 6,316,409). The affinity of a targeting agent for its target molecule is for example at least about 1 x 10⁻⁶ moles/liter, such as at least about 1 x 10⁻⁷ moles/liter, such as at least about 1 x 10⁻⁸ moles/liter, especially at least about 1 x 10⁻⁹ moles/liter, and particularly at least about 1 x 10⁻¹⁰ moles/liter. Antibody affinity measurement by Scatchard analysis is well known in the art, see, e.g. van Erp et al., J. Immunoassay 12: 425-43, 1991; Nelson and Griswold, Comput. Methods Programs Biomed. 27: 65-8, 1988.

In one embodiment, any publicly available antibodies that recognize gene products of genes encoding DLL3 may be used. In another embodiment, methods known to those skilled in the art are used to produce antibodies that recognize DLL3, a DLL3 analog, a DLL3-related polypeptide, or a fragment or derivative of any of the foregoing. One skilled in the art will recognize that many procedures are available for the production of antibodies, for example, as described in Antibodies, A Laboratory Manual, Ed Harlow and David Lane, Cold Spring Harbor Laboratory (1988), Cold Spring Harbor, N.Y. One skilled in the art will also appreciate that binding fragments or Fab fragments which mimic antibodies can also be prepared from genetic information by various procedures (Antibody Engineering: A Practical Approach (Borrebaeck, C., ed.), 1995, Oxford University Press, Oxford; J. Immunol. 149, 3914-3920 (1992)).

In one embodiment of the invention, antibodies to a specific domain of DLL3 are produced. In a specific embodiment, hydrophilic fragments of DLL3 are used as immunogens for antibody production.

In the production of antibodies, screening for the desired antibody can be accomplished by techniques known in the art, e.g. ELISA (enzyme-linked immunosorbent assay). For example, to select antibodies which recognize a specific domain of DLL3, one may assay generated hybridomas for a product which binds to a DLL3 fragment containing such domain. For selection of an antibody that specifically binds a first DLL3 homolog but which does not specifically bind to (or binds less avidly to) a second DLL3 homolog, one can select on the basis of positive binding to the first DLL3 homolog and a lack of binding to (or reduced binding to) the second DLL3 homolog. Similarly, for selection of an antibody that specifically binds DLL3 but which does not specifically bind to (or binds less avidly to) a different isoform of the same protein (such as a different glycoform having the same core peptide as DLL3), one can select on the basis of positive binding to DLL3 and a lack of binding to (or reduced binding to) the different isoform (e.g. a different glycoform). Thus, the present invention provides an antibody (such as a monoclonal antibody) that binds with greater affinity (for example at least 2-fold, such as at least 5-fold, particularly at least 10-fold greater affinity) to DLL3 than to a different isoform or isoforms (e.g. glycoforms) of DLL3.

Polyclonal antibodies which may be used in the methods of the invention are heterogeneous populations of antibody molecules derived from the sera of immunized animals. Unfractionated immune serum can also be used. Various procedures known in the art may be used for the production of polyclonal antibodies to DLL3, a fragment of DLL3, a DLL3-related polypeptide, or a fragment of a DLL3-related polypeptide. For example, one way is to purify polypeptides of interest or to synthesize the polypeptides of interest using, e.g. solid phase peptide synthesis methods well known in the art. See, e.g. Guide to Protein Purification, Murray P. Deutcher, ed., Meth. Enzymol. Vol 182 (1990); Solid Phase Peptide Synthesis, Greg B. Fields ed., Meth. Enzymol. Vol 289 (1997); Kiso et al., Chem. Pharm. Bull. (Tokyo) 38: 1192-99, 1990; Mostafavi et al., Biomed. Pept. Proteins Nucleic Acids 1: 255-60, 1995; Fujiwara et al., Chem. Pharm. Bull. (Tokyo) 44: 1326-31, 1996. The selected polypeptides may then be used to immunize by injection various host animals, including but not limited to rabbits, mice, rats, etc., to generate polyclonal or monoclonal antibodies. If DLL3 is purified by gel electrophoresis, DLL3 can be used for immunization with or without prior extraction from the polyacrylamide gel. Various adjuvants (i.e. immunostimulants) may be used to enhance the immunological response, depending on the host species, including, but not limited to, complete or incomplete Freund's adjuvant, a mineral gel such as aluminum hydroxide, surface active substance such as lysolecithin, pluronic polyol, a polyanion, a peptide, an oil emulsion, keyhole limpet hemocyanin, dinitrophenol, and an adjuvant such as BCG (bacille Calmette-Guerin) or corynebacterium parvum. Additional adjuvants are also well known in the art.

For preparation of monoclonal antibodies (mAbs) directed toward DLL3, a fragment of DLL3, a DLL3-related polypeptide, or a fragment of a DLL3-related polypeptide, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in Monoclonal Antibodies and Cancer Therapy, Alan R. Liss, Inc., pp. 77-96). Such antibodies may be of any immunoglobulin class including IgG, IgM, IgE, IgA, IgD and any subclass thereof. The hybridoma producing the mAbs of the invention may be cultivated *in vitro* or *in vivo.* In an additional embodiment of the invention, monoclonal antibodies can be produced in germ-free animals utilizing known technology (PCT/US90/02545, incorporated herein by reference).

The monoclonal antibodies include but are not limited to human monoclonal antibodies and chimeric monoclonal antibodies (e.g. human-mouse chimeras). A chimeric antibody is a molecule in which different portions are derived from different animal species, such as those having a human immunoglobulin constant region and a variable region derived from a murine mAb, (see, e.g. Cabilly et al., U.S. Patent No. 4,816,567; and Boss et al., U.S. Patent No. 4,816,397, which are incorporated herein by reference in their entirety.) Humanized antibodies are antibody molecules from non-human species having one or more complementarity determining regions (CDRs) from the non-human species and a framework region from a human immunoglobulin molecule, (see, e.g. Queen, U.S. Patent No. 5,585,089, which is incorporated herein by reference in its entirety.)

Chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Canc. Res. 47:999-1005; Wood et al., 1985, Nature 314:446-449; and Shaw et al., 1988, J. Natl. Cancer Inst. 80:1553-1559; Morrison, 1985, Science 229:1202-1207; Oi et al., 1986, BioTechniques 4:214; U.S. Patent 5,225,539; Jones et al., 1986, Nature 321:552-525; Verhoeyan et al. (1988) Science 239:1534; and Beidler et al., 1988, J. Immunol. 141:4053-4060.

Completely human antibodies are particularly desirable for therapeutic treatment of human subjects. Such antibodies can be produced using transgenic mice which are incapable of expressing endogenous immunoglobulin heavy and light chain genes, but which can express human heavy and light chain genes. The transgenic mice are immunized in the normal fashion with a selected antigen, e.g. all or a portion of DLL3. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, *see, e.g.* U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA) and Genpharm (San Jose, CA) can be engaged to provide human antibodies directed against a selected antigen using technology similar to that described above.

Completely human antibodies which recognize a selected epitope can be generated using a technique referred to as "guided selection". In this approach a selected non-human monoclonal antibody, e.g. a mouse antibody, is used to guide the selection of a completely human antibody recognizing the same epitope. (Jespers et al. (1994) BioTechnology 12:899-903).

The antibodies of the present invention can also be generated by the use of phage display technology to produce and screen libraries of polypeptides for binding to a selected target. See, e.g. Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Patent No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. See, e.g. U.S. Patent No. 6,057,098, which is hereby incorporated in its entirety, including all tables, figures, and claims. In particular, such phage can be utilized to display antigen binding domains expressed from a repertoire or combinatorial antibody library (e.g. human or murine). Phage expressing an antigen binding domain that binds the antigen of interest can be selected or identified with antigen, *e.g.* using labelled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Phage display methods that can be used to make the antibodies of the present invention include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT Application No. PCT/GB91/01134; PCT Publications WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108; each of which is incorporated herein by reference in its entirety.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g. as described in detail below. For example, techniques to recombinantly produce Fab, Fab' and F(ab')₂ fragments can also be employed using methods known in the art such as those disclosed in PCT publication WO 92/22324; Mullinax et al., BioTechniques 12(6):864-869 (1992); and Sawai et al., AJRI 34:26-34 (1995); and Better et al., Science 240:1041-1043 (1988) (said references incorporated by reference in their entireties).

Examples of techniques which can be used to produce single-chain Fvs and antibodies include those described in U.S. Patents 4,946,778 and 5,258,498; Huston et al., Methods in Enzymology 203:46-88 (1991); Shu et al., PNAS 90:7995-7999 (1993); and Skerra et al., Science 240:1038-1040 (1988).

The invention further provides for the use of bispecific antibodies, which can be made by methods known in the art. Traditional production of full length bispecific antibodies is based on the coexpression of two immunoglobulin heavy chain-light chain pairs, where the two chains have different specificities (Milstein et al., 1983, Nature 305:537-539). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, published 13 May 1993, and in Traunecker et al., 1991, EMBO J. 10:3655-3659.

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690 published March 3, 1994. For further details for generating bispecific antibodies see, for example, Suresh et al., Methods in Enzymology, 1986, 121:210.

The invention provides functionally active fragments, derivatives or analogs of the anti-DLL3 immunoglobulin molecules. Functionally active means that the fragment, derivative or analog is able to elicit anti-anti-idiotype antibodies (*i.e.,* tertiary antibodies) that recognize the same antigen that is recognized by the antibody from which the fragment, derivative or analog is derived. Specifically, in a preferred embodiment the antigenicity of the idiotype of the immunoglobulin molecule may be enhanced by deletion of framework and CDR sequences that are C-terminal to the CDR sequence that specifically recognizes the antigen. To determine which CDR sequences bind the antigen, synthetic peptides containing the CDR sequences can be used in binding assays with the antigen by any binding assay method known in the art.

The present invention provides antibody fragments such as, but not limited to, F(ab')₂ fragments and Fab fragments. Antibody fragments which recognize specific epitopes may be generated by known techniques. F(ab')₂ fragments consist of the variable region, the light chain constant region and the CH1 domain of the heavy chain and are generated by pepsin digestion of the antibody molecule. Fab fragments are generated by reducing the disulfide bridges of the F(ab')₂ fragments. The invention also provides heavy chain and light chain dimers of the antibodies of the invention, or any minimal fragment thereof such as Fvs or single chain antibodies (SCAs) (*e.g.* as described in U.S. Patent 4,946,778; Bird, 1988, Science 242:423-42; Huston et al., 1988, Proc. Natl. Acad. Sci. USA 85:5879-5883; and Ward et al., 1989, Nature 334:544-54), or any other molecule with the same specificity as the antibody of the invention. Single chain antibodies are formed by linking the heavy and light chain fragments of the Fv region via an amino acid bridge, resulting in a single chain polypeptide. Techniques for the assembly of functional Fv fragments in *E. coli* may be used (Skerra et al., 1988, Science 242:1038-1041).

In other embodiments, the invention provides fusion proteins of the immunoglobulins of the invention (or functionally active fragments thereof), for example in which the immunoglobulin is fused via a covalent bond (e.g. a peptide bond), at either the N-terminus or the C-terminus to an amino acid sequence of another protein (or portion thereof, preferably at least 10, 20 or 50 amino acid portion of the protein) that is not the immunoglobulin. Preferably the immunoglobulin, or fragment thereof, is covalently linked to the other protein at the N-terminus of the constant domain. As stated above, such fusion proteins may facilitate purification, increase half-life *in vivo,* and enhance the delivery of an antigen across an epithelial barrier to the immune system.

The immunoglobulins of the invention include analogs and derivatives that are modified, i.e., by the covalent attachment of any type of molecule as long as such covalent attachment does not impair immunospecific binding. For example, but not by way of limitation, the derivatives and analogs of the immunoglobulins include those that have been further modified, e.g. by glycosylation, acetylation, pegylation, phosphylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, etc. Additionally, the analog or derivative may contain one or more non-classical amino acids.

The foregoing antibodies can be used in methods known in the art relating to the localization and activity of DLL3, e.g. for imaging this protein, measuring levels thereof in appropriate physiological samples, in diagnostic methods, etc.

### Production of Affibodies to DLL3

Affibody molecules represent a new class of affinity proteins based on a 58-amino acid residue protein domain, derived from one of the IgG-binding domains of staphylococcal protein A. This three helix bundle domain has been used as a scaffold for the construction of combinatorial phagemid libraries, from which Affibody variants that target the desired molecules can be selected using phage display technology (Nord K, Gunneriusson E, Ringdahl J, Stahl S, Uhlen M, Nygren PA, Binding proteins selected from combinatorial libraries of an α-helical bacterial receptor domain, Nat Biotechnol 1997;15:772-7. Ronmark J, Gronlund H, Uhlen M, Nygren PA, Human immunoglobulin A (IgA)-specific ligands from combinatorial engineering of protein A, Eur J Biochem 2002;269:2647-55.). The simple, robust structure of Affibody molecules in combination with their low molecular weight (6 kDa), make them suitable for a wide variety of applications, for instance, as detection reagents (Ronmark J, Hansson M, Nguyen T, et al, Construction and characterization of Affibody-Fc chimeras produced in Escherichia coli, J Immunol Methods 2002;261:199-211) and to inhibit receptor interactions (Sandstorm K, Xu Z, Forsberg G, Nygren PA, Inhibition of the CD28-CD80 co-stimulation signal by a CD28-binding Affibody ligand developed by combinatorial protein engineering, Protein Eng 2003;16:691-7). Further details of Affibodies and methods of production thereof may be obtained by reference to US Patent No 5831012 which is herein incorporated by reference in its entirety.

Labelled Affibodies may also be useful in imaging applications for determining abundance of Isoforms.

### Production of Domain Antibodies to DLL3

References to antibodies herein embrace references to Domain Antibodies. Domain Antibodies (dAbs) are the smallest functional binding units of antibodies, corresponding to the variable regions of either the heavy (VH) or light (VL) chains of human antibodies. Domain Antibodies have a molecular weight of approximately 13 kDa. Domantis has developed a series of large and highly functional libraries of fully human VH and VL dAbs (more than ten billion different sequences in each library), and uses these libraries to select dAbs that are specific to therapeutic targets. In contrast to many conventional antibodies, Domain Antibodies are well expressed in bacterial, yeast, and mammalian cell systems. Further details of domain antibodies and methods of production thereof may be obtained by reference to US Patent 6,291,158; 6,582,915; 6,593,081; 6,172,197; 6,696,245; US Serial No. 2004/0110941; European patent application No. 1433846 and European Patents 0368684 and 0616640; WO05/035572, WO04/101790, WO04/081026, WO04/058821, WO04/003019 and WO03/002609, each of which is herein incorporated by reference in its entirety.

### Production of Nanobodies to DLL3

Nanobodies are antibody-derived therapeutic proteins that contain the unique structural and functional properties of naturally-occurring heavy-chain antibodies. These heavy-chain antibodies contain a single variable domain (VHH) and two constant domains (C_{H}2 and C_{H}3). Importantly, the cloned and isolated VHH domain is a perfectly stable polypeptide harbouring the full antigen-binding capacity of the original heavy-chain antibody. Nanobodies have a high homology with the V_{H} domains of human antibodies and can be further humanised without any loss of activity. Importantly, Nanobodies have a low immunogenic potential, which has been confirmed in primate studies with Nanobody lead compounds.

Nanobodies combine the advantages of conventional antibodies with important features of small molecule drugs. Like conventional antibodies, Nanobodies show high target specificity, high affinity for their target and low inherent toxicity. However, like small molecule drugs they can inhibit enzymes and readily access receptor clefts. Furthermore, Nanobodies are extremely stable, can be administered by means other than injection (see e.g. WO 04/041867, which is herein incorporated by reference in its entirety) and are easy to manufacture. Other advantages of Nanobodies include recognising uncommon or hidden epitopes as a result of their small size, binding into cavities or active sites of protein targets with high affinity and selectivity due to their unique 3-dimensional, drug format flexibility, tailoring of half-life and ease and speed of drug discovery.

Nanobodies are encoded by single genes and are efficiently produced in almost all prokaryotic and eukaryotic hosts e.g. *E. coli* (see e.g. US 6,765,087, which is herein incorporated by reference in its entirety), moulds (for example *Aspergillus* or *Trichoderma*) and yeast (for example *Saccharomyces, Kluyveromyces, Hansenula* or *Pichia*) (see e.g. US 6,838,254, which is herein incorporated by reference in its entirety). The production process is scalable and multi-kilogram quantities of Nanobodies have been produced. Because Nanobodies exhibit a superior stability compared with conventional antibodies, they can be formulated as a long shelf-life, ready-to-use solution.

The Nanoclone method (see e.g. WO 06/079372, which is herein incorporated by reference in its entirety) is a proprietary method for generating Nanobodies against a desired target, based on automated high-throughout selection of B-cells.

### Production of UniBodies to DLL3

UniBodies are another antibody fragment technology; however this one is based upon the removal of the hinge region of IgG4 antibodies. The deletion of the hinge region results in a molecule that is essentially half the size of traditional IgG4 antibodies and has a univalent binding region rather than the bivalent binding region of IgG4 antibodies. It is also well known that IgG4 antibodies are inert and thus do not interact with the immune system, which may be advantageous for the treatment of diseases where an immune response is not desired, and this advantage is passed onto UniBodies. For example, UniBodies may function to inhibit or silence, but not kill, the cells to which they are bound. Additionally, UniBody binding to cancer cells do not stimulate them to proliferate. Furthermore, because UniBodies are about half the size of traditional IgG4 antibodies, they may show better distribution over larger solid tumours with potentially advantageous efficacy. UniBodies are cleared from the body at a similar rate to whole IgG4 antibodies and are able to bind with a similar affinity for their antigens as whole antibodies. Further details of UniBodies may be obtained by reference to patent WO2007/059782, which is herein incorporated by reference in its entirety.

### Production of DARPins to DLL3

DARPins (Designed Ankyrin Repeat Proteins) are one example of an antibody mimetic DRP (Designed Repeat Protein) technology that has been developed to exploit the binding abilities of non-antibody polypeptides. Repeat proteins such as ankyrin or leucine-rich repeat proteins, are ubiquitous binding molecules, which occur, unlike antibodies, intra- and extracellularly. Their unique modular architecture features repeating structural units (repeats), which stack together to form elongated repeat domains displaying variable and modular target-binding surfaces. Based on this modularity, combinatorial libraries of polypeptides with highly diversified binding specificities can be generated. This strategy includes the consensus design of self-compatible repeats displaying variable surface residues and their random assembly into repeat domains.

DARPins can be produced in bacterial expression systems at very high yields and they belong to the most stable proteins known. Highly specific, high-affinity DARPins to a broad range of target proteins, including human receptors, cytokines, kinases, human proteases, viruses and membrane proteins, have been selected. DARPins having affinities in the single-digit nanomolar to picomolar range can be obtained.

DARPins have been used in a wide range of applications, including ELISA, sandwich ELISA, flow cytometric analysis (FACS), immunohistochemistry (IHC), chip applications, affinity purification or Western blotting. DARPins also proved to be highly active in the intracellular compartment for example as intracellular marker proteins fused to green fluorescent protein (GFP). DARPins were further used to inhibit viral entry with IC₅₀ in the pM range. DARPins are not only ideal to block protein-protein interactions, but also to inhibit enzymes. Proteases, kinases and transporters have been successfully inhibited, most often an allosteric inhibition mode. Very fast and specific enrichments on the tumour and very favorable tumour to blood ratios make DARPins well suited for in vivo diagnostics or therapeutic approaches.

Additional information regarding DARPins and other DRP technologies can be found in US Patent Application Publication No. 2004/0132028, and International Patent Application Publication No. WO02/20565, both of which are hereby incorporated by reference in their entirety.

### Production of Anticalins to DLL3

Anticalins are an additional antibody mimetic technology, however in this case the binding specificity is derived from lipocalins, a family of low molecular weight proteins that are naturally and abundantly expressed in human tissues and body fluids. Lipocalins have evolved to perform a range of functions in vivo associated with the physiological transport and storage of chemically sensitive or insoluble compounds. Lipocalins have a robust intrinsic structure comprising a highly conserved β-barrel which supports four loops at one terminus of the protein. These loops form the entrance to a binding pocket and conformational differences in this part of the molecule account for the variation in binding specificity between individual lipocalins.

While the overall structure of hypervariable loops supported by a conserved β-sheet framework is reminiscent of immunoglobulins, lipocalins differ considerably from antibodies in terms of size, being composed of a single polypeptide chain of 160-180 amino acids which is marginally larger than a single immunoglobulin domain.

Lipocalins are cloned and their loops are subjected to engineering in order to create Anticalins. Libraries of structurally diverse Anticalins have been generated and Anticalin display allows the selection and screening of binding function, followed by the expression and production of soluble protein for further analysis in prokaryotic or eukaryotic systems. Studies have successfully demonstrated that Anticalins can be developed that are specific for virtually any human target protein; they can be isolated and binding affinities in the nanomolar or higher range can be obtained.

Anticalins can also be formatted as dual targeting proteins, so-called Duocalins. A Duocalin binds two separate therapeutic targets in one easily produced monomeric protein using standard manufacturing processes while retaining target specificity and affinity regardless of the structural orientation of its two binding domains.

Modulation of multiple targets through a single molecule is particularly advantageous in diseases known to involve more than a single causative factor. Moreover, bi- or multivalent binding formats such as Duocalins have significant potential in targeting cell surface molecules in disease, mediating agonistic effects on signal transduction pathways or inducing enhanced internalization effects via binding and clustering of cell surface receptors. Furthermore, the high intrinsic stability of Duocalins is comparable to monomeric Anticalins, offering flexible formulation and delivery potential for Duocalins.

Additional information regarding Anticalins can be found in US Patent No. 7,250,297 and International Patent Application Publication No. WO 99/16873, both of which are hereby incorporated by reference in their entirety.

### Production of Avimers to DLL3

Avimers are evolved from a large family of human extracellular receptor domains by in vitro exon shuffling and phage display, generating multidomain proteins with binding and inhibitory properties. Linking multiple independent binding domains has been shown to create avidity and results in improved affinity and specificity compared with conventional single-epitope binding proteins. Other potential advantages include simple and efficient production of multitarget-specific molecules in Escherichia coli, improved thermostability and resistance to proteases. Avimers with sub-nanomolar affinities have been obtained against a variety of targets.

Additional information regarding Avimers can be found in US Patent Application Publication Nos. 2006/0286603, 2006/0234299, 2006/0223114, 2006/0177831, 2006/0008844, 2005/0221384, 2005/0164301, 2005/0089932, 2005/0053973, 2005/0048512, 2004/0175756, all of which are hereby incorporated by reference in their entirety.

### Production of Versabodies to DLL3

Versabodies are small proteins of 3-5 kDa with >15% cysteines, which form a high disulfide density scaffold, replacing the hydrophobic core that typical proteins have. The replacement of a large number of hydrophobic amino acids, comprising the hydrophobic core, with a small number of disulfides results in a protein that is smaller, more hydrophilic (less aggregation and non-specific binding), more resistant to proteases and heat, and has a lower density of T-cell epitopes, because the residues that contribute most to MHC presentation are hydrophobic. All four of these properties are well-known to affect immunogenicity, and together they are expected to cause a large decrease in immunogenicity.

The inspiration for Versabodies comes from the natural injectable biopharmaceuticals produced by leeches, snakes, spiders, scorpions, snails, and anemones, which are known to exhibit unexpectedly low immunogenicity. Starting with selected natural protein families, by design and by screening the size, hydrophobicity, proteolytic antigen processing, and epitope density are minimized to levels far below the average for natural injectable proteins.

Given the structure of Versabodies, these antibody mimetics offer a versatile format that includes multi-valency, multi-specificity, a diversity of half-life mechanisms, tissue targeting modules and the absence of the antibody Fc region. Furthermore, Versabodies are manufactured in E. coli at high yields, and because of their hydrophilicity and small size, Versabodies are highly soluble and can be formulated to high concentrations. Versabodies are exceptionally heat stable (they can be boiled) and offer extended shelf-life.

Additional information regarding Versabodies can be found in US Patent Application Publication No. 2007/0191272 which is hereby incorporated by reference in its entirety.

### Expression of Affinity Reagents

### Expression of Antibodies

The antibodies of the invention can be produced by any method known in the art for the synthesis of antibodies, in particular, by chemical synthesis or by recombinant expression, and are preferably produced by recombinant expression techniques.

Recombinant expression of antibodies, or fragments, derivatives or analogs thereof, requires construction of a nucleic acid that encodes the antibody. If the nucleotide sequence of the antibody is known, a nucleic acid encoding the antibody may be assembled from chemically synthesized oligonucleotides (e.g. as described in Kutmeier et al., 1994, BioTechniques 17:242), which, briefly, involves the synthesis of overlapping oligonucleotides containing portions of the sequence encoding antibody, annealing and ligation of those oligonucleotides, and then amplification of the ligated oligonucleotides by PCR.

Alternatively, the nucleic acid encoding the antibody may be obtained by cloning the antibody. If a clone containing the nucleic acid encoding the particular antibody is not available, but the sequence of the antibody molecule is known, a nucleic acid encoding the antibody may be obtained from a suitable source (e.g. an antibody cDNA library, or cDNA library generated from any tissue or cells expressing the antibody) by PCR amplification using synthetic primers hybridizable to the 3' and 5' ends of the sequence or by cloning using an oligonucleotide probe specific for the particular gene sequence.

If an antibody molecule that specifically recognizes a particular antigen is not available (or a source for a cDNA library for cloning a nucleic acid encoding such an antibody), antibodies specific for a particular antigen may be generated by any method known in the art, for example, by immunizing an animal, such as a rabbit, to generate polyclonal antibodies or, for example, by generating monoclonal antibodies. Alternatively, a clone encoding at least the Fab portion of the antibody may be obtained by screening Fab expression libraries (e.g. as described in Huse et al., 1989, Science 246:1275-1281) for clones of Fab fragments that bind the specific antigen or by screening antibody libraries (see, e.g. Clackson et al., 1991, Nature 352:624; Hane et al., 1997 Proc. Natl. Acad. Sci. USA 94:4937).

Once a nucleic acid encoding at least the variable domain of the antibody molecule is obtained, it may be introduced into a vector containing the nucleotide sequence encoding the constant region of the antibody molecule (see, e.g. PCT Publication WO 86/05807; PCT Publication WO 89/01036; and U.S. Patent No. 5,122,464). Vectors containing the complete light or heavy chain for co-expression with the nucleic acid to allow the expression of a complete antibody molecule are also available. Then, the nucleic acid encoding the antibody can be used to introduce the nucleotide substitution(s) or deletion(s) necessary to substitute (or delete) the one or more variable region cysteine residues participating in an intrachain disulfide bond with an amino acid residue that does not contain a sulfhydyl group. Such modifications can be carried out by any method known in the art for the introduction of specific mutations or deletions in a nucleotide sequence, for example, but not limited to, chemical mutagenesis, in vitro site directed mutagenesis (Hutchinson et al., 1978, J. Biol. Chem. 253:6551), PCT based methods, etc.

In addition, techniques developed for the production of "chimeric antibodies" (Morrison et al., 1984, Proc. Natl. Acad. Sci. USA 81:851-855; Neuberger et al., 1984, Nature 312:604-608; Takeda et al., 1985, Nature 314:452-454) by splicing genes from a mouse antibody molecule of appropriate antigen specificity together with genes from a human antibody molecule of appropriate biological activity can be used. As described *supra,* a chimeric antibody is a molecule in which different portions are derived from different animal species, such as those having a variable region derived from a murine mAb and a human antibody constant region, e.g. humanized antibodies.

Once a nucleic acid encoding an antibody molecule of the invention has been obtained, the vector for the production of the antibody molecule may be produced by recombinant DNA technology using techniques well known in the art. Thus, methods for preparing DLL3 by expressing nucleic acid containing the antibody molecule sequences are described herein. Methods which are well known to those skilled in the art can be used to construct expression vectors containing an antibody molecule coding sequences and appropriate transcriptional and translational control signals. These methods include, for example, *in vitro* recombinant DNA techniques, synthetic techniques, and *in vivo* genetic recombination. See, for example, the techniques described in Sambrook et al. (1990, Molecular Cloning, A Laboratory Manual, 2nd Ed., Cold Spring Harbor Laboratory, Cold Spring Harbor, NY) and Ausubel et al. (eds., 1998, Current Protocols in Molecular Biology, John Wiley & Sons, NY).

The expression vector is transferred to a host cell by conventional techniques and the transfected cells are then cultured by conventional techniques to produce an antibody of the invention.

The host cells used to express a recombinant antibody of the invention may be either bacterial cells such as *Escherichia coli,* or, preferably, eukaryotic cells, especially for the expression of whole recombinant antibody molecule. In particular, mammalian cells such as Chinese hamster ovary cells (CHO), in conjunction with a vector such as the major intermediate early gene promoter element from human cytomegalovirus are an effective expression system for antibodies (Foecking et al., 1986, Gene 45:101; Cockett et al., 1990, BioTechnology 8:2).

A variety of host-expression vector systems may be utilized to express an antibody molecule of the invention. Such host-expression systems represent vehicles by which the coding sequences of interest may be produced and subsequently purified, but also represent cells which may, when transformed or transfected with the appropriate nucleotide coding sequences, express the antibody molecule of the invention *in situ.* These include but are not limited to microorganisms such as bacteria (e.g. *E. coli, B. subtilis*) transformed with recombinant bacteriophage DNA, plasmid DNA or cosmid DNA expression vectors containing antibody coding sequences; yeast (e.g. *Saccharomyces, Pichia*) transformed with recombinant yeast expression vectors containing antibody coding sequences; insect cell systems infected with recombinant virus expression vectors (e.g. baculovirus) containing the antibody coding sequences; plant cell systems infected with recombinant virus expression vectors (e.g. cauliflower mosaic virus, CaMV; tobacco mosaic virus, TMV) or transformed with recombinant plasmid expression vectors (e.g. Ti plasmid) containing antibody coding sequences; or mammalian cell systems (e.g. COS, CHO, BHK, 293, 3T3 cells) harboring recombinant expression constructs containing promoters derived from the genome of mammalian cells (e.g. metallothionein promoter) or from mammalian viruses (e.g. the adenovirus late promoter; the vaccinia virus 7.5K promoter).

In bacterial systems, a number of expression vectors may be advantageously selected depending upon the use intended for the antibody molecule being expressed. For example, when a large quantity of such a protein is to be produced, for the generation of pharmaceutical compositions comprising an antibody molecule, vectors which direct the expression of high levels of fusion protein products that are readily purified may be desirable. Such vectors include, but are not limited, to the E. *coli* expression vector pUR278 (Ruther et al., 1983, EMBO J. 2:1791), in which the antibody coding sequence may be ligated individually into the vector in frame with the *lac Z* coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. The pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter). In mammalian host cells, a number of viral-based expression systems (e.g. an adenovirus expression system) may be utilized.

As discussed above, a host cell strain may be chosen which modulates the expression of the inserted sequences, or modifies and processes the gene product in the specific fashion desired. Such modifications (e.g. glycosylation) and processing (e.g. cleavage) of protein products may be important for the function of the protein.

For long-term, high-yield production of recombinant antibodies, stable expression is preferred. For example, cell lines that stably express an antibody of interest can be produced by transfecting the cells with an expression vector comprising the nucleotide sequence of the antibody and the nucleotide sequence of a selectable (e.g. neomycin or hygromycin), and selecting for expression of the selectable marker. Such engineered cell lines may be particularly useful in screening and evaluation of compounds that interact directly or indirectly with the antibody molecule.

The expression levels of the antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot, 1986, Nature 322:52; Kohler, 1980, Proc. Natl. Acad. Sci. USA 77:2197). The coding sequences for the heavy and light chains may comprise cDNA or genomic DNA.

Once the antibody molecule of the invention has been recombinantly expressed, it may be purified by any method known in the art for purification of an antibody molecule, for example, by chromatography (e.g. ion exchange chromatography, affinity chromatography such as with protein A or specific antigen, and sizing column chromatography), centrifugation, differential solubility, or by any other standard technique for the purification of proteins.

Alternatively, any fusion protein may be readily purified by utilizing an antibody specific for the fusion protein being expressed. For example, a system described by Janknecht et al. allows for the ready purification of non-denatured fusion proteins expressed in human cell lines (Janknecht et al., 1991, Proc. Natl. Acad. Sci. USA 88:8972-897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with recombinant vaccinia virus are loaded onto Ni²⁺ nitriloacetic acid-agarose columns and histidine-tagged proteins are selectively eluted with imidazole-containing buffers.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labelled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) is present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (*e.g.* in sandwich assays) may interfere with one another sterically, etc., assay performance of an antibody may be a more important measure than absolute affinity and specificity of an antibody.

Those skilled in the art will recognize that many approaches can be taken in producing antibodies or binding fragments and screening and selecting for affinity and specificity for the various polypeptides, but these approaches do not change the scope of the invention.

For therapeutic applications, antibodies (particularly monoclonal antibodies) may suitably be human or humanized animal (e.g. mouse) antibodies. Animal antibodies may be raised in animals using the human protein (e.g. DLL3) as immunogen. Humanisation typically involves grafting CDRs identified thereby into human framework regions. Normally some subsequent retromutation to optimize the conformation of chains is required. Such processes are known to persons skilled in the art.

### Expression of Affibodies

The construction of affibodies has been described elsewhere (Ronnmark J, Gronlund H, Uhlen, M., Nygren P.A, Human immunoglobulin A (IgA)-specific ligands from combinatorial engineering of protein A, 2002, Eur. J. Biochem. 269, 2647-2655.), including the construction of Affibody phage display libraries (Nord, K., Nilsson, J., Nilsson, B., Uhlen, M. & Nygren, P.A, A combinatorial library of an a-helical bacterial receptor domain, 1995, Protein Eng. 8, 601-608. Nord, K., Gunneriusson, E., Ringdahl, J., Stahl, S., Uhlen, M. & Nygren, P.A, Binding proteins selected from combinatorial libraries of an a-helical bacterial receptor domain, 1997, Nat. Biotechnol.15, 772-777.)

The biosensor analyses to investigate the optimal Affibody variants using biosensor binding studies has also been described elsewhere (Ronnmark J, Gronlund H, Uhlen, M., Nygren P.A, Human immunoglobulin A (IgA)-specific ligands from combinatorial engineering of protein A, 2002, Eur. J. Biochem. 269, 2647-2655.).

### Affinity Reagent Modifications

In a preferred embodiment, anti-DLL3 affinity reagents such as antibodies or fragments thereof are conjugated to a diagnostic moiety (such as a detectable label) or a therapeutic moiety. The antibodies can be used for diagnosis or to determine the efficacy of a given treatment regimen. Detection can be facilitated by coupling the antibody to a detectable substance (label). Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics according to the present invention. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include ¹²⁵I, ¹³¹I, ¹¹¹In and ⁹⁹Tc. ⁶⁸Ga may also be employed.

As indicated above affinity reagents, such as antibodies for use in the invention, may be conjugated to a therapeutic moiety, such as a cytotoxin, a drug (e.g. an immunosuppressant) or a radiotoxin. Such conjugates are referred to herein as "immunoconjugates". Immunoconjugates that include one or more cytotoxins are referred to as "immunotoxins". A cytotoxin or cytotoxic agent includes any agent that is detrimental to (e.g. kills) cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents also include, for example, antimetabolites (e.g. methotrexate, 6-mercaptopurine, 6-thioguanine, cytarabine, 5-fluorouracil decarbazine), alkylating agents (e.g. mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g. daunorubicin (formerly daunomycin) and doxorubicin), antibiotics (e.g. dactinomycin (formerly actinomycin), bleomycin, mithramycin, and anthramycin (AMC)), and anti-mitotic agents (e.g. vincristine and vinblastine).

Other preferred examples of therapeutic cytotoxins that can be conjugated to an antibody of the invention include duocarmycins, calicheamicins, maytansines and auristatins, and derivatives thereof. An example of a calicheamicin antibody conjugate is commercially available (Mylotarg®; American Home Products).

Cytotoxins can be conjugated to antibodies of the invention using linker technology available in the art. Examples of linker types that have been used to conjugate a cytotoxin to an antibody include, but are not limited to, hydrazones, thioethers, esters, disulfides and peptide-containing linkers. A linker can be chosen that is, for example, susceptible to cleavage by low pH within the lysosomal compartment or susceptible to cleavage by proteases, such as proteases preferentially expressed in tumour tissue such as cathepsins (e.g. cathepsins B, C, D).

Examples of cytotoxins are described, for example, in U.S. Patent Nos. 6,989,452, 7,087,600, and 7,129,261, and in PCT Application Nos. PCT/US2002/17210, PCT/US2005/017804, PCT/US2006/37793, PCT/US2006/060050, PCT/US2006/060711, WO2006/110476, and in U.S. Patent Application No. 60/891,028, all of which are incorporated herein by reference in their entirety. For further discussion of types of cytotoxins, linkers and methods for conjugating therapeutic agents to antibodies, see also Saito, G. et al. (2003) Adv. Drug Deliv. Rev. 55:199-215; Trail, P.A. et al. (2003) Cancer Immunol. Immunother. 52:328-337; Payne, G. (2003) Cancer Cell 3:207-212; Allen, T.M. (2002) Nat. Rev. Cancer 2:750-763; Pastan, I. and Kreitman, R. J. (2002) Curr. Opin. Investig. Drugs 3:1089-1091; Senter, P.D. and Springer, C.J. (2001) Adv. Drug Deliv. Rev. 53:247-264.

Affinity reagents can also be conjugated to a radioactive isotope to generate cytotoxic radiopharmaceuticals, also referred to as radioimmunoconjugates. Examples of radioactive isotopes that can be conjugated to antibodies for use diagnostically or therapeutically include, but are not limited to, iodine 131, indium 111, yttrium90 and lutetium 177. Methods for preparing radioimmunoconjugates are established in the art. Examples of radioimmunoconjugates are commercially available, including Zevalin® (IDEC Pharmaceuticals) and Bexxar® (Corixa Pharmaceuticals), and similar methods can be used to prepare radioimmunoconjugates using the antibodies of the invention.

Affinity reagents can also be conjugated to a phthalocyanine dye referred to hereafter as phthalocyanineconjugates. Examples of phthalocyanine dyes that can be conjugated to antibodies for use diagnostically or therapeutically include, but are not limited to, IR700. Methods for preparing phthalocyanineconjugates are described, for example, in Mitsunaga M, Ogawa M, Kosaka N, Rosenblum LT, Choyke PL and Kobayashi H (2011) Nat Med. 2011 Nov 6. doi: 10.1038/nm.2554.

The conjugates can be used to modify a given biological response, and the drug moiety is not to be construed as limited to classical chemical therapeutic agents. For example, the drug moiety may be a protein or polypeptide possessing a desired biological activity. Such proteins may include, for example, an enzymatically active toxin, or active fragment thereof, such as abrin, ricin A, pseudomonas exotoxin, or diphtheria toxin; a protein such as tumor necrosis factor or interferon-y; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors. Senter P.D. (2009) Curr. Opin. Chem. Biol. 13(3):235-244; Kovtun et al. (2010) Cancer Res. 70(6):2528-2537.

Techniques for conjugating such therapeutic moieties to antibodies are well known, see, e.g. Arnon et al., "Monoclonal Antibodies For Immunotargeting Of Drugs In Cancer Therapy" in Monoclonal Antibodies And Cancer Therapy, Reisfeld et al. (eds.), pp. 243-56 (Alan R. Liss, Inc. 1985); Hellstrom et al., "Antibodies For Drug Delivery," in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review" in Monoclonal Antibodies '84: Biological And Clinical Applications, Pinchera et al. (eds.), pp. 475-506 (1985); "Analysis, Results, And Future Prospective Of The Therapeutic Use Of Radiolabelled Antibody In Cancer Therapy" in Monoclonal Antibodies For Cancer Detection And Therapy, Baldwin et al. (eds.), pp. 303-16 (Academic Press 1985), and Thorpe et al., Immunol. Rev., 62:119-58 (1982).

Alternatively, an antibody can be conjugated to a second antibody to form an antibody heteroconjugate as described by Segal in U.S. Patent No. 4,676,980.

An antibody with or without a therapeutic moiety conjugated to it can be used as a therapeutic that is administered alone or in combination with cytotoxic factor(s) and/or cytokine(s).

The invention also provides for fully human, or humanised antibodies that induce antibody-directed cell-mediated cytotoxicity (ADCC). A fully human antibody is one in which the protein sequences are encoded by naturally occurring human immunoglobulin sequences, either from isolated antibody-producing human B-lymphocytes, or from transgenic murine B-lymphocytes of mice in which the murine immunoglobulin coding chromosomal regions have been replaced by orthologous human sequences. Transgenic antibodies of the latter type include, but are not restricted to, HuMab (Medarex, Inc, CA) and XenoMouse (Abgenix Inc., CA). A humanised antibody is one in which the constant region of a non-human antibody molecule of appropriate antigen specificity, is replaced by the constant region of a human antibody, preferably of the IgG subtype, with appropriate effector functions (Morrison et al., 1984, Proc. Natl. Acad. Sci. 81:851-855; Neuberger et al., 1984, Nature 312:604-608; Takeda et al., 1985, Nature 314:452-454). Appropriate effector functions include ADCC, which is a natural process by which fully-human antibodies or humanized antibodies, when bound to targets on the surface of cancer cells, switch on the cell killing properties of lymphocytes that are part of the normal immune system. These active lymphocytes, called Natural Killer (NK) cells, use a cytotoxic process to destroy living cells to which the antibodies are bound. ADCC activity may be detected and quantified by measuring release of Europium (Eu³⁺) from Eu³⁺ labelled, living cells in the presence of an antigen-specific antibody and peripheral blood mononuclear cells extracted from an immunocompetent, living human subject. The ADCC process is described in detail in Janeway Jr. C.A. et al., Immunobiology, 5th ed., 2001, Garland Publishing, ISBN 0-8153-3642-X; Pier G.B. et al., Immunology, Infection, and Immunity, 2004, p246-5; Albanell J. et al., Advances in Experimental Medicine and Biology, 2003, 532:p2153-68 and Weng, W.-K. et al., Journal of Clinical Oncology, 2003, 21:p 3940-3947. Suitable methods for the detection and quantification of ADCC can be found in Blomberg et al., Journal of Immunological Methods. 1986, 86:p225-9; Blomberg et al., Journal of Immunological Methods. 1986, 21;92:p117-23 and Patel & Boyd, Journal of Immunological Methods. 1995, 184:p29-38.

ADCC typically involves activation of NK cells and is dependent on the recognition of antibody-coated cells by Fc receptors on the surface of the NK cell. The Fc receptors recognize the Fc (crystalline) portion of antibodies such as IgG, bound specifically to the surface of a target cell. The Fc receptor that triggers activation of the NK cell is called CD16 or FcyRIIIa. Once the FcγRIIIa receptor is bound to the IgG Fc, the NK cell releases cytokines such as IFN-γ, and cytotoxic granules containing perforin and granzymes that enter the target cell and promote cell death by triggering apoptosis.

The induction of antibody-dependent cellular cytotoxicity (ADCC) by an antibody can be enhanced by modifications that alter interactions between the antibody constant region (Fc) and various receptors that are present on the surface of cells of the immune system. Such modifications include the reduction or absence of alpha1,6-linked fucose moieties in the complex oligosaccharide chains that are normally added to the Fc of antibodies during natural or recombinant synthesis in mammalian cells. In a preferred embodiment, non-fucosylated anti-DLL3 affinity reagents such as antibodies or fragments thereof are produced for the purpose of enhancing their ability to induce the ADCC response.

Techniques for reducing or ablating alpha 1,6-linked fucose moieties in the oligosaccharide chains of the Fc are well established. In one example, the recombinant antibody is synthesized in a cell line that is impaired in its ability to add fucose in an alpha 1,6 linkage to the innermost N-acetylglucosamine of the N-linked biantennary complex-type Fc oligosaccharides. Such cell lines include, but are not limited to, the rat hybridoma YB2/0, which expresses a reduced level of the alpha 1,6-fucosyltransferase gene, FUT8. Preferably, the antibody is synthesized in a cell line that is incapable of adding alpha 1,6-linked fucosyl moieties to complex oligosaccharide chains, due to the deletion of both copies of the FUT8 gene. Such cell lines include, but are not limited to, FUT8-/- CHO/DG44 cell lines. Techniques for synthesizing partially fucosylated, or non-fucosylated antibodies and affinity reagents are described in Shinkawa et al., J. Biol. Chem. 278:3466-34735 (2003); Yamane-Ohnuki et al., Biotechnology and Bioengineering 87: 614-22 (2004) and in WO00/61739 A1, WO02/31140 A1 and WO03/085107 A1. In a second example, the fucosylation of a recombinant antibody is reduced or abolished by synthesis in a cell line that has been genetically engineered to overexpress a glycoprotein-modifying glycosyl transferase at a level that maximizes the production of complex N-linked oligosaccharides carrying bisecting N-acetylglucosamine. For example, the antibody is synthesized in a Chinese Hamster Ovary cell line expressing the enzyme N-acetyl glucosamine transferase III (GnT III). Cell lines stably transfected with suitable glycoprotein-modifying glycosyl transferases, and methods of synthesizing antibodies using these cells are described in WO99/54342.

A non-fucosylated antibody or affinity reagent can be used as a therapeutic that is administered alone or in combination with cytotoxic factor(s) and/or cytokine(s).

In a further modification, the amino acid sequences of the antibody Fc are altered in a way that enhances ADCC activation, without affecting ligand affinity. Examples of such modifications are described in Lazar et al., Proceedings of the National Academy of Sciences 2006, 103: p4005-4010; WO03/074679 and WO2007/039818. In these examples, substitution of amino acids in the antibody Fc, such as aspartate for serine at position 239, and isoleucine for glutamate at position 332, altered the binding affinity of an antibody for Fc receptors, leading to an increase in ADCC activation.

An antibody reagent with enhanced ADCC activation due to amino acid substitutions can be used as a therapeutic that is administered alone or in combination with cytotoxic factor(s) and/or cytokine(s).

The invention also provides for bispecific molecules comprising at least one first binding specificity for a first target epitope (i.e. DLL3) and a second binding specificity for a second target epitope. The second target epitope maybe present on the same target protein as that bound by the first binding specificity; or the second target epitope may be present of a different target protein to that bound by the first protein to that bound by the first binding specificity. The second target epitope may be present on the same cell as the first target epitope (i.e. DLL3); or the second target epitope may be present on a target which is not displayed by the cell which displays the first target epitope. As used herein, the term 'binding specificity' refers to a moiety comprising at least one antibody variable domain.

In one embodiment, the bispecific molecule is a BiTE (bispecific T-cell engager). In particular, the invention provides a bispecific affinity reagent (preferably a bispecific antibody) which comprises a first binding domain for DLL3 and a second binding domain for a T-cell antigen, preferably CD3.

These bispecific molecules target DLL3 expressing cells to CD3 expressing effector cells (e.g. CD3 expressing cytotoxic T cells) and trigger CD3-mediated effector cell activities, such as T cell clonal expansion and T cell cytotoxicity. The bispecific antibodies of the invention may have a total of either two or three antibody variable domains, wherein first portion of the bispecific antibody is capable of recruiting the activity of a human immune effector cell by specifically binding to an effector antigen located on the human immune effector cell, in which the effector antigen is the human CD3 antigen, said first portion consisting of one antibody variable domain, and a second portion of the bispecific antibody is capable of specifically binding to a target antigen other than the effector antigen e.g. DLL3, said target antigen being located on a target cell other than said human immune effector cell, and said second portion comprising one or two antibody variable domains.

In one preferred embodiment, the invention provides a bispecific antibody (preferably a BiTE) which binds to DLL3 and CD3 for the treatment of lung cancer, preferably small cell lung cancer.

### Diagnosis of Cancer Including the diseases of the invention

According to another aspect of the invention, there is provided a method of detecting, diagnosing and/or screening for or monitoring the progression of cancer e.g. the diseases of the invention or of monitoring the effect of e.g. an anti-cancer drug or therapy directed towards the diseases of the invention in a subject which comprises detecting the presence or level of antibodies capable of immunospecific binding to DLL3, or one or more epitope-containing fragments thereof or which comprises detecting a change in the level thereof in said subject.

According to another aspect of the invention there is also provided a method of detecting, diagnosing and/or screening for cancer e.g. the diseases of the invention in a subject which comprises detecting the presence of antibodies capable of immunospecific binding to DLL3, or one or more epitope-containing fragments thereof in said subject, in which (a) the presence of an elevated level of antibodies capable of immunospecific binding to DLL3 or said one or more epitope-containing fragments thereof in said subject as compared with the level in a healthy subject or (b) the presence of a detectable level of antibodies capable of immunospecific binding to DLL3 or said one or more epitope-containing fragments thereof in said subject as compared with a corresponding undetectable level in a healthy subject indicates the presence of said cancer in said subject.

One particular method of detecting, diagnosing and/or screening for cancer, e.g. the diseases of the invention comprises:
bringing into contact with a biological sample to be tested DLL3, or one or more epitope-containing fragments thereof; and
detecting the presence of antibodies in the subject capable of immunospecific binding to DLL3, or one or more epitope-containing fragments thereof.

According to another aspect of the invention there is provided a method of monitoring the progression of cancer, e.g. the diseases of the invention or of monitoring the effect of e.g. an anti-cancer drug or therapy directed towards the diseases of the invention in a subject which comprises detecting the presence of antibodies capable of immunospecific binding to DLL3, or one or more epitope-containing fragments thereof in said subject at a first time point and at a later time point, the presence of an elevated or lowered level of antibodies capable of immunospecific binding to DLL3, or one or more epitope-containing fragments thereof in said subject at the later time point as compared with the level in said subject at said first time point, indicating the progression or regression of said cancer, or the effect or non-effect of said anti-cancer drug or therapy in said subject.

The presence of antibodies capable of immunospecific binding to DLL3, or one or more epitope-containing fragments thereof is typically detected by analysis of a biological sample obtained from said subject (exemplary biological samples are mentioned above, e.g. the sample is a sample of lung, pancreas and skin tissue, or else a sample of blood or saliva). The method typically includes the step of obtaining said biological sample for analysis from said subject. The antibodies that may be detected include IgA, IgM and IgG antibodies.

In accordance with the present invention, test samples of e.g. lung, pancreas or skin tissue, serum, plasma or urine obtained from a subject suspected of having or known to have the diseases of the invention can be used for diagnosis or monitoring. In one embodiment, a change in the abundance of DLL3 in a test sample relative to a control sample (from a subject or subjects free from the diseases of the invention) or a previously determined reference range indicates the presence of the diseases of the invention. In another embodiment, the relative abundance of DLL3 in a test sample compared to a control sample or a previously determined reference range indicates a subtype of the diseases of the invention (e.g. small cell carcinoma; squamous cell lung carcinoma; endocrine tumours of the pancreas or squamous cell skin carcinoma, melanoma). In yet another embodiment, the relative abundance of DLL3 in a test sample relative to a control sample or a previously determined reference range indicates the degree or severity of the diseases of the invention (e.g. the likelihood for metastasis). In any of the aforesaid methods, detection of DLL3 may optionally be combined with detection of one or more of additional biomarkers for the diseases of the invention. Any suitable method in the art can be employed to measure the level of DLL3, including but not limited to the Preferred Technologies described herein, kinase assays, immunoassays to detect and/or visualize the DLL3 (e.g. Western blot, immunoprecipitation followed by sodium dodecyl sulfate polyacrylamide gel electrophoresis, immunocytochemistry, etc.). In a further embodiment, a change in the abundance of mRNA encoding DLL3 in a test sample relative to a control sample or a previously determined reference range indicates the presence of the diseases of the invention. Any suitable hybridization assay can be used to detect DLL3 expression by detecting and/or visualizing mRNA encoding the DLL3 (e.g. Northern assays, dot blots, *in situ* hybridization, etc.).

In another embodiment of the invention, labelled antibodies (or other affinity reagents), derivatives and analogs thereof, which specifically bind to DLL3 can be used for diagnostic purposes to detect, diagnose, or monitor the diseases of the invention. Preferably, the diseases of the invention are detected in an animal, more preferably in a mammal and most preferably in a human.

### Screening Assays

The invention provides methods for identifying agents (e.g. candidate compounds or test compounds) that bind to DLL3 or have a stimulatory or inhibitory effect on the expression or activity of DLL3. The invention also provides methods of identifying agents, candidate compounds or test compounds that bind to a DLL3-related polypeptide or a DLL3 fusion protein or have a stimulatory or inhibitory effect on the expression or activity of a DLL3-related polypeptide or a DLL3 fusion protein. Examples of agents, candidate compounds or test compounds include, but are not limited to, nucleic acids (e.g. DNA and RNA), carbohydrates, lipids, proteins, peptides, peptidomimetics, small molecules and other drugs. Agents can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam, 1997, Anticancer Drug Des. 12:145; U.S. Patent No. 5,738,996; and U.S. Patent No. 5,807,683, each of which is incorporated herein in its entirety by reference).

Examples of methods for the synthesis of molecular libraries can be found in the art, for example in: DeWitt et al., 1993, Proc. Natl. Acad. Sci. USA 90:6909; Erb et al., 1994, Proc. Natl. Acad. Sci. USA 91:11422; Zuckermann et al., 1994, J. Med. Chem. 37:2678; Cho et al., 1993, Science 261:1303; Carrell et al., 1994, Angew. Chem. Int. Ed. Engl. 33:2059; Carell et al., 1994, Angew. Chem. Int. Ed. Engl. 33:2061; and Gallop et al., 1994, J. Med. Chem. 37:1233, each of which is incorporated herein in its entirety by reference.

Libraries of compounds may be presented, e.g. presented in solution (e.g. Houghten, 1992, BioTechniques 13:412-421), or on beads (Lam, 1991, Nature 354:82-84), chips (Fodor, 1993, Nature 364:555-556), bacteria (U.S. Patent No. 5,223,409), spores (Patent Nos. 5,571,698; 5,403,484; and 5,223,409), plasmids (Cull et al., 1992, Proc. Natl. Acad. Sci. USA 89:1865-1869) or phage (Scott and Smith, 1990, Science 249:386-390; Devlin, 1990, Science 249:404-406; Cwirla et al., 1990, Proc. Natl. Acad. Sci. USA 87:6378-6382; and Felici, 1991, J. Mol. Biol. 222:301-310), each of which is incorporated herein in its entirety by reference.

In one embodiment, agents that interact with (i.e. bind to) DLL3, a DLL3 fragment (e.g. a functionally active fragment), a DLL3-related polypeptide, a fragment of a DLL3-related polypeptide, or a DLL3 fusion protein are identified in a cell-based assay system. In accordance with this embodiment, cells expressing DLL3, a fragment of a DLL3, a DLL3-related polypeptide, a fragment of the DLL3-related polypeptide, or a DLL3 fusion protein are contacted with a candidate compound or a control compound and the ability of the candidate compound to interact with the DLL3 is determined. If desired, this assay may be used to screen a plurality (e.g. a library) of candidate compounds. The cell, for example, can be of prokaryotic origin (e.g. *E. coli*) or eukaryotic origin (e.g. yeast or mammalian). Further, the cells can express DLL3, a fragment of DLL3, a DLL3-related polypeptide, a fragment of the DLL3-related polypeptide, or a DLL3 fusion protein endogenously or be genetically engineered to express DLL3, a fragment of DLL3, a DLL3-related polypeptide, a fragment of the DLL3-related polypeptide, or a DLL3 fusion protein. In certain instances, DLL3, a fragment of DLL3, a DLL3-related polypeptide, a fragment of the DLL3-related polypeptide, or a DLL3 fusion protein or the candidate compound is labelled, for example with a radioactive label (such as ³²P, ³⁵S, and ¹²⁵I) or a fluorescent label (such as fluorescein isothiocyanate, rhodamine, phycoerythrin, phycocyanin, allophycocyanin, o-phthaldehyde or fluorescamine) to enable detection of an interaction between DLL3 and a candidate compound. The ability of the candidate compound to interact directly or indirectly with DLL3, a fragment of a DLL3, a DLL3-related polypeptide, a fragment of a DLL3-related polypeptide, or a DLL3 fusion protein can be determined by methods known to those of skill in the art. For example, the interaction between a candidate compound and DLL3, a DLL3-related polypeptide, a fragment of a DLL3-related polypeptide, or a DLL3 fusion protein can be determined by flow cytometry, a scintillation assay, immunoprecipitation or western blot analysis.

In another embodiment, agents that interact with (i.e. bind to) DLL3, a DLL3 fragment (e.g. a functionally active fragment), a DLL3-related polypeptide, a fragment of a DLL3-related polypeptide, or a DLL3 fusion protein are identified in a cell-free assay system. In accordance with this embodiment, native or recombinant DLL3 or a fragment thereof, or a native or recombinant DLL3-related polypeptide or fragment thereof, or a DLL3-fusion protein or fragment thereof, is contacted with a candidate compound or a control compound and the ability of the candidate compound to interact with DLL3 or DLL3-related polypeptide, or DLL3 fusion protein is determined. If desired, this assay may be used to screen a plurality (e.g. a library) of candidate compounds. Preferably, DLL3, a DLL3 fragment, a DLL3-related polypeptide, a fragment of a DLL3-related polypeptide, or a DLL3-fusion protein is first immobilized, by, for example, contacting DLL3, a DLL3 fragment, a DLL3-related polypeptide, a fragment of a DLL3-related polypeptide, or a DLL3 fusion protein with an immobilized antibody (or other affinity reagent) which specifically recognizes and binds it, or by contacting a purified preparation of DLL3, a DLL3 fragment, a DLL3-related polypeptide, fragment of a DLL3-related polypeptide, or a DLL3 fusion protein with a surface designed to bind proteins. DLL3, a DLL3 fragment, a DLL3-related polypeptide, a fragment of a DLL3-related polypeptide, or a DLL3 fusion protein may be partially or completely purified (e.g. partially or completely free of other polypeptides) or part of a cell lysate. Further, DLL3, a DLL3 fragment, a DLL3-related polypeptide, or a fragment of a DLL3-related polypeptide may be a fusion protein comprising DLL3 or a biologically active portion thereof, or DLL3-related polypeptide and a domain such as glutathionine-S-transferase. Alternatively, DLL3, a DLL3 fragment, a DLL3-related polypeptide, a fragment of a DLL3-related polypeptide or a DLL3 fusion protein can be biotinylated using techniques well known to those of skill in the art (e.g. biotinylation kit, Pierce Chemicals; Rockford, IL). The ability of the candidate compound to interact with DLL3, a DLL3 fragment, a DLL3-related polypeptide, a fragment of a DLL3-related polypeptide, or a DLL3 fusion protein can be determined by methods known to those of skill in the art.

In another embodiment, a cell-based assay system is used to identify agents that bind to or modulate the activity of a protein, such as an enzyme, or a biologically active portion thereof, which is responsible for the production or degradation of DLL3 or is responsible for the post-translational modification of DLL3. In a primary screen, a plurality (e.g. a library) of compounds are contacted with cells that naturally or recombinantly express: (i) DLL3, an isoform of DLL3, a DLL3 homolog, a DLL3-related polypeptide, a DLL3 fusion protein, or a biologically active fragment of any of the foregoing; and (ii) a protein that is responsible for processing of DLL3, a DLL3 isoform, a DLL3 homolog, a DLL3-related polypeptide, a DLL3 fusion protein, or a fragment in order to identify compounds that modulate the production, degradation, or post-translational modification of DLL3, a DLL3 isoform, a DLL3 homolog, a DLL3-related polypeptide, a DLL3 fusion protein or fragment. If desired, compounds identified in the primary screen can then be assayed in a secondary screen against cells naturally or recombinantly expressing DLL3. The ability of the candidate compound to modulate the production, degradation or post-translational modification of DLL3, isoform, homolog, DLL3-related polypeptide, or DLL3 fusion protein can be determined by methods known to those of skill in the art, including without limitation, flow cytometry, a scintillation assay, immunoprecipitation and western blot analysis.

In another embodiment, agents that competitively interact with (i.e. bind to) DLL3, a DLL3 fragment, a DLL3-related polypeptide, a fragment of a DLL3-related polypeptide, or a DLL3 fusion protein are identified in a competitive binding assay. In accordance with this embodiment, cells expressing DLL3, a DLL3 fragment, a DLL3-related polypeptide, a fragment of a DLL3-related polypeptide, or a DLL3 fusion protein are contacted with a candidate compound and a compound known to interact with DLL3, a DLL3 fragment, a DLL3-related polypeptide, a fragment of a DLL3-related polypeptide or a DLL3 fusion protein; the ability of the candidate compound to preferentially interact with DLL3, a DLL3 fragment, a DLL3-related polypeptide, a fragment of a DLL3-related polypeptide, or a DLL3 fusion protein is then determined. Alternatively, agents that preferentially interact with (i.e. bind to) DLL3, a DLL3 fragment, a DLL3-related polypeptide or fragment of a DLL3-related polypeptide are identified in a cell-free assay system by contacting DLL3, a DLL3 fragment, a DLL3-related polypeptide, a fragment of a DLL3-related polypeptide, or a DLL3 fusion protein with a candidate compound and a compound known to interact with DLL3, a DLL3-related polypeptide or a DLL3 fusion protein. As stated above, the ability of the candidate compound to interact with DLL3, a DLL3 fragment, a DLL3-related polypeptide, a fragment of a DLL3-related polypeptide, or a DLL3 fusion protein can be determined by methods known to those of skill in the art. These assays, whether cell-based or cell-free, can be used to screen a plurality (e.g. a library) of candidate compounds.

In another embodiment, agents that modulate (i.e. upregulate or downregulate) the expression or activity of DLL3 or a DLL3-related polypeptide are identified by contacting cells (e.g. cells of prokaryotic origin or eukaryotic origin) expressing DLL3 or a DLL3-related polypeptide with a candidate compound or a control compound (e.g. phosphate buffered saline (PBS)) and determining the expression of DLL3, DLL3-related polypeptide, or DLL3 fusion protein, mRNA encoding DLL3, or mRNA encoding the DLL3-related polypeptide. The level of expression of DLL3, DLL3-related polypeptide, mRNA encoding DLL3, or mRNA encoding the DLL3-related polypeptide in the presence of the candidate compound is compared to the level of expression of DLL3, DLL3-related polypeptide, mRNA encoding DLL3, or mRNA encoding the DLL3-related polypeptide in the absence of the candidate compound (e.g. in the presence of a control compound). The candidate compound can then be identified as a modulator of the expression of DLL3, or the DLL3-related polypeptide based on this comparison. For example, when expression of DLL3 or mRNA is significantly greater in the presence of the candidate compound than in its absence, the candidate compound is identified as a stimulator of expression of DLL3 or mRNA. Alternatively, when expression of DLL3 or mRNA is significantly less in the presence of the candidate compound than in its absence, the candidate compound is identified as an inhibitor of the expression of DLL3 or mRNA. The level of expression of DLL3 or the mRNA that encodes it can be determined by methods known to those of skill in the art. For example, mRNA expression can be assessed by Northern blot analysis or RT-PCR, and protein levels can be assessed by western blot analysis.

In another embodiment, agents that modulate the activity of DLL3 or a DLL3-related polypeptide are identified by contacting a preparation containing DLL3 or DLL3-related polypeptide or cells (e.g. prokaryotic or eukaryotic cells) expressing DLL3 or DLL3-related polypeptide with a test compound or a control compound and determining the ability of the test compound to modulate (e.g. stimulate or inhibit) the activity of DLL3 or DLL3-related polypeptide. The activity of DLL3 or a DLL3-related polypeptide can be assessed by detecting induction of a cellular signal transduction pathway of DLL3 or DLL3-related polypeptide (e.g. intracellular Ca²⁺, diacylglycerol, IP3, etc.), detecting catalytic or enzymatic activity of the target on a suitable substrate, detecting the induction of a reporter gene (e.g. a regulatory element that is responsive to DLL3 or a DLL3-related polypeptide and is operably linked to a nucleic acid encoding a detectable marker, e.g. luciferase), or detecting a cellular response, for example, cellular differentiation, or cell proliferation. Based on the present description, techniques known to those of skill in the art can be used for measuring these activities (see, e.g. U.S. Patent No. 5,401,639, which is incorporated herein by reference). The candidate compound can then be identified as a modulator of the activity of DLL3 or a DLL3-related polypeptide by comparing the effects of the candidate compound to the control compound. Suitable control compounds include phosphate buffered saline (PBS) and normal saline (NS).

In another embodiment, agents that modulate (i.e. upregulate or downregulate) the expression, activity or both the expression and activity of DLL3 or a DLL3-related polypeptide are identified in an animal model. Examples of suitable animals include, but are not limited to, mice, rats, rabbits, monkeys, guinea pigs, dogs and cats. Preferably, the animal used represent a model of the diseases of the invention (e.g. xenografts of small cell lung cancer cell lines such as NCI-H345; xenografts of non small cell lung cancer cell lines such as A549 and H460; xenografts of pancreatic cancer cell lines such as MIA PaCa-2 in nude mice, Marincola et al., J Surg Res 1989 Dec;47(6):520-9 or xenografts of skin cancer cell lines such as MV3 in nude mice, van Muijen et al., Int J Cancer 1991 Apr 22;48(1):85-91). These can be utilized to test compounds that modulate DLL3 levels, since the pathology exhibited in these models is similar to that of e.g. the diseases of the invention. In accordance with this embodiment, the test compound or a control compound is administered (e.g. orally, rectally or parenterally such as intraperitoneally or intravenously) to a suitable animal and the effect on the expression, activity or both expression and activity of DLL3 or DLL3-related polypeptide is determined. Changes in the expression of DLL3 or a DLL3-related polypeptide can be assessed by the methods outlined above.

In yet another embodiment, DLL3 or a DLL3-related polypeptide is used as a "bait protein" in a two-hybrid assay or three hybrid assay to identify other proteins that bind to or interact with DLL3 or a DLL3-related polypeptide (see, e.g. U.S. Patent No. 5,283,317; Zervos et al. (1993) Cell 72:223-232; Madura et al. (1993) J. Biol. Chem. 268:12046-12054; Bartel et al. (1993) BioTechniques 14:920-924; Iwabuchi et al. (1993) Oncogene 8:1693-1696; and PCT Publication No. WO 94/10300). As those skilled in the art will appreciate, such binding proteins are also likely to be involved in the propagation of signals by DLL3 as, for example, upstream or downstream elements of a signaling pathway involving DLL3.

This invention further provides novel agents identified by the above-described screening assays and uses thereof for treatments as described herein. In addition, the invention also provides the use of an agent which interacts with, or modulates the activity of, DLL3 in the manufacture of a medicament for the treatment of the diseases of the invention.

### Therapeutic Use of DLL3

The invention provides for treatment or prevention of various diseases and disorders by administration of a therapeutic compound. Such compounds include but are not limited to: DLL3, DLL3 analogs, DLL3-related polypeptides and derivatives and variants (including fragments) thereof; antibodies (or other affinity reagents) to the foregoing; nucleic acids encoding DLL3, DLL3 analogs, DLL3-related polypeptides and fragments thereof; antisense nucleic acids to a gene encoding DLL3 or a DLL3-related polypeptide; and modulator (e.g. agonists and antagonists) of a gene encoding DLL3 or a DLL3-related polypeptide. An important feature of the present invention is the identification of genes encoding DLL3 involved in cancers such as the diseases of the invention. The diseases of the invention, for example, can be treated (e.g. to ameliorate symptoms or to retard onset or progression) or prevented by administration of a therapeutic compound that reduces function or expression of DLL3 in the serum or tissue of subjects having the diseases of the invention.

In one embodiment, one or more antibodies (or other affinity reagents) each specifically binding to DLL3 are administered alone or in combination with one or more additional therapeutic compounds or treatments.

A biological product such as an antibody (or other affinity reagent) is allogeneic to the subject to which it is administered. In one embodiment, a human DLL3 or a human DLL3-related polypeptide, a nucleotide sequence encoding a human DLL3 or a human DLL3-related polypeptide, or an antibody (or other affinity reagent) to a human DLL3 or a human DLL3-related polypeptide, is administered to a human subject for therapy (e.g. to ameliorate symptoms or to retard onset or progression) or prophylaxis.

Without being limited by theory, it is conceived that the therapeutic activity of antibodies (or other affinity reagents) which specifically bind to DLL3 may be achieved through the phenomenon of Antibody Dependent Cell-mediated Cytotoxicity (ADCC) (see e.g. Janeway Jr. C.A. et al., Immunobiology, 5th ed., 2001, Garland Publishing, ISBN 0-8153-3642-X; Pier G.B. et al., Immunology, Infection, and Immunity, 2004, p246-5; Albanell J. et al., Advances in Experimental Medicine and Biology, 2003, 532:p2153-68 and Weng, W-K. et al., Journal of Clinical Oncology, 2003, 21:p 3940-3947).

### Treatment And Prevention Of The diseases of the invention

The diseases of the invention, for example, are treated or prevented by administration to a subject suspected of having or known to have one or more of the diseases of the invention or to be at risk of developing one or more of the diseases of the invention of a compound that modulates (i.e. increases or decreases) the level or activity (i.e. function) of DLL3 that is differentially present in the serum or tissue of subjects having one or more of the diseases of the invention compared with serum or tissue of subjects free from the diseases of the invention. In one embodiment, the diseases of the invention are treated or prevented by administering to a subject suspected of having or known to have one or more of the diseases of the invention or to be at risk of developing the diseases of the invention a compound that upregulates (i.e. decreases) the level or activity (i.e. function) of DLL3 that is increased in the serum or tissue of subjects having one or more of the diseases of the invention. Examples of such a compound include, but are not limited to, DLL3 antisense oligonucleotides, ribozymes, antibodies (or other affinity reagents) directed against DLL3, and compounds that inhibit the enzymatic activity of DLL3. Other useful compounds e.g. DLL3 antagonists and small molecule DLL3 antagonists, can be identified using *in vitro* assays.

Cancer, e.g. the diseases of the invention, may also be treated or prevented by administration to a subject suspected of having or known to have such cancer, or to be at risk of developing such cancer, of a compound that downregulates the level or activity (i.e. function) of DLL3 that are increased in the serum or tissue of subjects having such cancer. Examples of such a compound include but are not limited to: DLL3, DLL3 fragments and DLL3-related polypeptides; nucleic acids encoding DLL3, a DLL3 fragment and a DLL3-related polypeptide (e.g. for use in gene therapy); and, for those DLL3 or DLL3-related polypeptides with enzymatic activity, compounds or molecules known to modulate that enzymatic activity. Other compounds that can be used, e.g. DLL3 agonists, can be identified using in *in vitro* assays.

In another embodiment, therapy or prophylaxis is tailored to the needs of an individual subject. Thus, in specific embodiments, compounds that promote the level or function of DLL3 are therapeutically or prophylactically administered to a subject suspected of having or known to have cancer e.g. the diseases of the invention, in whom the levels or functions of DLL3 are absent or are decreased relative to a control or normal reference range. In further embodiments, compounds that promote the level or function of DLL3 are therapeutically or prophylactically administered to a subject suspected of having or known to have cancer e.g. the diseases of the invention in whom the levels or functions of DLL3 are increased relative to a control or to a reference range. In further embodiments, compounds that decrease the level or function of DLL3 are therapeutically or prophylactically administered to a subject suspected of having or known to have cancer e.g. the diseases of the invention in whom the levels or functions of DLL3 are increased relative to a control or to a reference range. In further embodiments, compounds that decrease the level or function of DLL3 are therapeutically or prophylactically administered to a subject suspected of having or known to have cancer e.g. the diseases of the invention in whom the levels or functions of DLL3 are decreased relative to a control or to a reference range. The change in DLL3 function or level due to the administration of such compounds can be readily detected, e.g. by obtaining a sample (e.g. blood or urine) and assaying *in vitro* the levels or activities of DLL3, or the levels of mRNAs encoding DLL3, or any combination of the foregoing. Such assays can be performed before and after the administration of the compound as described herein.

The compounds of the invention include but are not limited to any compound, e.g. a small organic molecule, protein, peptide, antibody (or other affinity reagent), nucleic acid, etc. that restores the DLL3 profile towards normal. The compounds of the invention may be given in combination with any other chemotherapy drugs.

### Vaccine Therapy

Another aspect of the invention is an immunogenic composition, suitably a vaccine composition, comprising DLL3 or an epitope containing fragment thereof, or nucleic acid encoding DLL3 or a fragment thereof optionally together with an immunostimulant.

There is also provided a method of raising an immune response which comprises administering to a subject such compositions and a method for treating or preventing cancer e.g. the diseases of the invention which comprises administering to a subject in need thereof a therapeutically effective amount of such compositions and such compositions for use in preventing or treating the diseases of the invention.

Thus, DLL3 may be useful as antigenic material, and may be used in the production of vaccines for treatment or prophylaxis of cancer, e.g. the diseases of the invention. Such material can be "antigenic" and/or "immunogenic". Generally, "antigenic" is taken to mean that the protein is capable of being used to raise antibodies (or other affinity reagents) or indeed is capable of inducing an antibody response in a subject or experimental animal. "Immunogenic" is taken to mean that the protein is capable of eliciting an immune response such as a protective immune response in a subject or experimental animal. Thus, in the latter case, the protein may be capable of not only generating an antibody response but, in addition, non-antibody based immune responses. "Immunogenic" also embraces whether the protein may elicit an immune-like response in an in-vitro setting e.g. a T-cell proliferation assay. The generation of an appropriate immune response may require the presence of one or more adjuvants and/or appropriate presentation of an antigen.

The skilled person will appreciate that homologues or derivatives of DLL3 will also find use as antigenic/immunogenic material. Thus, for instance proteins which include one or more additions, deletions, substitutions or the like are encompassed by the present invention. In addition, it may be possible to replace one amino acid with another of similar "type", for instance, replacing one hydrophobic amino acid with another. One can use a program such as the CLUSTAL program to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of analysis are contemplated in the present invention.

In the case of homologues and derivatives, the degree of identity with a protein as described herein is less important than that the homologue or derivative should retain its antigenicity and/or immunogenicity. However, suitably, homologues or derivatives having at least 60% similarity (as discussed above) with the proteins or polypeptides described herein are provided, for example, homologues or derivatives having at least 70% similarity, such as at least 80% similarity are provided. Particularly, homologues or derivatives having at least 90% or even 95% similarity are provided. Suitably, homologues or derivatives have at least 60% sequence identity with the proteins or polypeptides described herein. Preferably, homologues or derivatives have at least 70% identity, more preferably at least 80% identity. Most preferably, homologues or derivatives have at least 90% or even 95% identity.

In an alternative approach, the homologues or derivatives could be fusion proteins, incorporating moieties which render purification easier, for example by effectively tagging the desired protein or polypeptide. It may be necessary to remove the "tag" or it may be the case that the fusion protein itself retains sufficient antigenicity to be useful.

It is well known that it is possible to screen an antigenic protein or polypeptide to identify epitopic regions, i.e. those regions which are responsible for the protein or polypeptide's antigenicity or immunogenicity. Methods well known to the skilled person can be used to test fragments and/or homologues and/or derivatives for antigenicity. Thus, the fragments of the present invention should include one or more such epitopic regions or be sufficiently similar to such regions to retain their antigenic/immunogenic properties. Thus, for fragments according to the present invention the degree of identity is perhaps irrelevant, since they may be 100% identical to a particular part of a protein or polypeptide, homologue or derivative as described herein. The key issue, once again, is that the fragment retains the antigenic/immunogenic properties of the protein from which it is derived.

What is important for homologues, derivatives and fragments is that they possess at least a degree of the antigenicity/immunogenicity of the protein or polypeptide from which they are derived. Thus, in an additional aspect of the invention, there is provided antigenic/or immunogenic fragments of DLL3, or of homologues or derivatives thereof.

DLL3, or antigenic fragments thereof, can be provided alone, as a purified or isolated preparation. They may be provided as part of a mixture with one or more other proteins of the invention, or antigenic fragments thereof. In a further aspect, therefore, the invention provides an antigen composition comprising DLL3 and/or one or more antigenic fragments thereof. Such a composition can be used for the detection and/or diagnosis of cancer, e.g. the diseases of the invention.

Vaccine compositions according to the invention may be either a prophylactic or therapeutic vaccine composition.

The vaccine compositions of the invention can include one or more adjuvants (immunostimulants). Examples well-known in the art include inorganic gels, such as aluminium hydroxide, and water-in-oil emulsions, such as incomplete Freund's adjuvant. Other useful adjuvants will be well known to the skilled person.

Suitable adjuvants for use in vaccine compositions for the treatment of cancer include: 3De-O-acylated monophosphoryl lipid A (known as 3D-MPL or simply MPL see WO92/116556), a saponin, for example QS21 or QS7, and TLR4 agonists such as a CpG containing molecule, for example as disclosed in WO95/26204. The adjuvants employed may be a combination of components, for example MPL and QS21 or MPL, QS21 and a CpG containing moiety. Adjuvants may be formulated as oil-in-water emulsions or liposomal formulations. Such preparations may include other vehicles.

In another embodiment, a preparation of oligonucleotides comprising 10 or more consecutive nucleotides complementary to a nucleotide sequence encoding DLL3 or a DLL3 peptide fragments is used as vaccines for the treatment of cancer, e.g. the diseases of the invention. Such preparations may include adjuvants or other vehicles.

### Inhibition Of DLL3 To Treat The diseases of the invention

In one embodiment of the invention, cancer, e.g. the diseases of the invention is treated or prevented by administration of a compound that antagonizes (inhibits) the level and/or function of DLL3 which is elevated in the serum or tissue of subjects having such cancer as compared with serum or tissue of subjects free from such cancer.

Compounds useful for this purpose include but are not limited to anti-DLL3 antibodies (or other affinity reagents, and fragments and derivatives containing the binding region thereof), DLL3 antisense or ribozyme nucleic acids, and nucleic acids encoding dysfunctional DLL3 that may be used to "knockout" endogenous DLL3 function by homologous recombination (see, e.g. Capecchi, 1989, Science 244:1288-1292). Other compounds that inhibit DLL3 function can be identified by use of known *in vitro* assays, e.g. assays for the ability of a test compound to inhibit binding of DLL3 to another protein or a binding partner, or to inhibit a known DLL3 function.

Such inhibition may, for example, be assayed *in vitro* or in cell culture, but genetic assays may also be employed. The Preferred Technologies can also be used to detect levels of DLL3 before and after the administration of the compound. Suitable *in vitro* or *in vivo* assays are utilized to determine the effect of a specific compound and whether its administration is indicated for treatment of the affected tissue, as described in more detail below.

In a specific embodiment, a compound that inhibits DLL3 function (activity) is administered therapeutically or prophylactically to a subject in whom an increased serum or tissue level or functional activity of DLL3 (e.g. greater than the normal level or desired level) is detected as compared with serum or tissue of subjects with e.g. the diseases of the invention who do not receive treatment according to the invention or to bring the level or activity to that found in subjects free from such cancer, or a predetermined reference range. Methods standard in the art can be employed to measure the increase in DLL3 level or function, as outlined above. Suitable DLL3 inhibitor compositions may, for example, include small molecules, i.e. molecules of 1000 daltons or less. Such small molecules can be identified by the screening methods described herein.

### Assays for Therapeutic or Prophylactic Compounds

The present invention also provides assays for use in drug discovery in order to identify or verify the efficacy of compounds for treatment or prevention of cancers expressing DLL3, e.g. the diseases of the invention.

Thus there is provided a method of screening for compounds that modulate the activity of DLL3, the method comprising: (a) contacting DLL3 or a biologically active portion thereof with a candidate compound; and (b) determining whether activity of DLL3 is thereby modulated. Such a process may comprise (a) contacting DLL3 or a biologically active portion thereof with a candidate compound in a sample; and (b) comparing the activity of DLL3 or a biologically active portion thereof in said sample after contact with said candidate compound with the activity of DLL3 or a biologically active portion thereof in said sample before contact with said candidate compound, or with a reference level of activity.

The method of screening may be a method of screening for compounds that inhibit activity of DLL3.

DLL3 or a biologically active portion thereof may, for example be expressed on or by a cell. DLL3 or a biologically active portion thereof may, for example, be isolated from cells which express it. DLL3 or a biologically active portion thereof may, for example, be immobilised onto a solid phase.

There is also provided a method of screening for compounds that modulate the expression of DLL3 or nucleic acid encoding DLL3, the method comprising: (a) contacting cells expressing DLL3 or nucleic acid encoding DLL3 with a candidate compound; and (b) determining whether expression of DLL3 or nucleic acid encoding DLL3 is thereby modulated. Such a process may comprises (a) contacting cells expressing DLL3 or nucleic acid encoding DLL3 with a candidate compound in a sample; and (b) comparing the expression of DLL3 or nucleic acid encoding DLL3 by cells in said sample after contact with said candidate compound with the expression of DLL3 or nucleic acid encoding DLL3 of cells in said sample before contact with said candidate compound, or with a reference level of expression.

The method may be a method of screening for compounds that inhibit expression of DLL3 or nucleic acid encoding DLL3.

Other aspects of the invention include: a compound obtainable by an aforementioned screening method, a compound which modulates the activity or expression of DLL3 or nucleic acid encoding DLL3, for example a compound which inhibits the activity or expression of DLL3 or nucleic acid encoding DLL3.

Such a compound is provided for use in treating or preventing cancer, e.g. the diseases of the invention. There is also provided a method for treating or preventing cancer, e.g. the diseases of the invention which comprises administering to a subject in need thereof a therapeutically effective amount of such a compound.

Test compounds can be assayed for their ability to restore DLL3 levels in a subject having e.g. the diseases of the invention towards levels found in subjects free from such cancers or to produce similar changes in experimental animal models of such cancers. Compounds able to restore DLL3 levels in a subject having e.g. the diseases of the invention towards levels found in subjects free from such cancers or to produce similar changes in experimental animal models of such cancers can be used as lead compounds for further drug discovery, or used therapeutically. DLL3 expression can be assayed by the Preferred Technologies, immunoassays, gel electrophoresis followed by visualization, detection of DLL3 activity, or any other method taught herein or known to those skilled in the art. Such assays can be used to screen candidate drugs, in clinical monitoring or in drug development, where abundance of DLL3 can serve as a surrogate marker for clinical disease.

In various specific embodiments, *in vitro* assays can be carried out with cells representative of cell types involved in a subject's disorder, to determine if a compound has a desired effect upon such cell types.

Compounds for use in therapy can be tested in suitable animal model systems prior to testing in humans, including but not limited to rats, mice, chicken, cows, monkeys, rabbits, etc. For *in vivo* testing, prior to administration to humans, any animal model system known in the art may be used. Examples of animal models of the diseases of the invention include, but are not limited to xenografts of small cell lung cancer cell lines such as NCI-H345; xenografts of non small cell lung cancer cell lines such as A549 and H460; xenografts of pancreatic cancer cell lines such as MIA PaCa-2 in nude mice, Marincola et al., J Surg Res 1989 Dec;47(6):520-9 or xenografts of skin cancer cell lines such as MV3 in nude mice, van Muijen et al., Int J Cancer 1991 Apr 22;48(1):85-91. These can be utilized to test compounds that modulate DLL3 levels, since the pathology exhibited in these models is similar to that of e.g. the diseases of the invention. It is also apparent to the skilled artisan that based upon the present disclosure, transgenic animals can be produced with "knock-out" mutations of the gene or genes encoding DLL3. A "knock-out" mutation of a gene is a mutation that causes the mutated gene to not be expressed, or expressed in an aberrant form or at a low level, such that the activity associated with the gene product is nearly or entirely absent. Preferably, the transgenic animal is a mammal; more preferably, the transgenic animal is a mouse.

In one embodiment, test compounds that modulate the expression of DLL3 are identified in non-human animals (e.g. mice, rats, monkeys, rabbits, and guinea pigs), preferably non-human animal models for the diseases of the invention expressing DLL3. In accordance with this embodiment, a test compound or a control compound is administered to the animals, and the effect of the test compound on expression of DLL3 is determined. A test compound that alters the expression of DLL3 can be identified by comparing the level of DLL3 (or mRNA encoding the same) in an animal or group of animals treated with a test compound with the level of DLL3 or mRNA in an animal or group of animals treated with a control compound. Techniques known to those of skill in the art can be used to determine the mRNA and protein levels, for example, *in situ* hybridization. The animals may or may not be sacrificed to assay the effects of a test compound.

In another embodiment, test compounds that modulate the activity of DLL3 or a biologically active portion thereof are identified in non-human animals (e.g. mice, rats, monkeys, rabbits, and guinea pigs), preferably non-human animal models for the diseases of the invention expressing DLL3. In accordance with this embodiment, a test compound or a control compound is administered to the animals, and the effect of a test compound on the activity of DLL3 is determined. A test compound that alters the activity of DLL3 can be identified by assaying animals treated with a control compound and animals treated with the test compound. The activity of DLL3 can be assessed by detecting induction of a cellular second messenger of DLL3 (e.g. intracellular Ca²⁺, diacylglycerol, IP3, etc.), detecting catalytic or enzymatic activity of DLL3 or binding partner thereof, detecting the induction of a reporter gene (e.g. a regulatory element that is responsive to DLL3 operably linked to a nucleic acid encoding a detectable marker, such as luciferase or green fluorescent protein), or detecting a cellular response (e.g. cellular differentiation or cell proliferation). Techniques known to those of skill in the art can be utilized to detect changes in the activity of DLL3 (see, e.g. U.S. Patent No. 5,401,639, which is incorporated herein by reference).

In yet another embodiment, test compounds that modulate the level or expression of DLL3 are identified in human subjects having e.g. the diseases of the invention, preferably those having e.g. severe the diseases of the invention. In accordance with this embodiment, a test compound or a control compound is administered to the human subject, and the effect of a test compound on DLL3 expression is determined by analyzing the expression of DLL3 or the mRNA encoding the same in a biological sample (e.g. serum, plasma, or urine). A test compound that alters the expression of DLL3 can be identified by comparing the level of DLL3 or mRNA encoding the same in a subject or group of subjects treated with a control compound to that in a subject or group of subjects treated with a test compound. Alternatively, alterations in the expression of DLL3 can be identified by comparing the level of DLL3 or mRNA encoding the same in a subject or group of subjects before and after the administration of a test compound. Techniques known to those of skill in the art can be used to obtain the biological sample and analyze the mRNA or protein expression. For example, the Preferred Technologies described herein can be used to assess changes in the level of DLL3.

In another embodiment, test compounds that modulate the activity of DLL3 are identified in human subjects having e.g. the diseases of the invention (preferably those with e.g. severe the diseases of the invention). In this embodiment, a test compound or a control compound is administered to the human subject, and the effect of a test compound on the activity of DLL3 is determined. A test compound that alters the activity of DLL3 can be identified by comparing biological samples from subjects treated with a control compound to samples from subjects treated with the test compound. Alternatively, alterations in the activity of DLL3 can be identified by comparing the activity of DLL3 in a subject or group of subjects before and after the administration of a test compound. The activity of DLL3 can be assessed by detecting in a biological sample (e.g. serum, plasma, or urine) induction of a cellular signal transduction pathway of DLL3 (e.g. intracellular Ca²⁺, diacylglycerol, IP3, etc.), catalytic or enzymatic activity of DLL3 or a binding partner thereof, or a cellular response, for example, cellular differentiation, or cell proliferation. Techniques known to those of skill in the art can be used to detect changes in the induction of a second messenger of DLL3 or changes in a cellular response. For example, RT-PCR can be used to detect changes in the induction of a cellular second messenger.

In another embodiment, a test compound that changes the level or expression of DLL3 towards levels detected in control subjects (e.g. humans free from e.g. the diseases of the invention) is selected for further testing or therapeutic use. In another embodiment, a test compound that changes the activity of DLL3 towards the activity found in control subjects (e.g. humans free from e.g. the diseases of the invention) is selected for further testing or therapeutic use.

In another embodiment, test compounds that reduce the severity of one or more symptoms associated with e.g. the diseases of the invention are identified in human subjects having e.g. the diseases of the invention, preferably subjects with e.g. severe the diseases of the invention. In accordance with this embodiment, a test compound or a control compound is administered to the subjects, and the effect of a test compound on one or more symptoms of e.g. the diseases of the invention is determined. A test compound that reduces one or more symptoms can be identified by comparing the subjects treated with a control compound to the subjects treated with the test compound. Techniques known to physicians familiar with e.g. the diseases of the invention can be used to determine whether a test compound reduces one or more symptoms associated with e.g. the diseases of the invention. For example, a test compound that reduces tumour burden in a subject having e.g. the diseases of the invention will be beneficial for such subject.

In another embodiment, a test compound that reduces the severity of one or more symptoms associated with cancer, e.g. the diseases of the invention is selected for further testing or therapeutic use.

### Therapeutic and Prophylactic Compositions and their Use

The invention provides methods of treatment (and prophylaxis) comprising administering to a subject an effective amount of a compound of the invention (e.g. DLL3 protein, an affinity reagent capable of specific binding to DLL3 or a fragment thereof. or a nucleic acid encoding DLL3). In a particular aspect, the compound is substantially purified (e.g. substantially free from substances that limit its effect or produce undesired side-effects).

Formulations and methods of administration that can be employed when the compound comprises a nucleic acid are described above; additional appropriate formulations and routes of administration are described below.

Various delivery systems are known and can be used to administer a compound of the invention, e.g. encapsulation in liposomes, microparticles, microcapsules, recombinant cells capable of expressing the compound, receptor-mediated endocytosis (see, e.g. Wu and Wu, 1987, J. Biol. Chem. 262:4429-4432), construction of a nucleic acid as part of a retroviral or other vector, etc. Methods of introduction can be enteral or parenteral and include but are not limited to intradermal, intramuscular, intraperitoneal, intravenous, subcutaneous, intranasal, epidural, and oral routes. The compounds may be administered by any convenient route, for example by infusion or bolus injection, by absorption through epithelial or mucocutaneous linings (e.g. oral mucosa, rectal and intestinal mucosa, etc.) and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g. by use of an inhaler or nebulizer, and formulation with an aerosolizing agent.

In one aspect of the invention a nucleic acid employed in the invention may be delivered to the dermis, for example employing particle mediated epidermal delivery.

In a specific embodiment, it may be desirable to administer the pharmaceutical compositions of the invention locally to the area in need of treatment; this may be achieved, for example, and not by way of limitation, by local infusion during surgery, topical application, e.g. by injection, by means of a catheter, or by means of an implant, said implant being of a porous, non-porous, or gelatinous material, including membranes, such as sialastic membranes, or fibers. In one embodiment, administration can be by direct injection into e.g. lung, pancreas and skin tissue or at the site (or former site) of a malignant tumour or neoplastic or pre-neoplastic tissue.

In another embodiment, the compound can be delivered in a vesicle, in particular a liposome (see Langer, 1990, Science 249:1527-1533; Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; see generally *ibid*.)

In yet another embodiment, the compound can be delivered in a controlled release system. In one embodiment, a pump may be used (see Langer, *supra;* Sefton, 1987, CRC Crit. Ref. Biomed. Eng. 14:201; Buchwald et al., 1980, Surgery 88:507; Saudek et al., 1989, N. Engl. J. Med. 321:574). In another embodiment, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J., 1983, Macromol. Sci. Rev. Macromol. Chem. 23:61; see also Levy et al., 1985, Science 228:190; During et al., 1989, Ann. Neurol. 25:351; Howard et al., 1989, J. Neurosurg. 71:105). In yet another embodiment, a controlled release system can be placed in proximity of the therapeutic target, e.g. the diseases of the invention thus requiring only a fraction of the systemic dose (see, e.g. Goodson, in Medical Applications of Controlled Release, supra, vol. 2, pp. 115-138 (1984)). Other controlled release systems are discussed in the review by Langer (1990, Science 249:1527-1533).

In a specific embodiment where the compound of the invention is a nucleic acid encoding a protein, the nucleic acid can be administered *in vivo* to promote expression of its encoded protein, by constructing it as part of an appropriate nucleic acid expression vector and administering it so that it becomes intracellular, e.g. by use of a retroviral vector (see U.S. Patent No. 4,980,286), or by direct injection, or by use of microparticle bombardment (e.g. a gene gun; Biolistic, Dupont), or coating with lipids or cell-surface receptors or transfecting agents, or by administering it in linkage to a homeobox-like peptide which is known to enter the nucleus (see e.g. Joliot et al., 1991, Proc. Natl. Acad. Sci. USA 88:1864-1868), etc. Alternatively, a nucleic acid can be introduced intracellularly and incorporated within host cell DNA for expression, by homologous recombination.

The present invention also provides pharmaceutical compositions. Such compositions comprise a therapeutically effective amount of a compound of the invention, and a pharmaceutically acceptable carrier. In a specific embodiment, the term "pharmaceutically acceptable" means suitable for approval by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the compound, for example in purified form, together with a suitable amount of carrier so as to provide the form for proper administration to the subject. The formulation should suit the mode of administration.

In one embodiment, for example where one or more antibodies are employed, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds of the invention can be formulated as neutral or salt forms. Pharmaceutically acceptable salts, where appropriate, include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.

The amount of the compound of the invention which will be effective in the treatment of cancer, for example, the diseases of the invention can be determined by standard clinical techniques. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each subject's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from *in vitro* or animal model test systems.

Suppositories generally contain active ingredient in the range of 0.5% to 10% by weight; oral formulations preferably contain 10% to 95% active ingredient.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects (a) approval by the agency of manufacture, use or sale for human administration, (b) directions for use, or both.

Thus in one aspect the kit comprises antibodies employed in the invention, for example the antibodies may be lyophilized for reconstitution before administration or use. Where the kit is for use in therapy/treatment such as cancer the antibody or antibodies may be reconstituted with an isotonic aqueous solution, which may optionally be provided with the kit. In one aspect the kit may comprise a polypeptide such as an immunogenic polypeptide employed in the invention, which may for example be lyophilized. The latter kit may further comprise an adjuvant for reconstiting the immunogenic polypeptide.

The invention also extends to a composition as described herein for example a pharmaceutical composition and/or vaccine composition for use in inducing an immune response in a subject.

In yet a further embodiment, the invention provides a medicament comprising, separately or together:
(a) an affinity reagents which binds to DLL3, and
(b) an anti-cancer agent or other active agent,
for simultaneous, sequential or separate administration in the treatment of cancer, preferably in the treatment of one of the diseases of the invention.

### Determining Abundance of DLL3 by Imaging Technology

An advantage of determining abundance of DLL3 by imaging technology may be that such a method is non-invasive (save that reagents may need to be administered) and there is no need to extract a sample from the subject.

Suitable imaging technologies include positron emission tomography (PET) and single photon emission computed tomography (SPECT). Visualisation of DLL3 using such techniques requires incorporation or binding of a suitable label e.g. a radiotracer such as ¹⁸F, ¹¹C or ¹²³I (see e.g. NeuroRx - The Journal of the American Society for Experimental NeuroTherapeutics (2005) 2(2), 348-360 and idem pages 361-371 for further details of the techniques). Radiotracers or other labels may be incorporated into DLL3 by administration to the subject (e.g. by injection) of a suitably labelled specific ligand. Alternatively they may be incorporated into a binding affinity reagent (e.g. antibody) specific for DLL3 which may be administered to the subject (e.g. by injection). For discussion of use of Affibodies for imaging see e.g. Orlova A, Magnusson M, Eriksson TL, Nilsson M, Larsson B, Hoiden-Guthenberg I, Widstrom C, Carlsson J, Tolmachev V, Stahl S, Nilsson FY, Tumor imaging using a picomolar affinity HER2 binding Affibody molecule, Cancer Res. 2006 Apr 15;66(8):4339-48).

### Diagnosis And Treatment Of Cancer Including The diseases of the invention Using Immunohistochemistrv

Immunohistochemistry is an excellent detection technique and may therefore be very useful in the diagnosis and treatment of cancer, including the diseases of the invention. Immunohistochemistry may be used to detect, diagnose, or monitor cancers such as those mentioned above, through the localization of DLL3 antigens in tissue sections by the use of labelled antibodies (or other affinity reagents), derivatives and analogs thereof, which specifically bind to DLL3, as specific reagents through antigen-antibody interactions that are visualized by a marker such as fluorescent dye, enzyme, radioactive element or colloidal gold.

The advancement of monoclonal antibody technology has been of great significance in assuring the place of immunohistochemistry in the modern accurate microscopic diagnosis of human neoplasms. The identification of disseminated neoplastically transformed cells by immunohistochemistry allows for a clearer picture of cancer invasion and metastasis, as well as the evolution of the tumour cell associated immunophenotype towards increased malignancy. Future antineoplastic therapeutical approaches may include a variety of individualized immunotherapies, specific for the particular immunophenotypical pattern associated with each individual patient's neoplastic disease. For further discussion see e.g. Bodey B, The significance of immunohistochemistry in the diagnosis and therapy of neoplasms, Expert Opin Biol Ther. 2002 Apr; 2(4):371-93.

Preferred features of each aspect of the invention are as for each of the other aspects *mutatis mutandis.* The prior art documents mentioned herein are incorporated to the fullest extent permitted by law.

### Items:

1. A method for the treatment or prophylaxis of lung cancer wherein DLL3 is expressed in said lung cancer, which comprises administering to a subject in need thereof a therapeutically effective amount of a bispecific antibody which binds to DLL3 and CD3.
2. A method for the treatment or prophylaxis of cancer wherein DLL3 is expressed in said cancer, which comprises administering to a subject in need thereof a therapeutically effective amount of an affinity reagent which binds to DLL3.
3. The method according to item 2, for the treatment or prophylaxis of a cancer selected from the group consisting of lung cancer, pancreatic cancer and skin cancer, preferably small cell lung cancer.
4. The method according to item 2 or 3, wherein the affinity reagent binds specifically to DLL3.
5. The method according to any one of items 2 to 4, wherein the affinity reagent is an antibody or a functional fragment thereof or an antibody mimetic.
6. The method according to item 5, wherein the affinity reagent is a monoclonal antibody or an antigen-binding fragment thereof.
7. The method according to item 5 or 6, wherein the affinity reagent is a chimeric antibody, a human antibody, a humanized antibody, a single chain antibody, a defucosylated antibody or a bispecific antibody (preferably a BiTE).
8. The method according to item 5, wherein:
   (a) the functional antibody fragment is a UniBody, a domain antibody or a Nanobody; or
   (b) the antibody mimetic is an Affibody, a DARPin, an Anticalin, an Avimer, a Versabody or a Duocalin.
9. The method according to any one of items 2 to 8, wherein the affinity reagent contains or is conjugated to a therapeutic moiety.
10. The method according to item 9, wherein the therapeutic moiety is a cytotoxic moiety or a radioactive isotope.
11. The method according to item 9 or 10, wherein the affinity reagent is an antibody drug conjugate.
12. The method according to any one of items 1 to 8 wherein the affinity reagent elicits antibody-dependent cellular cytotoxicity (ADCC).
13. The method according to any one of items 1 to 8 wherein the affinity reagent elicits complement dependent cytotoxicity (CDC).
14. The method according to any one of items 1 to 8 wherein the affinity reagent elicits T-cell cytotoxicity.
15. The method according to any one of items 1 to 8 wherein the affinity reagent induces apoptosis of cancer cells, kills or reduces the number of cancer stem cells and/or kills or reduces the number of circulating cancer cells.
16. A method of detecting, diagnosing and/or screening for or monitoring the progression cancer wherein DLL3 is expressed in said cancer, or of monitoring the effect of a cancer drug or therapy wherein DLL3 is expressed in said cancer, in a subject which comprises detecting the presence or level of DLL3, or one or more fragments thereof, or the presence or level of nucleic acid encoding DLL3 or which comprises detecting a change in the level thereof in said subject.
17. The method according to item 16 which comprises detecting the presence of DLL3, or one or more fragments thereof, or the presence of nucleic acid encoding DLL3, in which either (a) the presence of an elevated level of DLL3 or said one or more fragments thereof or an elevated level of nucleic acid encoding DLL3 in the subject as compared with the level in a healthy subject, or (b) the presence of a detectable level of DLL3 or said one or more fragments thereof or a detectable level of nucleic acid encoding DLL3 in the subject as compared with a corresponding undetectable level in a healthy subject is indicative of the presence of cancer wherein DLL3 is expressed in said cancer, in said subject.
18. A method of detecting, diagnosing and/or screening for or monitoring the progression cancer wherein DLL3 is expressed in said cancer, or of monitoring the effect of a cancer drug or therapy wherein DLL3 is expressed in said cancer, in a subject which comprises detecting the presence or level of antibodies capable of immunospecific binding to DLL3, or one or more fragments thereof.
19. The method according to any one of items 16 to 18 wherein the presence of DLL3, or one or more fragments thereof, or the presence of nucleic acid encoding DLL3, or the presence or level of antibodies capable of immunospecific binding to DLL3, or one or more fragments thereof, is detected by analysis of a biological sample obtained from the subject.
20. The method according to any one of items 16 to 19 wherein the presence of DLL3, or one or more fragments thereof, is detected using an affinity reagent which binds to DLL3.
21. The method according to item 20 wherein the affinity reagent is as defined in any one of items 3 to 8.
22. The method according to item 20 or 21 wherein the affinity reagent contains or is conjugated to a detectable label.
23. The method according to any one of items 16 to 22, wherein the cancer is selected from the group consisting of lung cancer, pancreatic cancer and skin cancer.
24. The method according to any one of items 1 to 23 wherein the subject is a human.
25. A method for identifying an agent for the treatment or prophylaxis of cancer wherein DLL3 is expressed in said cancer, wherein the method comprises (a) contacting DLL3, or one or more fragments thereof, with a candidate agent; and (b) determining whether the agent binds to DLL3, or one or more fragments thereof.
26. The method according to item 25 further comprising the step of testing the ability of an agent which binds to DLL3, or one or more fragments thereof, to inhibit cancer wherein DLL3 is expressed in said cancer.
27. The method according to items 25 or 26 wherein the affinity reagent modulates a physiological function of DLL3, inhibits ligand binding to DLL3 and/or inhibits a signal transduction pathway mediated by DLL3.
28. The method according to any one of items 25 to 27, wherein the cancer is selected from the group consisting of lung cancer, pancreatic cancer and skin cancer.

The invention is illustrated by the following non-limiting examples.

### EXAMPLE 1: IDENTIFICATION OF DLL3 EXPRESSED IN NON-SMALL CELL LUNG CANCER. SMALL CELL LUNG CANCER. PANCREATIC CANCER AND SKIN CANCER TISSUE SAMPLES USING LIQUID CHROMATOGRAPHY-MASS SPECTROMETRY (LC/MS)

Using the following protocol, membrane proteins extracted from non-small cell lung cancer, small cell lung cancer, pancreatic cancer and skin cancer tissue and corresponding normal or normal adjacent tissue (NAT) samples were digested and resulting peptides sequenced by tandem mass spectrometry.

### 1.1 MATERIALS AND METHODS

### 1.1.1 Plasma Membrane Fractionation

The cells recovered from a non-small cell lung cancer, small cell lung cancer, pancreatic cancer or skin cancer or a normal or normal adjacent tissue were homogenised and submitted to centrifugation at 1000 x g. The supernatant was taken and ultra-centrifuged at 49500 x g. The resulting pellet was re-homogenized and separated by discontinuous sucrose density centrifugation. After ultra-centrifugation at 107000 x g, the fractions at the phase boundary were recovered and pelleted.

### 1.1.2 Plasma membrane solubilisation

Plasma membrane fractions were resuspended in SDS (Sodium dodecyl sulfate) to give a final SDS concentration of 0.5%, centrifuged and the solubilized protein extracted.

### 1.1.3 Trypsinolysis

For in-solution digestion, the volume of a 50µg protein solution was made up to 100µl using 200mM ammonium bicarbonate. 10µl of the reducing agent DL-Dithiothreitol (75mM) was added to the sample and incubated at 80°C for 15 minutes. This was followed by a cysteine blocking step using 10µl of 150mM iodoacetamide and incubation in the dark for 30 minutes at room temperature. The SDS concentration was then diluted to 0.05% with the addition of ultra-pure water. A sufficient volume of trypsin (Promega V5111) was added to the mixture allowing for 1µg of trypsin to 2.75 µg of protein and incubated overnight at 37°C.

Alternatively, 105µg of protein solutions were reduced using 3µl of 50mM TCEP and incubating at 60°C for 1 hr. The sample was then processed on the FASP filtration devices of the Protein Digestion Kit (Protein Discovery) according to the manufacturer's instructions, but using triethylammonium bicarbonate instead of ammonium bicarbonate. Trypsinolysis was performed in a final volume of 75µl, using 1µg of trypsin to 50µg of protein.

### 1.1.4 Peptide fractionation

The digested protein samples were dried under a vacuum, re-suspended in 0.1% aqueous formic acid and trifluoroacetic acid (TFA) was added to reduce the pH of the solution to <3. Peptides were separated by ion exchange using an Agilent Zorbax Bio-Strong Cation Exchange series II column on an Agilent LC1200 Series liquid chromatography system. Alternatively, the Agilent 3100 OFFGEL Fractionator and the OFFGEL Kit pH 3 - 10 was used for pI-based separation, according to the protocol of the supplier. Following re-hydration of the IPG strips, equal volumes of a membrane digest were loaded into each well. Following separation, the resulting fractions were acidified.

### 1.1.5 Mass spectrometry

Fractionated samples were analysed by liquid chromatography-mass spectrometry using a Waters nanoACQUITY UPLC System fitted with a nanoACQUITY UPLC BEH 130 C18 column, 75 µm x 250mm (186003545) and a LTQ Orbitrap Velos (Thermo Fisher Scientific). Peptides were eluted with a 300nl/min gradient increasing from 3% to 35% acetonitrile over 120 min. Full-scan mass spectra were acquired at 60000 resolving power between 400-2000 m/z mass range in the Orbitrap. In each cycle, the twenty most intense peptides were selected for CID MS/MS scans in the linear ion trap with nanospray ion source fitted on the instrument.

### 1.1.6 Amino acid sequence analysis of peptide

The raw data generated from the LTQ Orbitrap Velos was processed through the Mascot software (Matrix Science) which uses the Mowse algorithm (Curr Biol. 1993 Jun 1;3(6):327-3) to infer amino acids sequences from the peak lists by searching against a sequence database consisting of Ensembl (http://www.ensembl.org/index.html), IPI (www.ebi.ac.uk/IPI/IPIhuman.html) and SwissProt (http://www.uniprot.org) along with contaminant protein sequences. Criteria for peptide identification included trypsin digestion, up to 2 missed cleavage sites and various biological and chemical modifications (oxidized methionine, cysteine modification by MMTS or iodoacetamide and phosphorylation of serine, threonine and tyrosine). Peptides ranked 1 with an expectation value of 0.05% or less, an ion score of 28 or higher were loaded into our OGAP database where they were processed into protein groups.

### 1.1.7 Discrimination of non-small cell lung cancer, small cell lung cancer, pancreatic cancer and skin cancer associated proteins

The process to identify DLL3 used the peptide sequences obtained experimentally by mass spectrometry, as described above, of naturally occurring human proteins to identify and organize coding exons in the published human genome sequence. These experimentally determined sequences indicated in Table 1, were compared with the OGAP® database which was compiled by processing and integration of peptide masses, peptide signatures, ESTs and Public Domain Genomic Sequence Data as described in International Patent Application WO2009/087462.

**Table 1. DLL3 Specific Peptides Identified By LC/MS in the plasma membranes of non-small cell lung cancer, small cell lung cancer, pancreatic cancer and skin cancer tissue samples.**

| **SEQ ID No** | **Peptide Identified** |
|---|---|
| SEQ ID No:5 | VCLKPGLSEEAAESPCALGAALSAR |
| SEQ ID No:6 | AGAWELR |
| SEQ ID No:7 | CEPPAVGTACTR |
| SEQ ID No:8 | AGCSPEHGFCEQPGECR |
| SEQ ID No:9 | SFECTCPR |
| SEQ ID No:10 | NGGLCLDLGHALR |
| SEQ ID No:11 | CSCALGFGGR |

### 1.1.8 Protein Index

The protein index is a measure of both protein prevalence and peptide abundance. The algorithm takes into account both the number of samples in which the protein has been observed and the number of peptides observed vs observable peptides from each sample. The resulting value is then graded by pairwise comparison of corresponding normal samples vs cancer samples.

### 1.2 RESULTS

These experiments identified DLL3 as further described herein. The full-length DLL3 was detected in the plasma membrane of non-small cell lung cancer, small cell lung cancer, pancreatic cancer and skin cancer tissue samples. Table 2 shows the expression distribution of DLL3 measured by the protein index. Expression of DLL3 in these cancer tissues indicates DLL3 is a valuable therapeutic and diagnostic target in these cancers.

**Table 2. DLL3 Protein Index (+++++ = Very High; ++++ = High; +++ = Medium; ++ = Low; + = Very low; - = Not Observed)**

| **Tissue** | **Cancer** | **Normal** |
|---|---|---|
| *Non-small cell lung* | + | - |
| *Pancreas* | + | - |
| *Skin* | + | - |
| *Small cell lung* | + | - |

### EXAMPLE 2: Specificity of Antibodies to DLL3 Determined by Flow Cytometry Analysis

The specificity of polyclonal antibodies to DLL3 were tested by flow cytometry analysis, carried out in DLL3-expressing cell lines.

### Materials and Methods

Anti-DLL3 antibodies were incubated with the DLL3-expressing cells, SHP-77. Cells were washed in FACS buffer (DPBS, 2% FBS), centrifuged and resuspended in 100µl of the diluted primary SHP-77 antibody (also diluted in FACS buffer). The antibody-H322 complex were incubated on ice for 60 min and then washed twice with FACS buffer as described above. The cell-antibody pellet was resuspended in 100µl of the diluted secondary antibody (also diluted in FACS buffer) and incubated on ice for 60 min on ice. The pellet was washed as before and resuspended in 200µl FACS buffer. The samples were loaded onto the BD FACScanto II flow cytometer and the data analyzed using the BD FACSdiva software.

### Results

The results of the flow cytometry analysis demonstrated that anti-SHP-77 polyclonal antibodies bound effectively to the cell-surface human DLL3. The results indicate strong binding of those antibodies against DLL3 on SHP-77 cells.

### EXAMPLE 3: Internalization of anti-DLL3 polyclonal antibodies by SHP-77 and N82 cells.

Anti-DLL3 polyclonal antibodies were shown to be internalized by SHP-77 (human small cell lung cancer) and N82 upon binding to the cells using PabZAP assays. The PabZAP antibodies were bound to the primary antibodies. Next, the PabZAP complex was internalized by the cells. The entrance of Saporin into the cells resulted in protein synthesis inhibition and eventual cell death

The PabZAP assay was conducted as follows. Each of the cells was seeded at a density of 5x103 cells per well. The anti-DLL3 polyclonal antibodies or an isotype control human IgG were serially diluted then added to the cells. The PabZAP were then added at a concentration of 50 µg/ml and the plates allowed to incubate for 48 and 72 hours. Cell viability in the plates was detected by CellTiter-Glo® Luminescent Cell Viability Assay kit (Promega, G7571) and the plates were read at 490nM by a Luminomitor (Tuner BioSystems, Sunnyvale, CA). The data was analyzed by Prism (Graphpad). Cell death was proportional to the concentration of anti-DLL3 polyclonal antibodies.

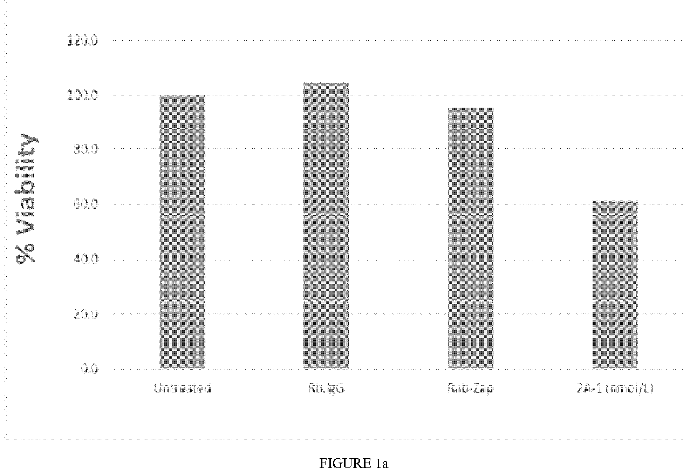

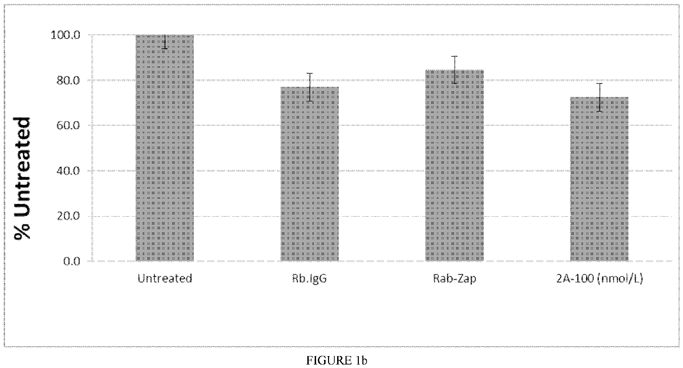

The results show that the anti-DLL3 polyclonal antibodies was efficiently internalized by SHP77 (Figure 1a) and N82 (Figure 1b), as compared to the anti-human IgG isotype control antibody. The results also show anti-DLL3 polyclonal antibodies induced approximately 40% cell kill at 1nmol/L in SHP77 and 25% cell kill at 100nmol/L in N82.

### EXAMPLE 4: T cell activation and specific lysis of DLL3 expressing cells

### Background:

In order to assess the possibility of a target being amenable to a BiTE approach (bispecific antibody fragment combining anti-CD3 binding epitope combined with a binding site for a specific antigen on a target cell or tissue), an assay was developed to test T cell activation with anti-CD3 and a polyclonal antibody specific for a target antigen of interest.

### Methods:

For this assay the target DLL3 is expressed on DMS79 cells. The cells were painted with SIGMA PKH26 Red Fluorescent Cell Linker Kits for General Cell Membrane Labeling Catalog number PKH26GL by diluting 15 ul of dye into 0.5 ml of Buffer C (provided in the kit). DMS79 cells were counted, centrifuged at 800 xg, resuspended in serum free media at 10 million cells in 0.5 ml. The 0.5 ml Buffer C containing the 15 ul FKH26 dye was added to the cells, mixed gently and incubated from 1 to 5 minutes at room temperature. Media plus FBS was added to quench the dye. The cells were centrifuged as above, resuspended in assay media (RPMI plus 10% ultra low IgG FBS - Invitrogen catalog #16250078), centrifuges once more and resuspended in assay media at 200, 000 cells per ml. 10,000 cells (50 ul) will be added to each appropriate well of a 96 well flat bottom tissue culture plate.The plate was previously coated overnight with goat anti-mouse kappa from Southern Biotech (catalog # 1050-01) at 3 ug/ml in PBS. The excess antibody solution was removed from the plates prior to adding the DMS69 cells. Human CD8+ T cells (frozen) were purchased from AllCells catalog number PB009-3F. The T cells were thawed and washed according to manufacturer's directions. Cells were resuspended at 1,500,000 cells per ml in assay media. The T cells were added to the DMS79 cells in the 96 well anti-kappa coated plate at 150,000 cells per well. Each of the DLL3 antibodies were added to the appropriate wells at 18 ug/ml, 6 ug/ml or 2 ug/ml. Functional grade anti-CD3 clone OKT3 (eBioscience catalog number 16-0037-85) was added to the appropriate wells at 9 ug/ml, 3 ug/ml or 1 ug/ml. Control wells received no antibody. The plate was incubated for two days in a 5% CO₂, humidified tissue culture incubator at 37 degrees.
The plate was centrifuged at 400 Xg for 5 minutes, the cells were washed in FACS buffer (PBS + 5% FBS) and again centrifuged at 400 xg for five minutes. The cells were resuspended again in FACS buffer and centrifuged at 400 xg. The cells were resuspended at 200 ul per well of FACS buffer. The wells were mixed gently and immediately analyzed on a Guava Easycyte flow cytometer. The red FKH26 painted cells were analyzed on the yellow channel. The same number of total cells were acquired for each well and the cells in the yellow (FKH26 painted cell gate) were counted and the percent cytotoxicity was calculated relative to control wells T cells plus DMS79 cells without anti-rabbit, anti-CD3 or DLL3 polyclonal (the average cell count was the same +/- plate bound anti-kappa antibody).

### Results:

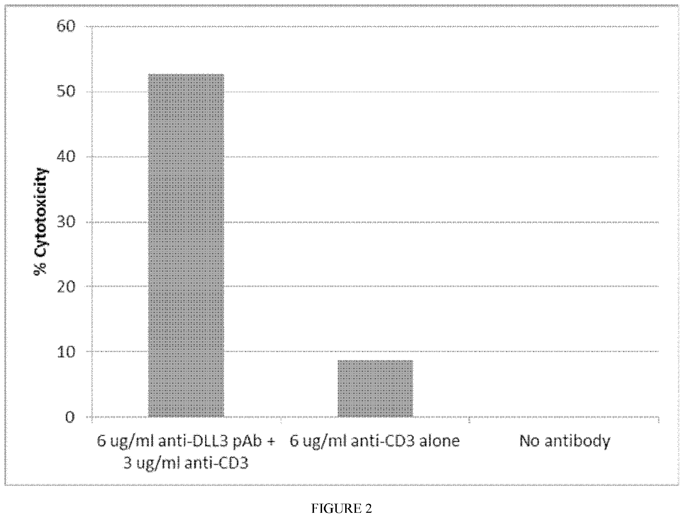

Figure 2 shows the specific lysis of DMS69 cells by anti-DLL3 polyclonal antibodies and that cell death was proportional to antibody concentration. Thus Anti-DLL3 polyclonal antibodies are able to induce T-cell cytotoxicity via activation by CD3.

### EXAMPLE 5: Immunohistochemistrv using antibody to DLL3

Using the following Reference Protocol, immunohistochemistry was performed on FFPE lung tumor and normal tissues using a polyclonal antibody to DLL3.

### 5.1MATERIALS AND METHODS

### 5.1.1 Materials

Citroclear (HC5005) from TCS Biosciences, UK.
Reagent alcohol (R8382) from Sigma-Aldrich, UK.
Target Retrieval Solution, pH6 (S2369) from Dako, UK.
REAL Peroxidase Blocking Solution (S2023) from Dako, UK
Antibody Diluent (S0809) from Dako, UK
EnVision+ HRP-conjugated polymer, Mouse (K4000) from Dako, UK
Liquid DAB+ substrate (K3468) from Dako, UK
Mayer's Hematoxylin (X0909) from Dako, UK
Aquatex (1.08562.0050) from VWR, UK
Tissue sections and arrays were from US Biomax Inc., MD, USA.

### 5.1.2 Deparaffinisation and Rehydration

Slides were deparaffinised in Citroclear (2 x 5 minutes) then rehydrated through 100% alcohol (2 x 5 minutes), 50% alcohol (1 x 5 minutes) and tap water (1 x 5 minutes).

### 5.1.3 Antigen Retrieval (Pressure Cooker)

The DLL3 antigen was retrieved under pressure for 20 minutes in 50ml Target Retrieval Solution in a Coplin jar. Slides were then left to cool to room temperature for a further 20 min. Circles were drawn around each tissue section/TMA with a hydrophobic barrier pen and slides were then washed twice in PBS, 3 minutes each wash.

### 5.1.4 Tissue staining

Endogenous peroxidase activity was blocked by incubating tissues with Peroxidase Blocking Solution for 10 minutes at RT in a humidified chamber. Slides were then washed once in PBS and once in PBS-T (PBS containing Tween-20, 0.125%v/v), 3 minutes each wash. Primary antibody (diluted 1/160 in Antibody Diluent) was applied to each tissue section and/or microarray, and the slides were incubated for 45 min at room temperature in a humidified chamber. Slides were then washed once in PBS and once in PBS-T, 3 minutes each wash. The EnVision+ HRP-conjugated polymer was then applied to the tissues and the slides were incubated for 30 min at room temperature in a humidified chamber. Slides were then washed once in PBS and once in PBS-T, 3 minutes each wash. Tissues were incubated in Liquid DAB+ substrate at room temperature for 10 min in a humidified chamber. Slides were then washed once in PBS and once in PBS-T, counterstained with Hematoxylin for 1 min at room temperature in a humidified chamber, and washed again, once in PBS and once in PBS-T, 3 minutes each wash. Coverslips were then mounted onto the slides using Aquatex.

### 5.2 RESULTS

Anti-DLL3 polyclonal antibodies showed positivity in FFPE lung samples, where 60% of the sections exhibited robust (2+/3+) staining.

Therefore antibodies directed to DLL3 may have utility as therapeutics and diagnostics in some of the tested cancers and possibly other cancer types showing expression of DLL3.

**SEQUENCES**

| **SEQ ID No** | **Description** | **Sequence** |
|---|---|---|
| 1 | Delta-like protein 3 (DLL3) | |
| 2 | Isoform 2 of Delta-like protein 3 (NP_982353) | MVSPRMSGLLSQTVILALIFLPQTRPAGVFELQIHSFGPGPGPGAPRSPCSARLPCRLFFRVCL KPGLSEEAAESPCALGAALSARGPVYTEQPGAPAPDLPLPDGLLQVPFRDAWPGTFSFIIETW REELGDQIGGPAWSLLARVAGRRRLAAGGPWARDIQRAGAWELRFSYRARCEPPAVGTACT RLCRPRSAPSRCGPGLRPCAPLEDECEAPLVCRAGCSPEHGFCEQPGECRCLEGWTGPLCT VPVSTSSCLSPRGPSSATTGCLVPGPGPCDGNPCANGGSCSETPRSFECTCPRGFYGLRCEV SGVTCADGPCFNGGLCVGGADPDSAYICHCPPGFQGSNCEKRVDRCSLQPCRNGGLCLDLG HALRCRCRAGFAGPRCEHDLDDCAGRACANGGTCVEGGGAHRCSCALGFGGRDCRERADP CAARPCAHGGRCYAHFSGLVCACAPGYMGARCEFPVHPDGASALPAAPPGLRPGDPQRYLL PPALGLLVAAGVAGAALLLVHVRRRGHSQDAGSRLLAGTPEPSVHALPDALNNLRTQEGSGD GPSSSVDWNRPEDVDPQGIYVISAPSIYAREA |
| 3 | Delta-like protein 3 (NM_016941) | agatataaggcttggaagccagcagctgcgactcccgagacccccccaccagaaggccatggtctccccacggatgtccgggctc ctctcccagactgtgatcctagcgctcattttcctcccccagacacggcccgctggcgtcttcgagctgcagatccactctttcggcccgg gtccaggccctggggccccgcggtccccctgcagcgcccggctcccctgccgcctcttcttcagagtctgcctgaagcctgggctctc agaggaggccgccgagtccccgtgcgccctgggcgcggcgctgagtgcgcgcggaccggtctacaccgagcagcccggagcgc ccgcgcctgatctcccactgcccgacggcctcttgcaggtgcccttccgggacgcctggcctggcaccttctctttcatcatcgaaacct ggagagaggagttaggagaccagattggagggcccgcctggagcctgctggcgcgcgtggctggcaggcggcgcttggcagccg gaggcccgtgggcccgggacattcagcgcgcaggcgcctgggagctgcgcttctcgtaccgcgcgcgctgcgagccgcctgccgt cgggaccgcgtgcacgcgcctctgccgtccgcgcagcgcccccctcgcggtgcggtccgggactgcgcccctgcgcaccgctcgag gacgaatgtgaggcgccgctggtgtgccgagcaggctgcagccctgagcatggcttctgtgaacagcccggtgaatgccgatgcct agagggctggactggacccctctgcacggtccctgtctccaccagcagctgcctcagccccaggggcccgtcctctgctaccaccgg atgccttgtccctgggcctgggccctgtgacgggaacccgtgtgccaatggaggcagctgtagtgagacacccaggtcctttgaatgc acctgcccgcgtgggttctacgggctgcggtgtgaggtgagcggggtgacatgtgcagatggaccctgcttcaacggcggcttgtgtgt cggggggtgcagaccctgactctgcctacatctgccactgcccacccggtttccaaggctccaactgtgagaagagggtggaccggtg cagcctgcagccatgccgcaatggcggactctgcctggacctgggccacgccctgcgctgccgctgccgcgccggcttcgcgggtc ctcgctgcgagcacgacctggacgactgcgcgggccgcgcctgcgctaacggcggcacgtgtgtggagggcggcggcgcgcac cgctgctcctgcgcgctgggcttcggcggccgcgactgccgcgagcgcgcggacccgtgcgccgcgcgccccctgtgctcacggcg gccgctgctacgcccacttctccggcctcgtctgcgcttgcgctcccggctacatgggagcgcggtgtgagttcccagtgcaccccgac ggcgcaagcgccttgcccgcggccccgccgggcctcaggcccggggaccctcagcgctaccttttgcctccggctctgggactgctc gtggccgcgggcgtggccggcgctgcgctcttgctggtccacgtgcgccgccgtggccactcccaggatgctgggtctcgcttgctgg ctgggaccccggagccgtcagtccacgcactcccggatgcactcaacaacctaaggacgcaggagggttccggggatggtccga gctcgtccgtagattggaatcgccctgaagatgtagaccctcaagggatttatgtcatatctgctccttccatctacgctcgggaggtagc gacgccccttttccccccgctacacactgggcgcgctgggcagaggcagcacctgctttttccctacccttcctcgattctgtccgtgaaa tgaattgggtagagtctctggaaggttttaagcccattttcagttctaacttactttcatcctattttgcatccctcttatcgttttgagctacctgc catcttctctttgaaaaacctatgggcttgaggaggtcacgatgccgactccgccagagcttttccactgattgtactcagcggggaggc aggggaggcagaggggcagcctctctaatgcttcctactcattttgtttctaggcctgacgcgtctcctccatccgcacctggagtcaga gcgtggatttttgtatttgctcggtggtgcccagtctctgccccagaggctttggagttcaatcttgaaggggtgtctgggggaactttactgt tgcaagttgtaaataatggttatttatatcctatttttctcaccccatctctctagaaacacctataaaggctattattgtgatcagttttgacta acaaaaaa |
| 4 | Isoform 2 of Delta-like protein 3 (NM_203486) | agatataaggcttggaagccagcagctgcgactcccgagacccccccaccagaaggccatggtctccccacggatgtccgggctc ctctcccagactgtgatcctagcgctcattttcctcccccagacacggcccgctggcgtcttcgagctgcagatccactctttcgggccgg gtccaggccctggggccccgcggtccccctgcagcgcccggctcccctgccgcctcttcttcagagtctgcctgaagcctgggctctc agaggaggccgccgagtccccgtgcgccctgggcgcggcgctgagtgcgcgcggaccggtctacaccgagcagcccggagcgc ccgcgcctgatctcccactgcccgacggcctcttgcaggtgcccttccgggacgcctggcctggcaccttctctttcatcatcgaaacct ggagagaggagttaggagaccagattggagggcccgcctggagcctgctggcgcgcgtggctggcaggcggcgcttggcagccg gaggcccgtgggcccgggacattcagcgcgcaggcgcctgggagctgcgcttctcgtaccgcgcgcgctgcgagccgcctgccgt cgggaccgcgtgcacgcgcctctgccgtccgcgcagcgcccccctcgcggtgcggtccgggactgcgcccctgcgcaccgctcgag gacgaatgtgaggcgccgctggtgtgccgagcaggctgcagccctgagcatggcttctgtgaacagcccggtgaatgccgatgcct agagggctggactggacccctctgcacggtccctgtctccaccagcagctgcctcagccccaggggcccgtcctctgctaccaccgg atgccttgtccctgggcctgggccctgtgacgggaacccgtgtgccaatggaggcagctgtagtgagacacccaggtcctttgaatgc acctgcccgcgtgggttctacgggctgcggtgtgaggtgagcggggtgacatgtgcagatggaccctgcttcaacggcggcttgtgtgt cgggggtgcagaccctgactctgcctacatctgccactgcccacccggtttccaaggctccaactgtgagaagagggtggaccggtg |
| | | cagcctgcagccatgccgcaatggcggactctgcctggacctgggccacgccctgcgctgccgctgccgcgccggcttcgcgggtc ctcgctgcgagcacgacctggacgactgcgcgggccgcgcctgcgctaacggcggcacgtgtgtggagggcggcggcgcgcac cgctgctcctgcgcgctgggcttcggcggccgcgactgccgcgagcgcgcggacccgtgcgccgcgcgccccctgtgctcacggcg gccgctgctacgcccacttctccggcctcgtctgcgcttgcgctcccggctacatgggagcgcggtgtgagttcccagtgcaccccgac ggcgcaagcgccttgcccgcggccccgccgggcctcaggcccggggaccctcagcgctaccttttgcctccggctctgggactgctc gtggccgcgggcgtggccggcgctgcgctcttgctggtccacgtgcgccgccgtggccactcccaggatgctgggtctcgcttgctgg ctgggaccccggagccgtcagtccacgcactcccggatgcactcaacaacctaaggacgcaggagggttccggggatggtccga gctcgtccgtagattggaatcgccctgaagatgtagaccctcaagggatttatgtcatatctgctccttccatctacgctcgggaggcctg acgcgtctcctccatccgcacctggagtcagagcgtggatttttgtatttgctcggtggtgcccagtctctgccccagaggctttggagttc aatcttgaaggggtgtctgggggaactttactgttgcaagttgtaaataatggttatttatatcctatttttttctcaccccatctctctagaaaca cctataaaggctattattgtgatcagttttgactaacaaaaaa |
| 5 | DLL3 Peptide 1 | VCLKPGLSEEAAESPCALGAALSAR |
| 6 | DLL3 Peptide 2 | AGAWELR |
| 7 | DLL3 Peptide 3 | CEPPAVGTACTR |
| 8 | DLL3 Peptide 4 | AGCSPEHGFCEQPGECR |
| 9 | DLL3 Peptide 5 | SFECTCPR |
| 10 | DLL3 Peptide 6 | NGGLCLDLGHALR |
| 11 | DLL3 Peptide 7 | CSCALGFGGR |
| 12 | DLL3 ECD (amino acids 27-492 of SEQ ID NO: 1) | AGVFELQIHSFGPGPGPGAPRSPCSARLPCRLFFRVCLKPGLSEEAAESPCALGAALSAR GPVYTEQPGAPAPDLPLPDGLLQVPFRDAWPGTFSFIIETWREELGDQIGGPAWSLLARV AGRRRLAAGGPWARDIQRAGAWELRFSYRARCEPPAVGTACTRLCRPRSAPSRCGPGLRP CAPLEDECEAPLVCRAGCSPEHGFCEQPGECRCLEGWTGPLCTVPVSTSSCLSPRGPSSA TTGCLVPGPGPCDGNPCANGGSCSETPRSFECTCPRGFYGLRCEVSGVTCADGPCFNGGL CVGGADPDSAYICHCPPGFQGSNCEKRVDRCSLQPCRNGGLCLDLGHALRCRCRAGFAGP RCEHDLDDCAGRACANGGTCVEGGGAHRCSCALGFGGRDCRERADPCAARPCAHGGRCYA HFSGLVCACAPGYMGARCEFPVHPDGASALPAAPPGLRPGDPQRYL |

## Claims

1. An affinity reagent which binds to DLL3 for use in the treatment or prophylaxis of cancer, wherein DLL3 is expressed in said cancer.

2. The affinity reagent for use according to claim 1, wherein said cancer is selected from the group consisting of lung cancer, pancreatic cancer and skin cancer, preferably small cell lung cancer.

3. The affinity reagent for use according to claim 1 or 2, wherein the affinity reagent binds specifically to DLL3.

4. The affinity reagent for use according to any one of claims 1 to 3, wherein the affinity reagent is an antibody or a functional fragment thereof or an antibody mimetic.

5. The affinity reagent for use according to claim 4, wherein the affinity reagent is a monoclonal antibody or an antigen-binding fragment thereof.

6. The affinity reagent for use according to claim 4 or 5, wherein the affinity reagent is a chimeric antibody, a human antibody, a humanized antibody, a single chain antibody, a defucosylated antibody or a bispecific antibody (preferably a BiTE).

7. The affinity reagent for use according to any one of claims 4 to 6, wherein the affinity reagent elicits T-cell cytotoxicity.

8. The affinity reagent for use according to claims 6 or 7, wherein the affinity reagent is a bispecific affinity reagent comprising a first binding domain for DLL3 and a second binding domain for CD3.

9. A bispecific affinity reagent comprising a first binding domain for DLL3 and a second binding domain for CD3.
